# EUROPEAN PATENT APPLICATION

(11) **EP 4 036 093 A1**
(43) Date of publication of application: **03.08.2022**
(21) Application number: 20867475.4
(22) Date of filing: 25.09.2020
(51) Int. Cl.: C07D 417/14, C07D 401/14, C07D 417/04, C07D 405/14, A61K 31/45, A61K 31/44, A61P 35/00

(54) **PROTAC SMALL MOLECULE COMPOUND AND APPLICATION THEREOF**

(30) Priority: 25.09.2019 CN 201910912417; 24.09.2020 CN 202011016521
(71) Applicant: Zhuhai Yufan Biotechnologies Co., Ltd, Guangdong 519000 (CN); Tsinghua University, Haidian District Beijing 100084 (CN)
(72) Inventor: LIAO, Xuebin, Zhuhai, Guangdong 519000 (CN); SUN, Shuhao, Zhuhai, Guangdong 519000 (CN); LIN, Xingyu, Zhuhai, Guangdong 519000 (CN); LIU, Yahui, Zhuhai, Guangdong 519000 (CN)
(74) Representative: HGF
(86) International application number: PCT/CN2020/117573
(87) International publication number: WO 2021/057872

(57) **Abstract**

The present invention discloses a compound of general formula I, or a pharmaceutically acceptable salt, a stereoisomer, an ester, a prodrug, a solvate and a deuterated compound thereof, a composition comprising the compound of general formula I, and use of the compound of general formula I, or the pharmaceutically acceptable salt, the stereoisomer, the ester, the prodrug, the solvate and the deuterated compound thereof for preparing a medicament for treating a disease associated with the serine/threonine kinase family (MAP4Ks), and preferably for preparing a medicament for treating a disease associated with hematopoietic progenitor kinase 1 (HPK1).

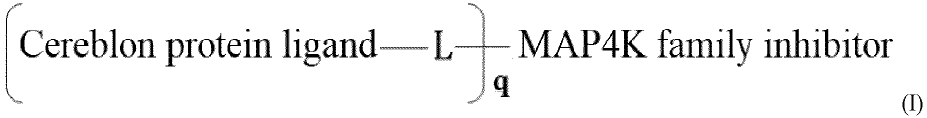

## Description

### TECHNICAL FIELD

The present invention belongs to the field of biomedicine, and particularly relates to a PROTAC small molecular compound and use thereof.

### BACKGROUND

Proteolysis-targeting chimera (PROTAC) technology originated from the discovery of ubiquitin (Ub)-regulated proteolysis processes by scientists. Eukaryotic cells are constantly struggling to maintain appropriate protein levels, producing and degrading thousands of proteins every moment. A key factor to maintain protein balance is a small protein molecule called ubiquitin. When it binds to proteins, the proteins will be transported to proteasomes for degradation.

Targeted protein degradation is an emerging direction in the field of medicament development. Targeted protein degradation medicaments aim to design small molecules as a novel medicament; conventional small molecules act to block the functions of proteins, while targeted protein degraders act to completely degrade these proteins by feeding them into the proteasomes.

In 2010, a study by Handa and co-workers found that E3 ligase Cereblon (CRBN) is a main target of thalidomide protein. Considering the binding ability of phthalimide to CRBN, the Crews Lab and others have attempted to hijack CRBN to degrade the target protein using phthalimide as an E3 ligase recruiting ligand. Conjugation of a small molecule BRD4 binding moiety (OTX015) to pomalidomide results in PROTAC capable of degrading the epigenetic regulator BRD4 at picomolar efficiency. Compared with the BRD4 inhibitors JQ1 and OTX015, CRBN-based PROTAC is able to inhibit an expression level of c-myc more persistently by counteracting an increased expression of BRD4, feedback known to be associated with BRD4 inhibition. At a cellular level, this persistent inhibition results in superior anti-proliferative and apoptotic effects on Burkitt's lymphoma cell lines. In one study, the BRD4 inhibitor JQ1 is conjugated to a thalidomide derivative, and the resulting PROTAC, called dBET1, is able to induce BRD4 degradation at low nanomolar concentrations. Proteomic research on this PROTAC shows that the compound significantly reduces levels of c-myc, BRD2, BRD3 and BRD4, and meanwhile has reduced off-target degradation effects.

Cereblon is a protein encoded by the human CRBN gene, and CRBN homologous genes are highly conserved, indicating its importance in physiology. Cereblon, together with damaged DNA binding protein 1(DDB1), Cullin-4A (CUL4A) and Cullin-1 regulator (ROC1), forms an E3 ubiquitin ligase complex. This complex is capable of ubiquitinating a range of proteins, but the exact mechanism is not known. Cereblon ubiquitinated target protein causes an increase of fibroblast growth factor 8 (FGF8) and fibroblast growth factor 10 (FGF10), indicating that the ubiquitinase complex is important for embryonic limb growth.

Hematopoietic progenitor kinase 1 (HPK1) is involved in many signaling cascade reactions including growth factor signaling, MAPK signaling, cytokine signaling, apoptosis signaling, growth factor signaling, antigen receptor signaling and the like. HPK1 kinase is a key functional activator of JNK/SAPK signaling pathway, and when it is activated, the HPK1 kinase selectively activates MAPK signaling pathway of C-Jun N-terminal kinase (JNK). HPK1 kinase can be used as a target of immunotherapy, and it is activated by lymphocyte antigen receptors and inhibits AP-1; AP-1 plays a role in promoting cell proliferation, inhibiting differentiation, promoting invasion and metastasis of tumor cells and the like in processes of tumor formation and development. However, targeted disruption of alleles of the HPK1 kinase can lead to increased production of Thl cytokines by T cells in TCR responses.

A proteolysis targeting chimera is designed by the present invention, which can carry out ubiquitination labeling on HPK1, induce proteolysis, and has an inhibition effect superior to that of an HPK1 kinase inhibitor alone, that is, a small dose of medicament can be used to inhibit expression of HPK1 protein. On the premise of the same therapeutic effect, dosage of the medicament is less, and toxic and side effects are less.

### SUMMARY

The present invention aims to provide a proteolysis targeting chimera for degrading HPK1 induced by a Cereblon ligand, and use of the proteolysis targeting chimera, and a pharmaceutically acceptable salt, a stereoisomer, an ester, a prodrug, a solvate and a deuterated compound thereof for treating diseases such as tumor, inflammation and immunity diseases.

The objectives of the present invention are implemented through the following technical solutions.

The present invention provides a compound of general formula I: 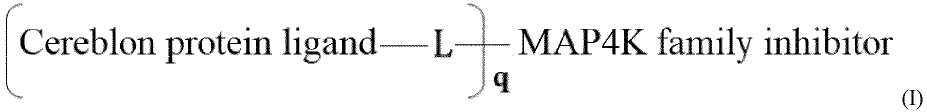
wherein the MAP4K family inhibitor is selected from: a HPK1 inhibitor, an MAP4K2 inhibitor, an MAP4K3 inhibitor, an MAP4K4 inhibitor, an MAP4K5 inhibitor, and an MAP4K6 inhibitor; and preferably, the MAP4K family inhibitor is selected from: an HPK1(MAP4K1) inhibitor;
wherein q is selected from integers between 1 and 5;
the Cereblon protein ligand is a small molecular ligand of Cereblon protein in an E3 ubiquitin ligase complex; L is a linking group between the Cereblon protein ligand and the HPK1 inhibitor; and the attachment sites and the number of attachments of the Cereblon protein ligands affect activities of the compound, and in the present application, q is selected from integers between 1 and 5, and preferably, q is selected from integers between 1 and 3, such as 1, 2 or 3.

The Cereblon protein ligand refers to a small molecule ligand of the Cereblon protein in an E3 ubiquitin ligase complex, and is selected from: an amide compound, a phthalimide compound, thalidomide and a derivative thereof, lenalidomide and a derivative thereof, and pomalidomide and a derivative thereof.

Preferably, the Cereblon protein ligand moiety has a general formula as follows: 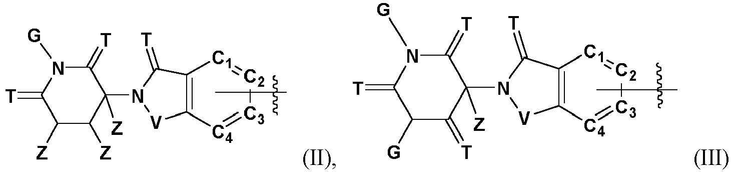 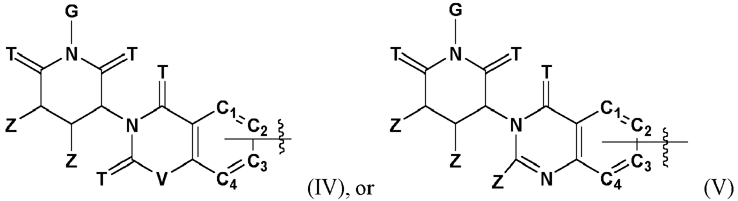 wherein C₁, C₂, C₃ and C₄ are independently selected from C and N; preferably, C₁, C₂, C₃ and C₄ are C; when C₁ is C, at least one of C₂, C₃ and C₄ is N; when C₂ is C, at least one of C₁, C₃ and C₄ is N; when C₃ is C, at least one of C₁, C₂ and C₄ is N; and when C₄ is C, at least one of C₁, C₂ and C₃ is N;
T is selected from O and S;
V is selected from -O-, -S-, 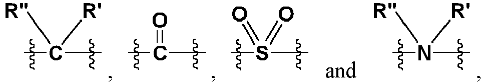 wherein R' and R" are independently selected from C₀₋₁₀ alkyl, cyclohydrocarbyl and heterocyclohydrocarbyl; and G and Z are independently selected from H, -OH, C₁₋₁₀ linear/branched alkyl, C₃₋₁₀ cyclohydrocarbyl, O-containing heterocyclohydrocarbyl, N-containing heterocyclohydrocarbyl and S-containing heterocyclohydrocarbyl.

Preferably, C₁, C₂, C₃ and C₄ are C;
T is O;
V is selected from -CH₂-, 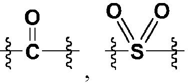 and -NH-; and
G and Z are independently selected from H, -CH₃ and -CH₂CH₃.

In a preferred embodiment of the present invention, the Cereblon protein ligand moiety has a structural formula as follows:

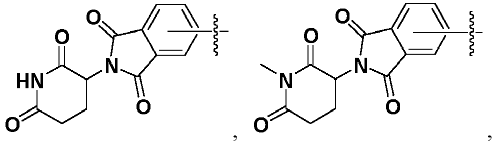

L is a linking group between the Cereblon protein ligand and the HPK1 inhibitor, the Cereblon protein ligand is covalently bonded to the HPK1 inhibitor, and the linking group is -A₁-A₂ ....... Aₜ-, wherein
A₁, A₂......Aₜ are independently selected from:
CR^{L1}R^{L2}, O, S, S=O, S(=O)₂, -S(O)₂O, -OS(O)₂, OS(O)₂O, NR^{L3}, S(=O)₂NR^{L3}, S(=O)NR^{L3}, C(=O)NR^{L3}, OC(O)NR^{L3}, NR^{L3}C(=O), NR^{L3}C(=O)NR^{L4}, NR^{L3}S(=O)₂NR^{L4}, C(=O), CR^{L1}=CR^{L2}, C=C, C≡C, SiR^{L1}R^{L2}, P(=O)R^{L1}, P(=O)OR^{L1}, NR^{L3}C(=N-CN)NR^{L4}, NR^{L3}C(=N-CN), NR^{L3}C(=C-NO₂)NR^{L4}, C₃₋₁₁ cyclohydrocarbyl, C₃₋₁₁ heterocyclohydrocarbyl, succinimide, 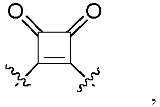 -CH₂CH₂O-, -OCH₂CH₂-, 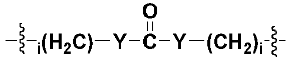 and 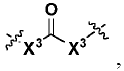 or are absent, wherein any hydrogen atom of the cyclohydrocarbyl and the heterocyclohydrocarbyl may be substituted with 1-6 R^{L1} or R^{L2};
when A₁, A₂......Aₜ are CR^{L1}R^{L2} or SiR^{L1}R^{L2}, R^{L1} and R^{L2} can be independently attached to other A to form cyclohydrocarbyl or heterocyclohydrocarbyl, and preferably, the cyclohydrocarbyl or heterocyclohydrocarbyl is optionally substituted with 1-11 R^{L5} groups;
R^{L1}, R^{L2}, R^{L3}, R^{L4} and R^{L5} are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cyclohydrocarbyl, C₃₋₁₁ heterocyclohydrocarbyl, O(C₁₋₈ cyclohydrocarbyl), S(C₁₋₈ cyclohydrocarbyl), NH(C₁₋₈ cyclohydrocarbyl), N(C₁₋₈ cyclohydrocarbyl)(C₁₋₈ alkyl), OH, NH₂, SH, SO₂(C₁₋₈ alkyl), P(=O)(OC₁₋₈ alkyl)(C₁₋₈ alkyl), P(=O)(OC₁₋₈ alkyl)₂, C₁₋₈ alkynyl, CH=CH(C₁₋₈ alkyl), C(C₁₋₈ alkyl)=CH(C₁₋₈ alkyl), C(C₁₋₈alkyl)=C(C₁₋₈alkyl)₂, Si(OH)₃, Si(C₁₋₈ alkyl)₃, Si(OH)(C₁₋₈ alkyl)₂, C(=O)(C₁₋₈ alkyl), CO₂H, CN, CF₃, CHF₂, CH₂F, NO₂, SF₅, SO₂NH(C₁₋₈ alkyl), SO₂N(C₁₋₈ alkyl)₂, S(=O)N(C₁₋₈ alkyl)₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), NHSO₂N(C₁₋₈ alkyl)₂ and NHSO₂NH₂;
X³ is selected from -O-, -S-, -NR- and -CHR-;
R is selected from H and C₁₋₃ alkyl substituted with 1 or 2 -OH;
i is selected from integers between 1 and 6;
Y is selected from -O-, -S- and -NR-, or is absent;
t is selected from integers between 1 and 100, and preferably, t is selected from integers between 1 and 50.

In one embodiment of the present invention, the linking group L is wherein s is selected from integers between 1 and 6; 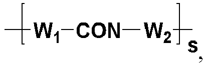 CON is selected from H, 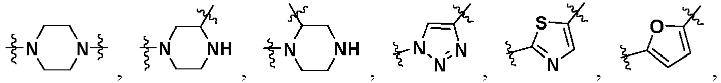 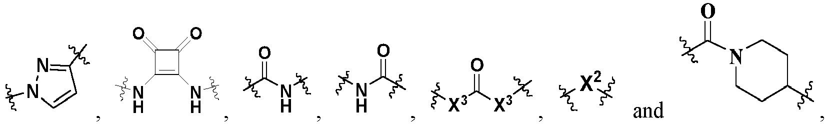 or is absent;
W₁ and W₂ are independently selected from 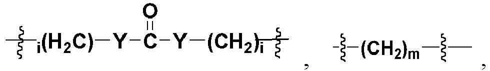 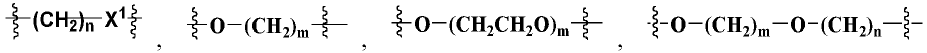 and 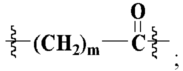 i is selected from integers between 1 and 6;
n is selected from integers between 1 and 10, and preferably from integers between 1 and 4;
m is selected from integers between 0 and 10, and preferably from integers between 2 and 7;
X¹ is selected from -O-, -S- and -NR-; and
X² is selected from -O-, -S-, NR-, -S(O)-, -S(O)₂O, -OS(O)₂- and -OS(O)₂O-.

In another embodiment of the present invention, the linking group is selected from: 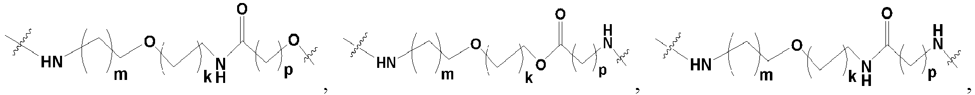 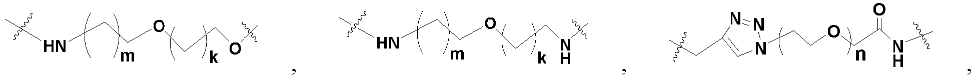 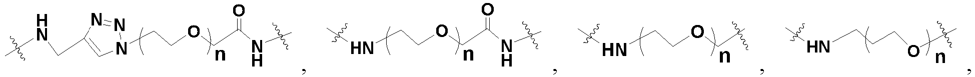 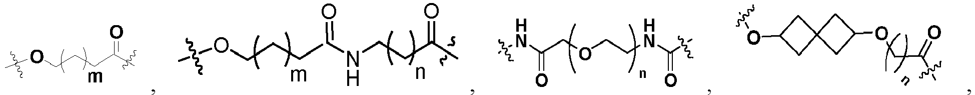 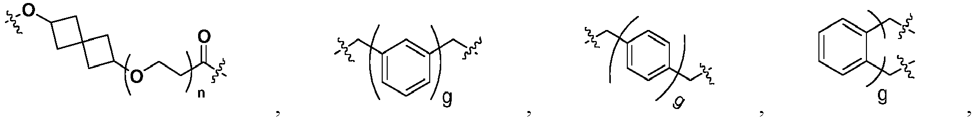 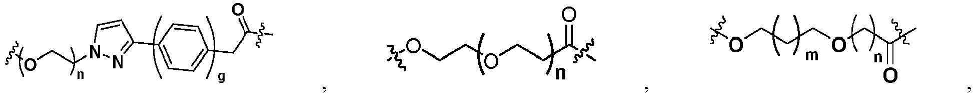 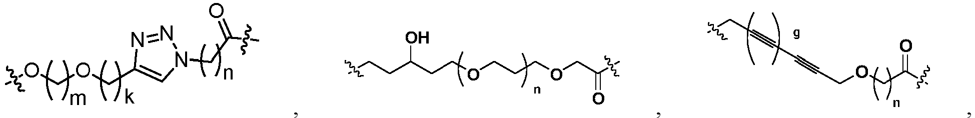 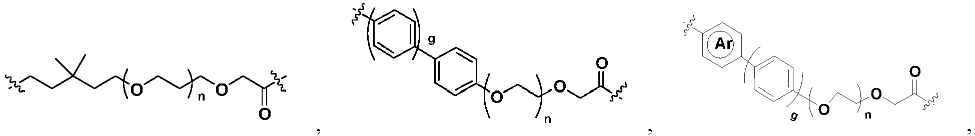 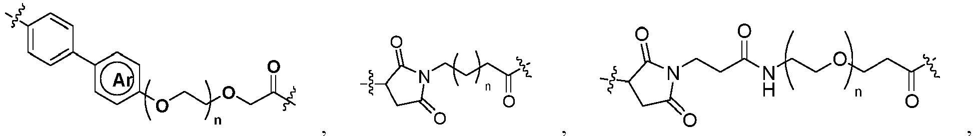 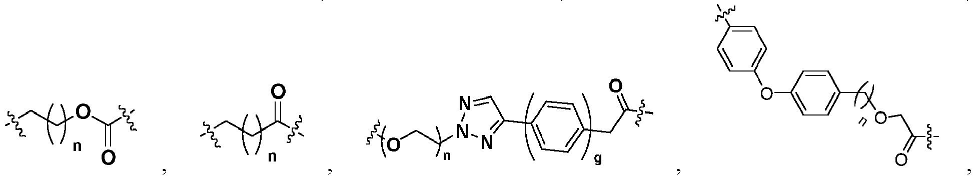 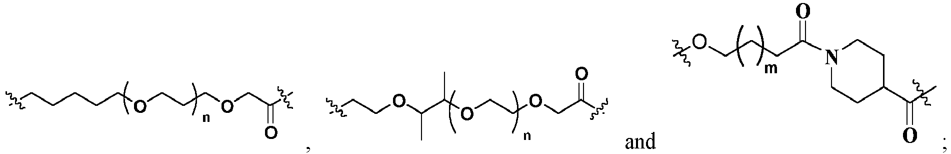 wherein Ar is selected from N-containing six-membered aryl;
g is selected from 0 and 1;
n is selected from integers between 1 and 10, and preferably from integers between 1 and 4;
m is selected from integers between 1 and 10, and preferably from integers between 2 and 7;
k is selected from integers between 1 and 10, and preferably from integers between 1 and 4; and
p is selected from integers between 1 and 10, and preferably from integers between 1 and 2.

In a preferred embodiment of the present invention, the linking group L has a structure as follows:

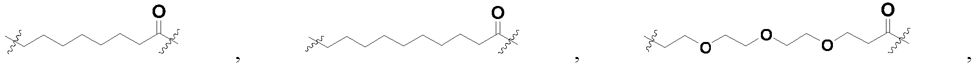

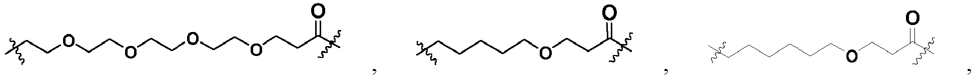

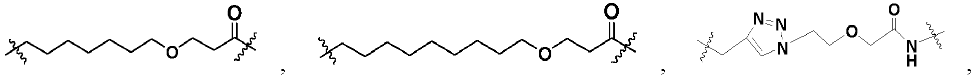

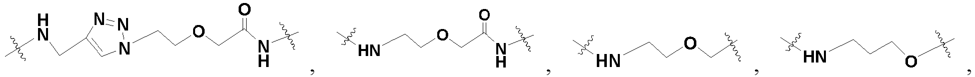

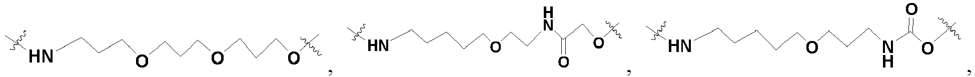

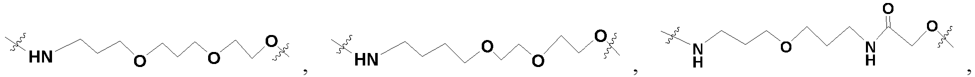

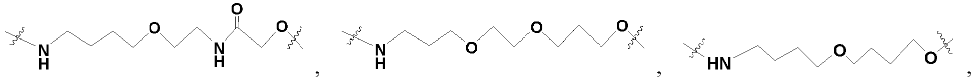

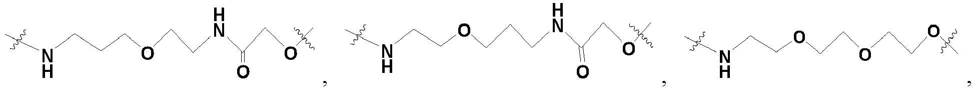

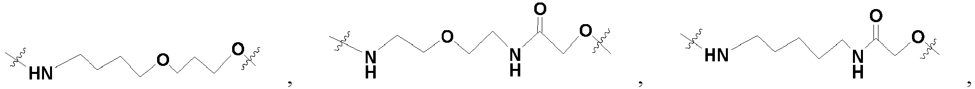

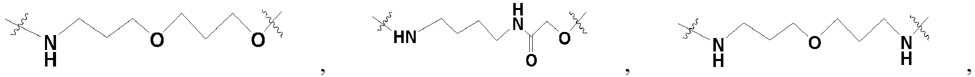

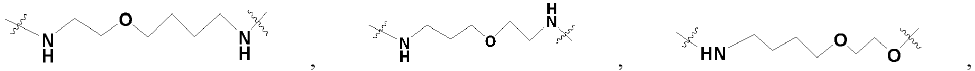

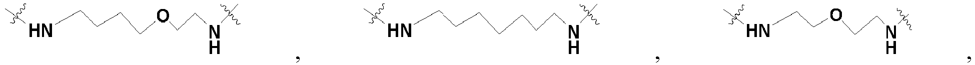

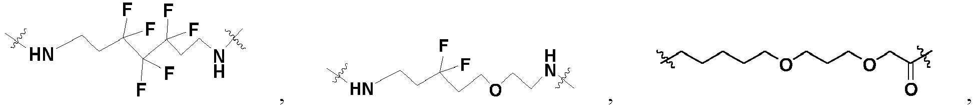

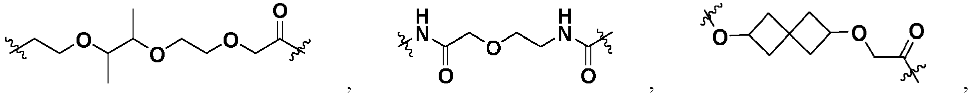

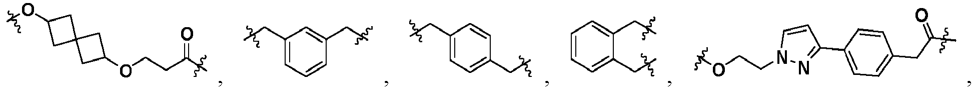

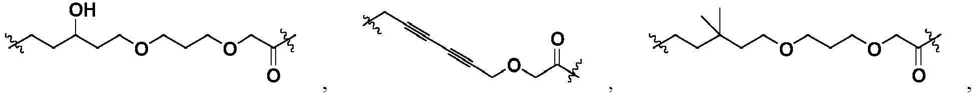

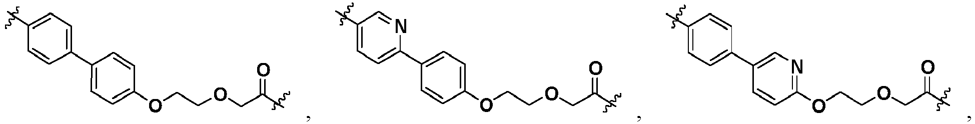

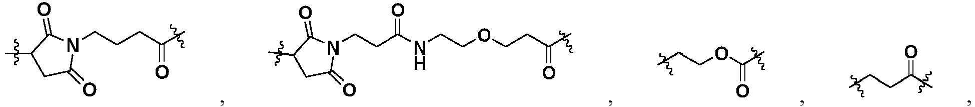

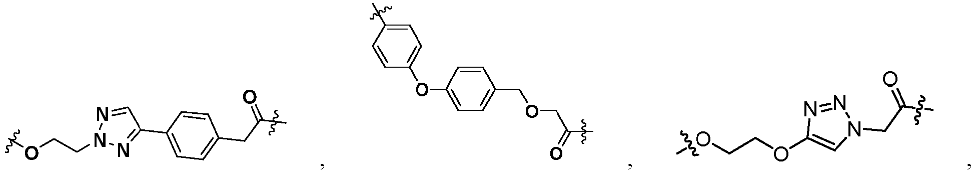

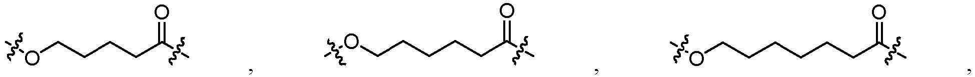

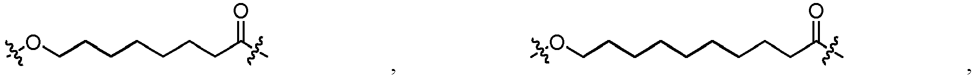

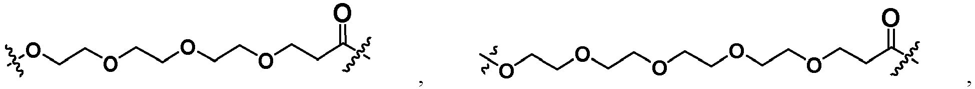

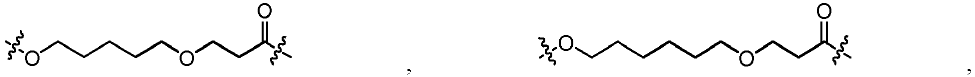

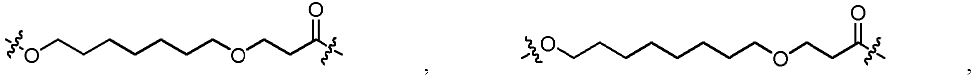

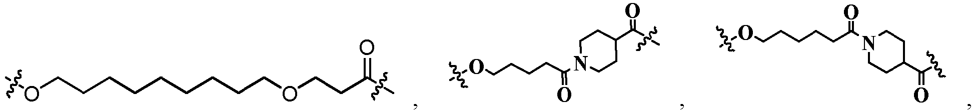

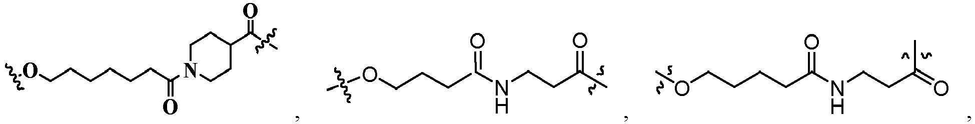

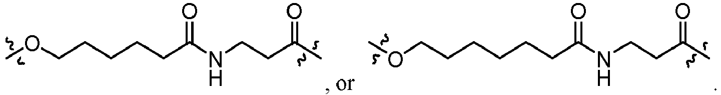

In the most preferred examples of the present invention, the L is 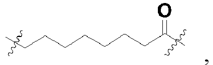 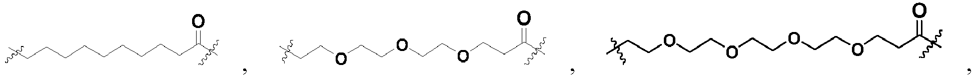 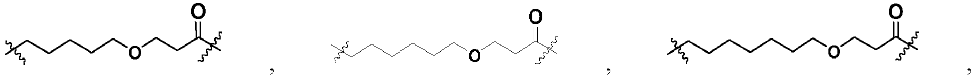 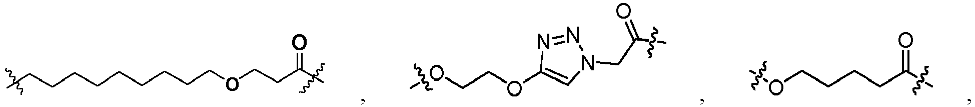 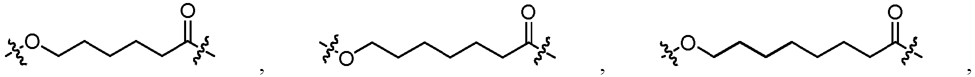 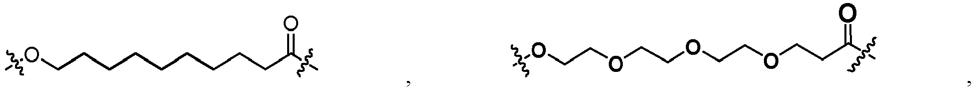 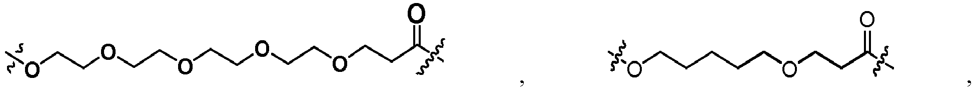 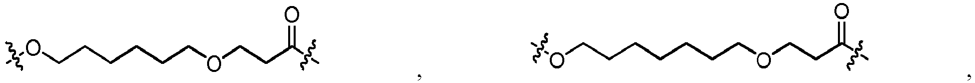 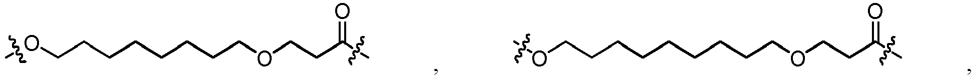 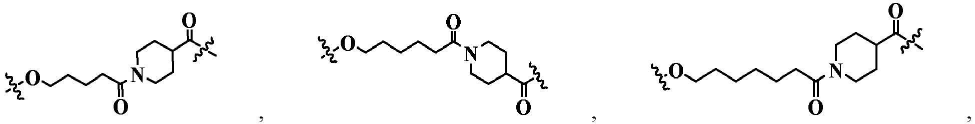 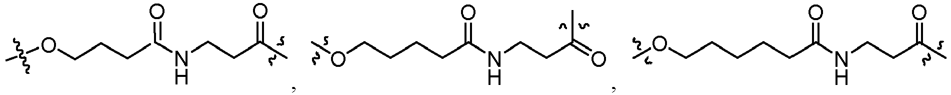 or

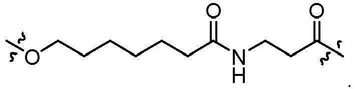

The HPK1 inhibitor moiety has a general formula as follows: 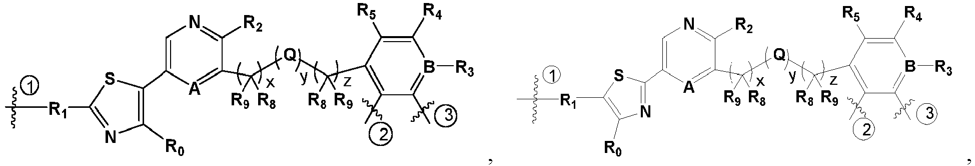 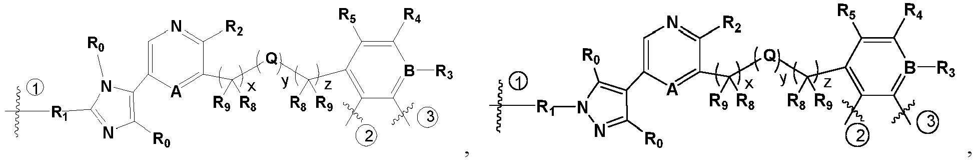 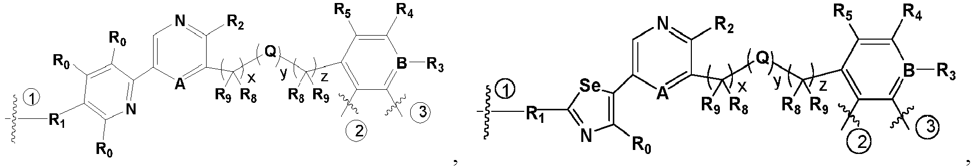 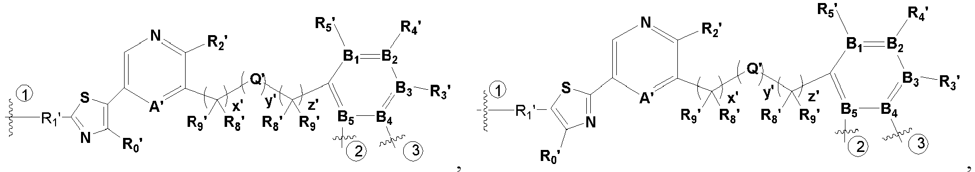 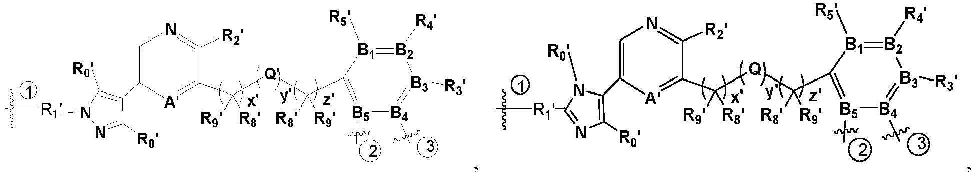 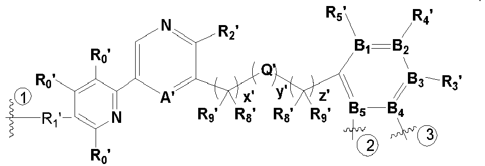 or 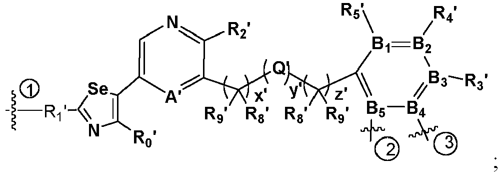 the general formula of HPK1 has 3 attachment sites to Cereblon protein ligands, and can be optionally attached to 3, 2 or 1 Cereblon protein ligand, with the rest attachment sites being replaced with -R"', wherein R'" is selected from -H, halogen, -NO₂, -CN, C₁₋₅ linear/branched alkyl, C₃₋₁₀ cyclohydrocarbyl, -N(C₀₋₁₀ alkyl)(Co-io alkyl), -CF₃, -OCF₃, -OCHF₂, -OCH₂F and -OC₀₋₁₀ alkyl; preferably, R‴ is selected from -H, halogen, C₁₋₅ linear/branched alkyl and -N(C₀₋₁₀ alkyl)(C₀₋₁₀ alkyl); and more preferably, R‴ is selected from -H and halogen.

Preferably, a position ①, a position ② and a position ③ are all attachment sites of the Cereblon protein ligands, or the position ① and the position ②are attachment sites of the Cereblon protein ligands while the position ③ is attached to R‴, or the position ② and the postion ③ are attachment sites of the Cereblon protein ligands while the position ① is attached to R‴, or the position ① and the position ③ are attachment sites of the Cereblon protein ligands while the position ② is attached to R‴, or the position ① is a attachment site of the Cereblon protein ligand while the position ② and the position ③ are attached to R‴, or the position ② is a attachment site of the Cereblon protein ligand while the position ① and the position ③ are attached to R‴, or the position ③ is a attachment site of the Cereblon protein ligand while the position ① and the position ② are attached to R‴.

Most preferably, the attachment sites of the position ① the position ② and the position ③ are all attached to the Cereblon protein ligands.

For the general formula of the HPK1 inhibitor described above, A is selected from C and N; B is selected from C and N.

Q is selected from O and S; x and z are independently selected from integers between 0 and 6; and y is selected from 0 and 1.

Preferably, x and z are independently selected from integers between 0 and 2, such as 0, 1 or 2.

Preferably, the HPK1 inhibitor moiety has a structural formula as follows:

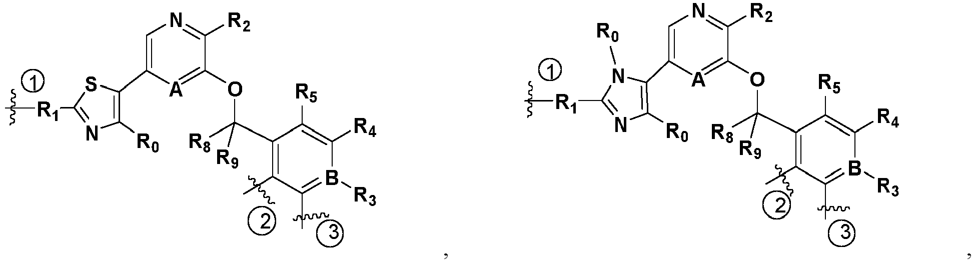

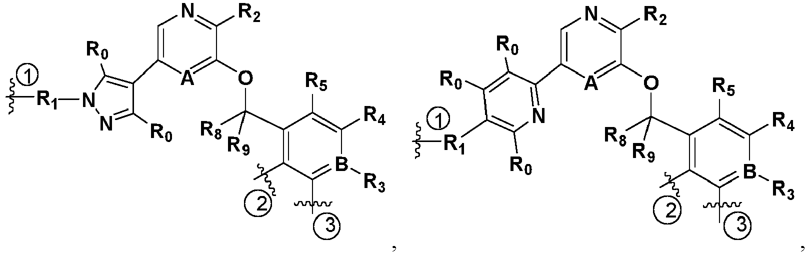

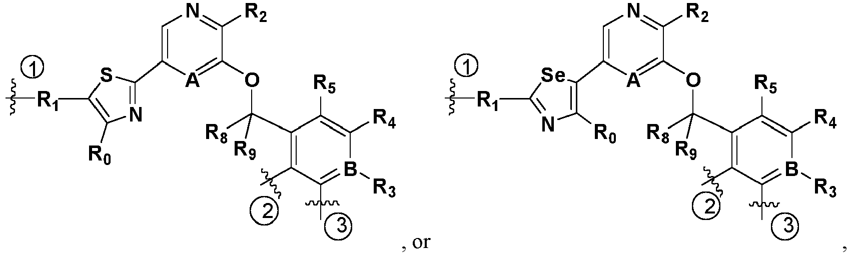

R₀ is independently selected from: -H, C₁₋₁₀ linear/branched alkyl, -N(C₀₋₁₀ alkyl)(C₀₋₁₀ alkyl), -OC₀₋₁₀ alkyl and C₃₋₁₀ cyclohydrocarbyl.

Preferably, R₀ is independently selected from: C₁₋₅ linear/branched alkyl and -N(C₀₋₁₀ alkyl)(C₀₋₁₀ alkyl).

In a preferred embodiment of the present invention, R₀ is independently selected from: -CH₃, -CH₂CH₃ and -NH₂.

R₁ is selected from -O heterocycloalkyl, -N heterocycloalkyl, C₁₋₁₀ linear/branched alkyl, C₃₋₁₀ cycloalkyl, -OC₀₋₁₀ alkyl, -N(C₀₋₁₀ alkyl)(Co-io alkyl), -SO₂(C₀₋₁₀ alkyl), -O(C₀₋₁₀ alkyl), -O-phenyl, -S(C₀₋₁₀ alkyl), -N heterocycloaryl, -O heterocycloaryl and -S heterocycloaryl, wherein H attached to a C atom or heteroatom may be substituted with C₁₋₃ linear alkyl, -N(C₀₋₁₀ alkyl)(C₀₋₁₀ alkyl) or -CF₃.

Preferably, R₁ is selected from: -O heterocycloalkyl and -N heterocycloalkyl, -SO₂(C₀₋₃ alkyl), -O-phenyl, -S(C₀₋₄ alkyl), C₃₋₆ cycloalkyl and C₃₋₅ linear/branched alkyl, wherein H attached to a C atom or heteroatom may be substituted with -CH₃, -NH₂ or -CF₃.

In a preferred embodiment of the present invention, R₁ is selected from:

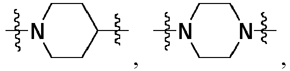

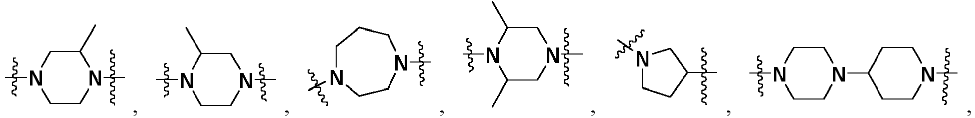

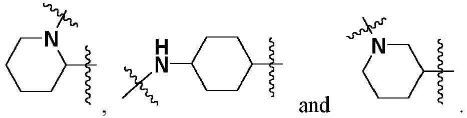

R₂ is selected from: -H, halogen, -NO₂, -CN, C₁₋₅ linear/branched alkyl, C₃₋₁₀ cyclohydrocarbyl, -N(C₀₋₁₀ alkyl)(C₀₋₁₀ alkyl), -CF₃, -OCF₃, -OCHF₂, -OCH₂F and -OC₀₋₁₀ alkyl.

Preferably, R₂ is selected from: -NO₂, -N(C₀₋₁₀alkyl)(C₀₋₁₀ alkyl), -OC₀₋₁₀ alkyl and -OCF₃.

In a preferred embodiment of the present invention, R₂ is selected from: -NH₂ and -NO₂.

When B is N, R₃ is absent; and when B is C, R₃ is selected from: -H, halogen, -OC₀₋₁₀ alkyl, C₁₋₁₀ linear/branched alkyl, -N(C₀₋₁₀alkyl)(C₀₋₁₀ alkyl) and C₃₋₁₀ cyclohydrocarbyl.

Preferably, R₃ is selected from: -H, halogen, -OC₀₋₁₀ alkyl and C₁₋₁₀ linear/branched alkyl.

In a preferred embodiment of the present invention, R₃ is selected from: -H, -F and -OCH₃.

R₄ is selected from: -H, halogen, -OC₀₋₁₀ alkyl, -CN, C₃₋₁₀ cyclohydrocarbyl, -C≡C-R₁₀, C₁₋₁₀ linear/branched alkyl, -N(C₀₋₁₀alkyl)(C₀₋₁₀ alkyl), -O heterocyclohydrocarbyl and -N heterocyclohydrocarbyl.

Preferably, R₄ is selected from: -H, halogen, -OC₀₋₁₀ alkyl, -CN, C₃₋₁₀ cycloalkyl and -C≡C-R₁₀,

In a preferred embodiment of the present invention, R₄ is selected from: -H, -F, -Cl, -OCH₃, -CN, -C≡C-R₁₀, 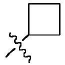 and

R₁₀ is selected from: H, C₁₋₅ linear/branched alkyl, C₃₋₁₀ cyclohydrocarbyl and

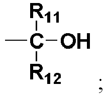

R₁₁ and R₁₂ are independently selected from: -H, -CF₃, -CHF₂H, -CH₂F, C₁₋₁₀ linear/branched alkyl, -CH=C(C₀₋₁₀ alkyl)(Co-io alkyl), -C=C(C₀₋₁₀ alkyl), C₃₋₁₀ cycloalkyl, an aromatic five-membered cyclic group and an aromatic six-membered cyclic group, or R₁₁ and R₁₂, together with carbon atoms between R₁₁ and R₁₂, form C₃₋₈ cycloalkyl or C₃₋₈ heterocycloalkyl containing -O and -S, C₄₋₉ fused cycloalkyl, C₃₋₇ cyclolactam, C₃₋₇ cyclic lactone, or C₃₋₇ cyclic ketone, wherein H attached to a C atom may be substituted with alkyl or halogen.

Preferably, R₁₁ and R₁₂ are independently selected from: -H, -CF₃, -CHF₂H, -CH₂F, C₁₋₁₀ linear/branched alkyl, -CH=C(C₀₋₁₀ alkyl)(Co-io alkyl), C₃₋₁₀ cycloalkyl and an aromatic six-membered cyclic group, or R₁₁ and R₁₂, together with carbon atoms between R₁₁ and R₁₂, form C₃₋₈ cycloalkyl, C₄₋₇ fused cycloalkyl, C₃₋₇ cyclolactam, C₃₋₇ cyclic lactone, or C₃₋₇ cyclic ketone, wherein H attached to a C atom may be substituted with alkyl or -F.

More preferably, R₁₁ and R₁₂ are independently selected from: -H, -CF₃, -CHF₂H, -CH₂F, C₁₋₅ linear/branched alkyl, -CH=CH(C₀₋₁₀ alkyl), C₃₋₁₀ cycloalkyl and an aromatic six-membered cyclic group, or R₁₁ and R₁₂, together with carbon atoms between R₁₁ and R₁₂, form C₃₋₆ cycloalkyl, C₄₋₆ fused cycloalkyl, C₃₋₇ cyclolactam, C₃₋₇ cyclic lactone, or C₃₋₇ cyclic ketone, wherein H attached to a C atom may be substituted with alkyl or -F. In a preferred embodiment of the present invention, R₁₁ and R₁₂ are independently selected from: -H, -CF₃, -CHF₂, -CH₂F, -CH₃, -CH₂CH₃, -CH=CH₂, 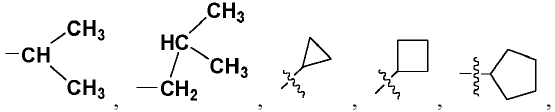 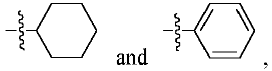 or R₁₁ and R₁₂, together with carbon atoms between R₁₁ and R₁₂, form 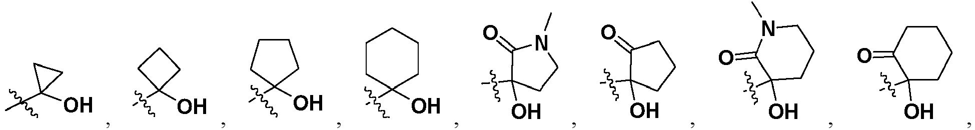 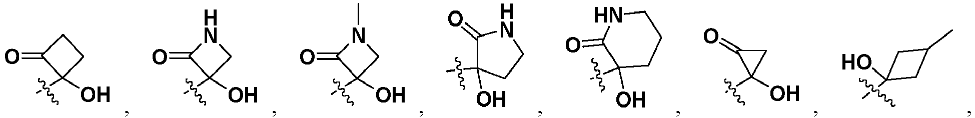 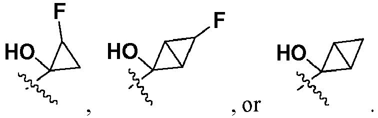 R₅ is selected from: -H, halogen, -CN, -OC₀₋₁₀ alkyl, C₁₋₁₀ linear/branched alkyl, heteroalkyl containing O and N, -N(C₀₋₁₀alkyl)(C₀₋₁₀ alkyl), C₃₋₁₀ cyclohydrocarbyl, -C=C-R₁₀, -O heterocyclohydrocarbyl and -N heterocyclohydrocarbyl, wherein H on a C atom can be substituted with the following groups: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, C₁₋₁₀ linear/branched alkyl, -N(C₀₋₁₀ alkyl)(C₀₋₁₀ alkyl), -OC₀₋₁₀ alkyl, C₃₋₁₀ cyclohydrocarbyl, -O heterocycloalkyl, -N heterocycloalkyl, -N heterocycloaryl, -O heterocycloaryl and -S heterocycloaryl.

Preferably, R₅ is selected from: -H, halogen, C₃₋₆ cycloalkyl, -OC₀₋₅ alkyl, C₁₋₅ linear/branched alkyl and C₁₋₅ linear/branched alkyl containing O and N, wherein H on a C atom can be substituted with -F.

More preferably, R₅ is selected from: -H, halogen, -OC₀₋₃ alkyl, C₁₋₃ linear/branched alkyl and C₁₋₃ linear/branched alkyl containing N, wherein H on a C atom can be substituted with -F.

In a preferred embodiment of the present invention, R₅ is selected from: -H, -F, -Cl, -CH₃, -CH₂NH₂, -CN and -OCH₃.

R₈ and R₉ are independently selected from: -H, halogen and C₁₋₁₀ linear/branched alkyl.

Preferably, R₈ and R₉ are independently selected from: -H and C₁₋₁₀ linear/branched alkyl.

More preferably, R₈ and R₉ are independently selected from: -H and C₁₋₃ linear/branched alkyl.

In a preferred embodiment of the present invention, R₈ and R₉ are independently selected from: -H and -CH₃. A' is selected from C and N; B₁, B₂, B₃, B₄ or B₅ is independently selected from C and N.

Preferably, B₁, B₂, B₃, B₄ or B₅ is C, or at least one of B₁, B₂, B₃, B₄ and B₅ is N; and
more preferably, B₂ is C, and at least one of B₁, B₃, B₄ and B₅ is N. In one embodiment of the present invention, B₂ is C, and B₁ is N; B₂ is C, and B₃ is N; B₂ is C, and B₄ is N; or B₂ is C, and B₅ is N. In another embodiment of the present invention, B₂ is C, B₃ and B₄ are N, or B₃ and B₅ are N.

Q' is selected from O and S; x' and z' are independently selected from integers between 0 and 6; and y' is selected from 0 and 1; and
preferably, x' and z' are independently selected from integers between 0 and 2, such as 0, 1 or 2.

Preferably, the HPK1 inhibitor moiety has a structural formula as follows:

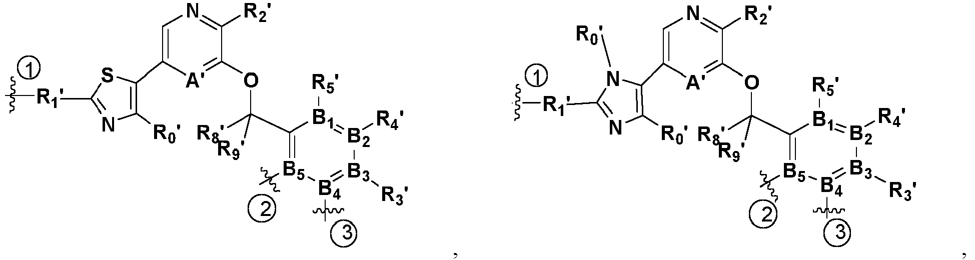

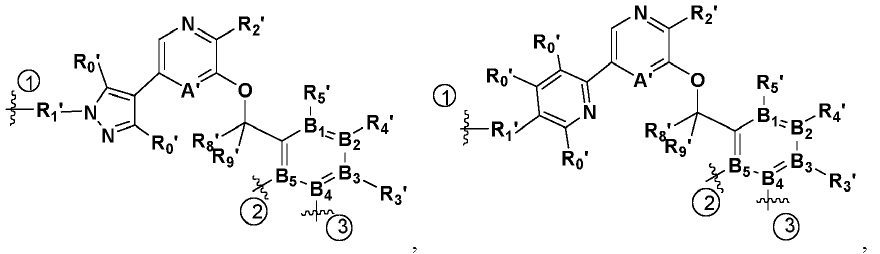

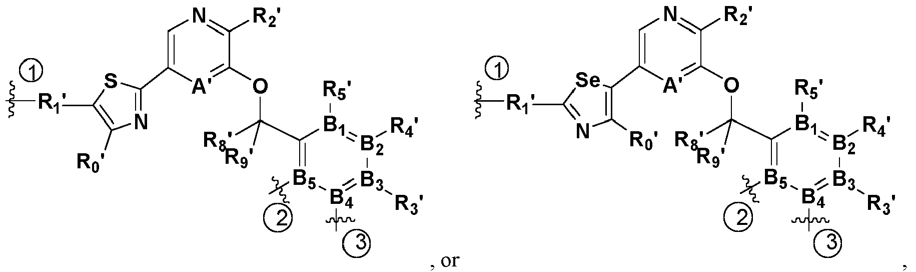

R₀' is independently selected from: -H, C₁₋₁₀ linear/branched alkyl, -N(C₀₋₁₀alkyl)(C₀₋₁₀ alkyl), -OC₀₋₁₀ alkyl and C₃₋₁₀ cyclohydrocarbyl.

Preferably, R₀' is independently selected from: C₁₋₅ linear/branched alkyl and -N(C₀₋₁₀alkyl)(C₀₋₁₀ alkyl).

In a preferred embodiment of the present invention, R₀' is independently selected from: -CH₃, -CH₂CH₃ and -NH₂.

R₁' is selected from: -O heterocycloalkyl, -N heterocycloalkyl, C₁₋₁₀ linear/branched alkyl, C₃₋₁₀ cyclohydrocarbyl, -OC₀₋₁₀ alkyl, -N(C₀₋₁₀alkyl)(C₀₋₁₀ alkyl), -SO₂(C₀₋₁₀ alkyl), -O(C₀₋₁₀ alkyl), -O-phenyl, -S(Co-io alkyl), -N heterocycloaryl, -O heterocycloaryl and -S heterocycloaryl, wherein H on a C atom or heteroatom can be substituted with C₁₋₃ linear alkyl, -N(C₀₋₁₀alkyl)(C₀₋₁₀ alkyl) or -CF₃.

Preferably, R₁' is selected from: -O heterocycloalkyl and -N heterocycloalkyl, -SO₂(C₀₋₃ alkyl), -O-phenyl, -S(C₀₋₄ alkyl), C₃₋₆ cycloalkyl and C₃₋₅ linear/branched alkyl, wherein H on a C atom or heteroatom can be substituted with -CH₃, -NH₂ or -CF₃.

In a preferred embodiment of the present invention, R₁' is selected from:

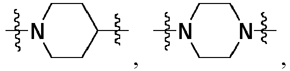

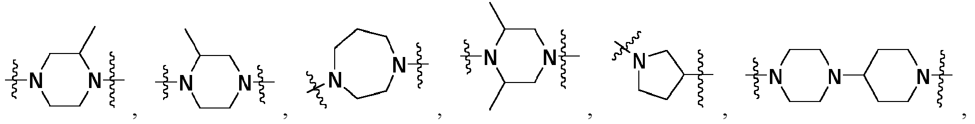

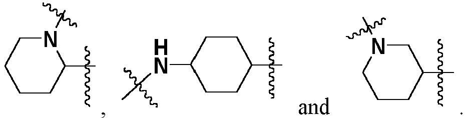

R₂' is selected from: -H, halogen, -NO₂, -CN, C₁₋₅ linear/branched alkyl, C₃₋₁₀ cyclohydrocarbyl, -N(C₀₋₁₀ alkyl)(C₀₋₁₀ alkyl), -CF₃, -OCF₃, -OCHF₂, -OCH₂F and -OC₀₋₁₀ alkyl.

Preferably, R₂' is selected from: -NO₂, -N(C₀₋₁₀alkyl)(C₀₋₁₀ alkyl), -OC₀₋₁₀ alkyl and -OCF₃.

In a preferred embodiment of the present invention, R₂' is selected from: -NH₂ and -NO₂.

When B₁, B₂, B₃, B₄ or B₅ is N, the corresponding R₃', R₄' or R₅' is absent; when B₁, B₂, B₃, B₄ or B₅ is C, R₃', R₄' and R₅' are independently selected from: -H, halogen, -CN, -OC₀₋₁₀ alkyl, C₁₋₁₀ linear/branched alkyl, heteroalkyl containing O and N, -N(C₀₋₁₀alkyl)(C₀₋₁₀ alkyl), C₃₋₁₀ cyclohydrocarbyl, -C≡C-R₁₀', -O heterocyclohydrocarbyl and -N heterocyclohydrocarbyl, or R₅' and R₄', or R₄' and R₃', together with carbon atoms therebetween, form C₃₋₈ cyclohydrocarbyl or C₃₋₈ heterocycloalkyl containing -O- and -S-, -N heterocycloaryl, -O heterocycloaryl or -S heterocycloaryl, or phenyl, wherein H attached to a C atom can be substituted with the following groups: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, C₁₋₁₀ linear/branched alkyl, -N(C₀₋₁₀ alkyl)(C₀₋₁₀ alkyl), -OC₀₋₁₀ alkyl, C₃₋₁₀ cyclohydrocarbyl, -O heterocycloalkyl, -N heterocycloalkyl, -N heterocycloaryl, -O heterocycloaryl and -S heterocycloaryl.

Preferably, R₃' is selected from: -H, halogen, -OC₀₋₁₀ alkyl, C₁₋₁₀ linear/branched alkyl, -N(Co-io alkyl)(C₀₋₁₀ alkyl) and C₃₋₁₀ cycloalkyl.

More preferably, R₃' is selected from: -H, halogen, -OC₀₋₁₀ alkyl and C₁₋₁₀ linear/branched alkyl.

In a preferred embodiment of the present invention, R₃' is selected from: -H, -F and -OCH₃.

Preferably, R₄' is selected from: -H, halogen, -OC₀₋₁₀ alkyl, -CN, C₃₋₁₀ cycloalkyl, -C≡C-R₁₀', C₁₋₁₀ linear/branched alkyl, -N(C₀₋₁₀alkyl)(C₀₋₁₀ alkyl), -O heterocycloalkyl and -N heterocycloalkyl.

More preferably, R₄' is selected from: -H, halogen, -OC₀₋₁₀ alkyl, -CN, C₃₋₁₀ cycloalkyl and -C≡C-R₁₀',

In a preferred embodiment of the present invention, R₄' is selected from: -H, -F, -Cl, -OCH₃, -CN, -C≡C-R₁₀', 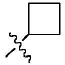 and

Preferably, R₅' is independently selected from: -H, halogen, -CN, -OC₀₋₁₀ alkyl, C₁₋₁₀ linear/branched alkyl, -N(C₀₋₁₀alkyl)(Co-io alkyl), C₃₋₁₀ cycloalkyl, -C≡C-R₁₀', -O heterocycloalkyl and -N heterocycloalkyl, and C₁₋₅ linear/branched alkyl containing O and N, wherein H attached to a C atom may be substituted with -F.

More preferably, R₅' is independently selected from: -H, halogen, C₁₋₃ linear/branched alkyl, -OC₀₋₃ alkyl and C₁₋₃ linear/branched alkyl containing N, wherein H attached to a C atom may be substituted with -F.

In a preferred embodiment of the present invention, R₅' is selected from: -H, -F, -Cl, -CH₃, -CH₂NH₂, -CN and -OCH₃.

R₁₀' is selected from: H, C₁₋₅ linear/branched alkyl, C₃₋₁₀ cyclohydrocarbyl and 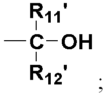 R₁₁' and R₁₂' are independently selected from: -H, -CF₃, -CHF₂H, -CH₂F, C₁₋₁₀ linear/branched alkyl, -CH=C(C₀₋₁₀ alkyl)(C₀₋₁₀ alkyl), -C≡C(C₀₋₁₀ alkyl), C₃₋₁₀ cyclohydrocarbyl, an aromatic five-membered cyclic group and an aromatic six-membered cyclic group, or R₁₁' and R₁₂', together with carbon atoms between R₁₁' and R₁₂', form C₃₋₈ cycloalkyl or C₃₋₈ heterocycloalkyl containing -O or -S, C₄₋₉ fused cycloalkyl, C₅₋₁₀ spiro cycloalkyl, C₄₋₉ bridged cycloalkyl, C₃₋₇ cyclolactam, C₃₋₇ cyclic lactone, or C₃₋₇ cyclic ketone, wherein H attached to a C atom may be substituted with the following groups: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, C₁₋₁₀ linear/branched alkyl, -N(C₀₋₁₀ alkyl)(C₀₋₁₀ alkyl), -OC₀₋₁₀ alkyl, C₃₋₁₀ cyclohydrocarbyl, -O heterocycloalkyl, -N heterocycloalkyl, -N heterocycloaryl, -O heterocycloaryl and -S heterocycloaryl, wherein the alkyl moieties may be optionally substituted with one or more of the following groups: -SO₂, -SO₂N(C₀₋₁₀ alkyl)(Co-io alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, -N(C₀₋₁₀ alkyl)(C₀₋₁₀ alkyl), -OC₀₋₁₀ alkyl, -N heterocycloaryl, -O heterocycloaryl and -S heterocycloaryl.

Preferably, R₁₁' and R₁₂' are independently selected from: -H, -CF₃, -CHF₂H, -CH₂F, C₁₋₁₀ linear/branched alkyl, -CH=C(C₀₋₁₀ alkyl)(C₀₋₁₀ alkyl), C₃₋₁₀ cycloalkyl and an aromatic six-membered cyclic group, or R₁₁' and R₁₂', together with carbon atoms between R₁₁' and R₁₂', form C₃₋₈ cycloalkyl, C₄₋₇ fused cycloalkyl, C₅₋₉ spiro cycloalkyl, C₄₋₉ bridged cycloalkyl, C₃₋₇ cyclolactam, C₃₋₇ cyclic lactone, or C₃₋₇ cyclic ketone, wherein H attached to a C atom may be substituted with the following groups: -SO₂, -SO₂N(C₀₋₁₀ alkyl)(C₀₋₁₀ alkyl), -N(C₀₋₁₀alkyl)SO₂(C₀₋₁₀ alkyl), -CON(C₀₋₁₀ alkyl)(C₀₋₁₀ alkyl), -N(C₀₋₁₀alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, C₁₋₁₀ linear/branched alkyl, -N(C₀₋₁₀alkyl)(C₀₋₁₀ alkyl), -OC₀₋₁₀ alkyl, C₃₋₁₀ cycloalkyl, -O heterocycloalkyl, -N heterocycloalkyl, -N heterocycloaryl, -O heterocycloaryl and -S heterocycloaryl, wherein the alkyl moieties may be optionally substituted with one or more of the following groups: -SO₂, -SO₂N(C₀₋₁₀ alkyl)(Co-io alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, -N(C₀₋₁₀ alkyl)(C₀₋₁₀ alkyl), -OC₀₋₁₀ alkyl, -N heterocycloaryl, -O heterocycloaryl and -S heterocycloaryl.

More preferably, R₁₁' and R₁₂' are independently selected from: -H, -CF₃, -CHF₂H, -CH₂F, C₁₋₅ linear/branched alkyl, -CH=CH(C₀₋₁₀ alkyl), C₃₋₁₀ cycloalkyl and an aromatic six-membered cyclic group, or R₁₁' and R₁₂', together with carbon atoms between R₁₁' and R₁₂', form C₃₋₆ cycloalkyl, C₄₋₆ fused cycloalkyl, C₅₋₈ spiro cycloalkyl, C₄₋₈ bridged cycloalkyl, C₃₋₇ cyclolactam, C₃₋₇ cyclic lactone, or C₃₋₇ cyclic ketone, wherein H attached to a C atom may be substituted with the following groups: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, C₁₋₁₀ linear/branched alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -N heterocycloaryl, -O heterocycloaryl and -S heterocycloaryl, wherein the alkyl moieties may be optionally substituted with one or more of the following groups: -SO₂, -SO₂N(C₀₋₁₀ alkyl)(Co-io alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, -N(C₀₋₁₀ alkyl)(C₀₋₁₀ alkyl), -OC₀₋₁₀ alkyl, -N heterocycloaryl, -O heterocycloaryl and -S heterocycloaryl.

In a preferred embodiment of the present invention, R₁₁' and R₁₂' are independently selected from: -H, -CF₃, -CHF₂, -CH₂F, -CH₃, -CH₂CH₃, -CH=CH₂, 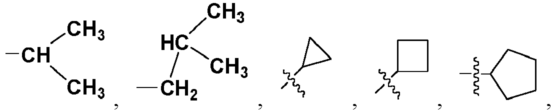 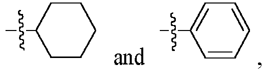 or R₁₁' and R₁₂', together with carbon atoms between R₁₁' and R₁₂', form

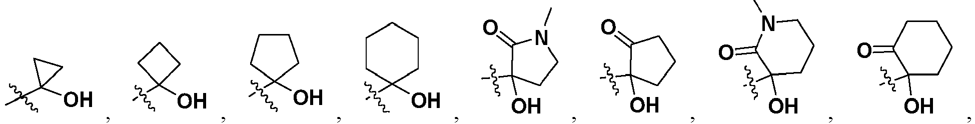

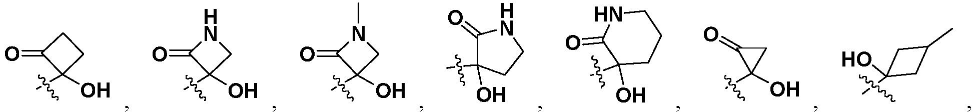

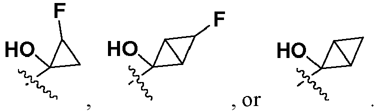

R₈' and R₉' are independently selected from: -H, halogen and C₁₋₁₀ linear/branched alkyl.

Preferably, R₈' and R₉' are independently selected from: -H and C₁₋₁₀ linear/branched alkyl.

More preferably, R₈' and R₉' are independently selected from: -H and C₁₋₃ linear/branched alkyl.

In a preferred embodiment of the present invention, R₈' and R₉' are independently selected from: -H and -CH₃. In a preferred embodiment of the present invention, the HPK1 inhibitor moiety has a specific structure as follows:

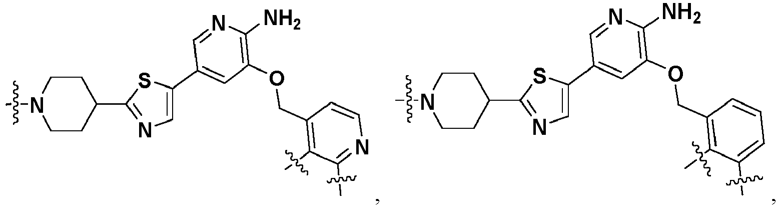

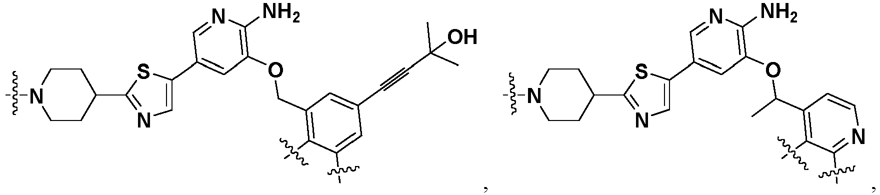

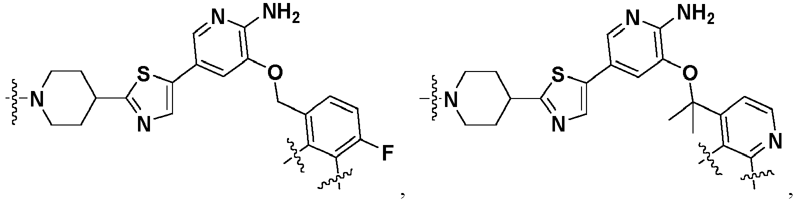

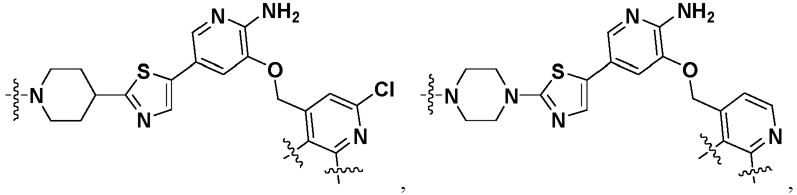

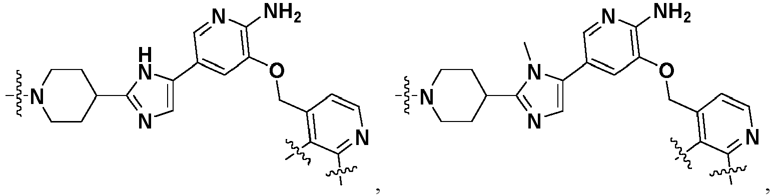

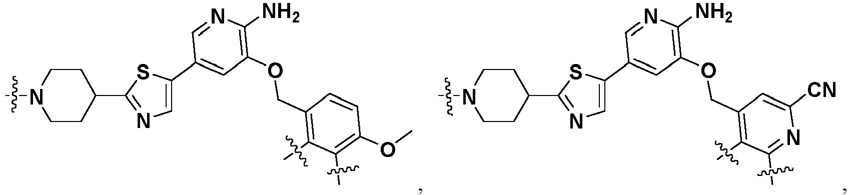

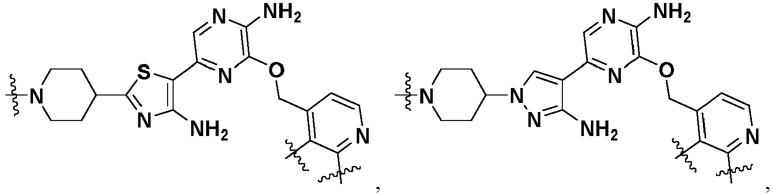

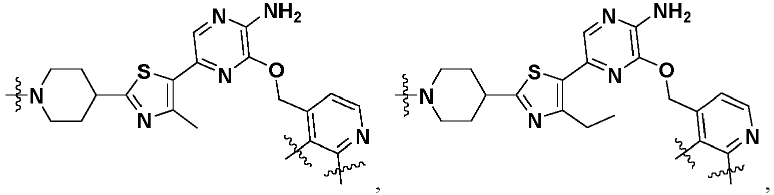

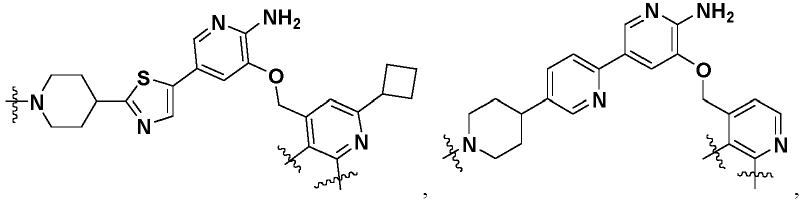

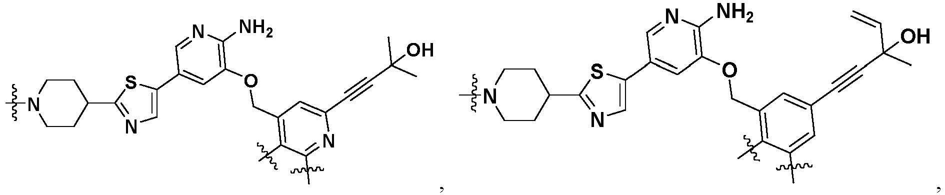

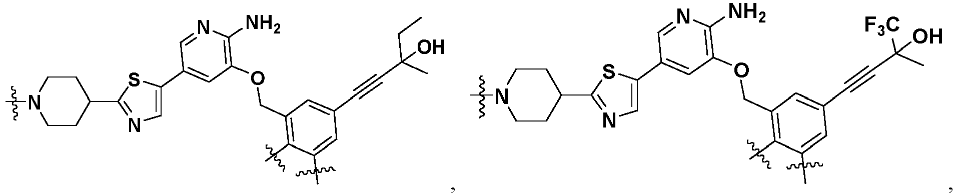

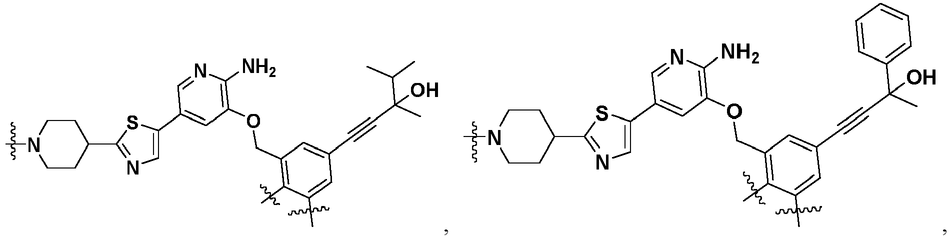

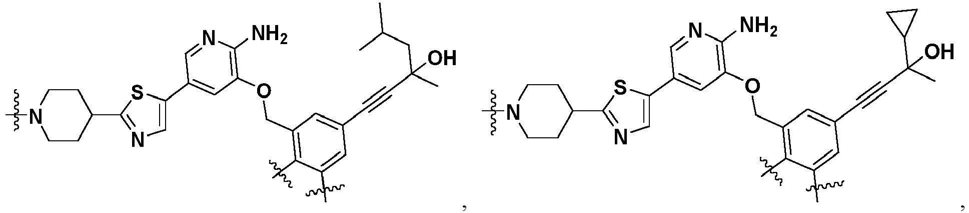

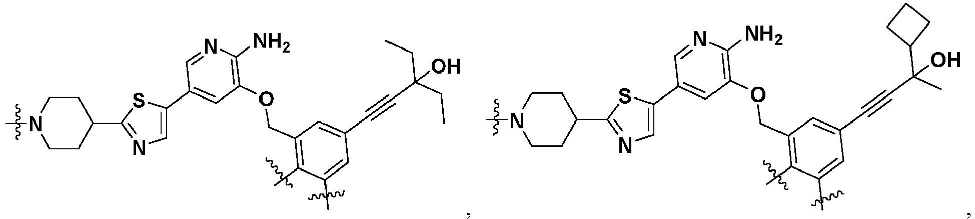

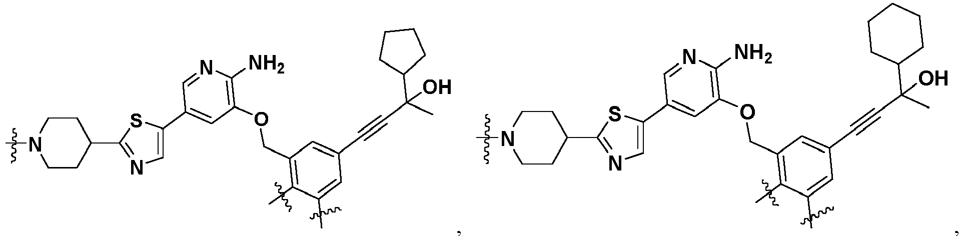

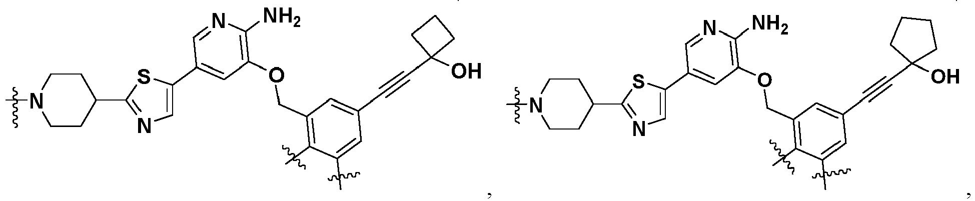

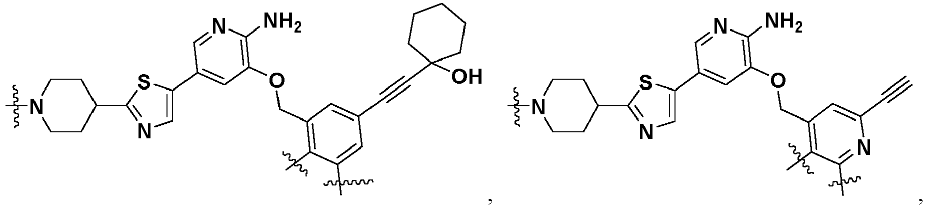

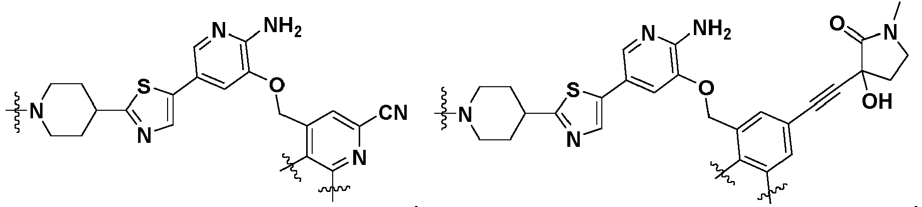

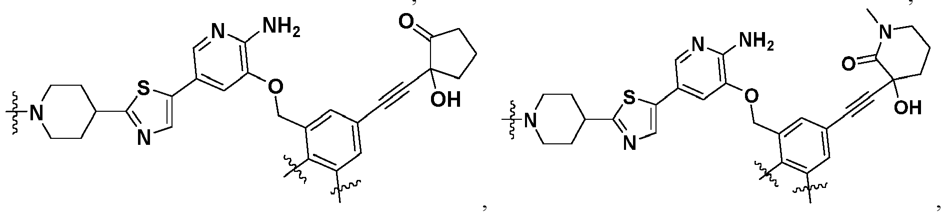

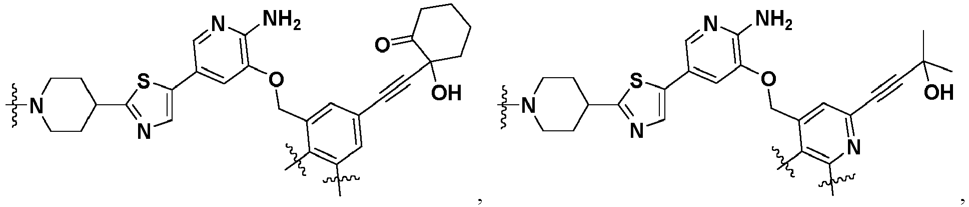

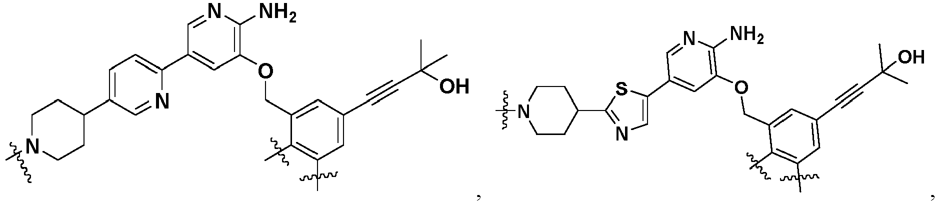

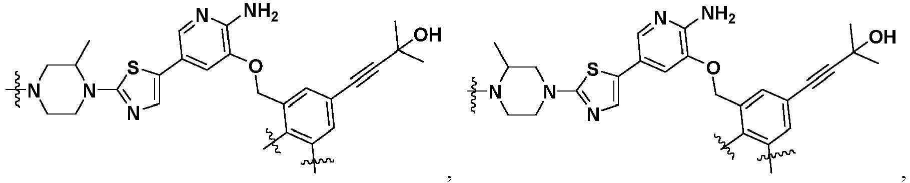

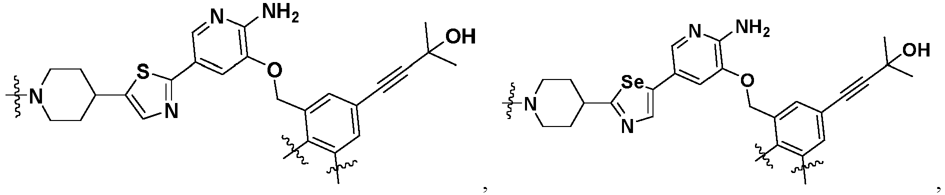

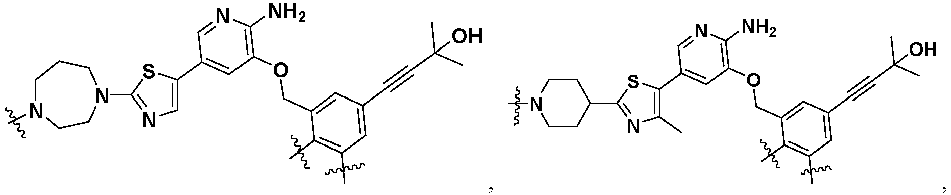

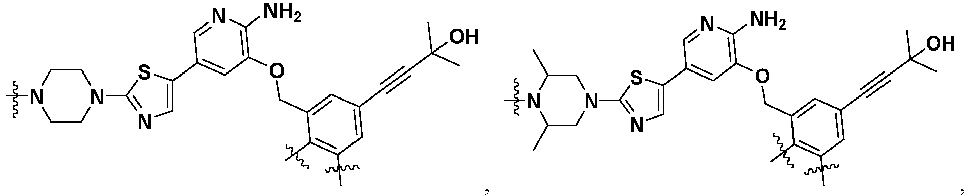

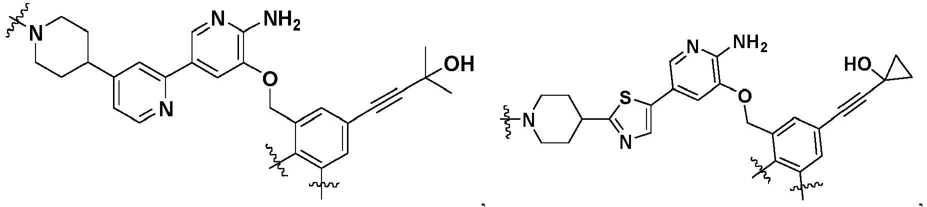

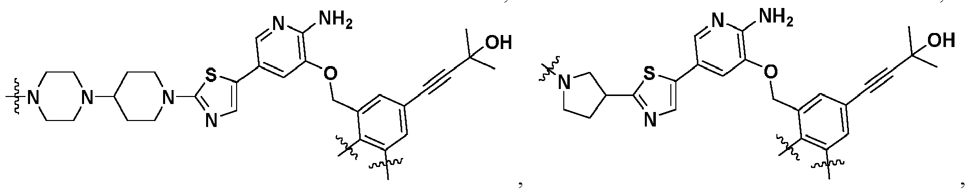

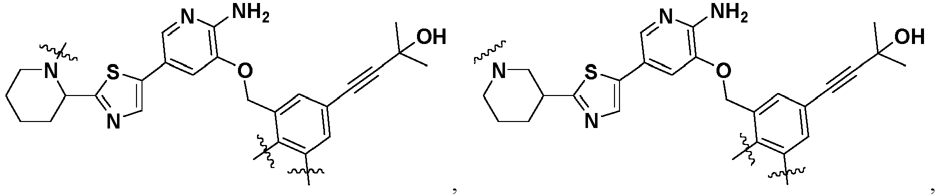

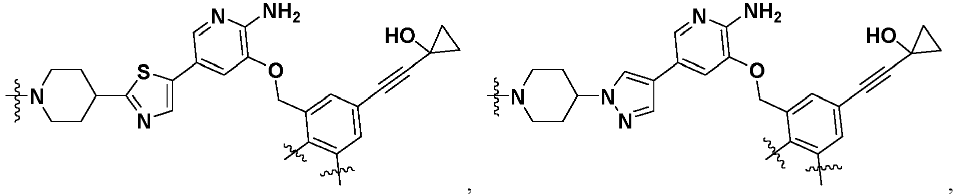

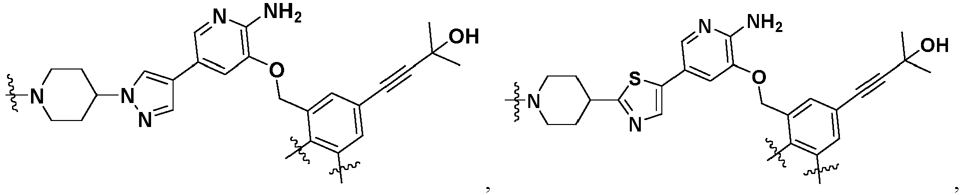

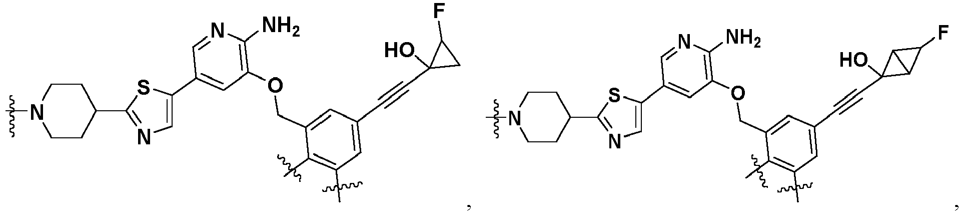

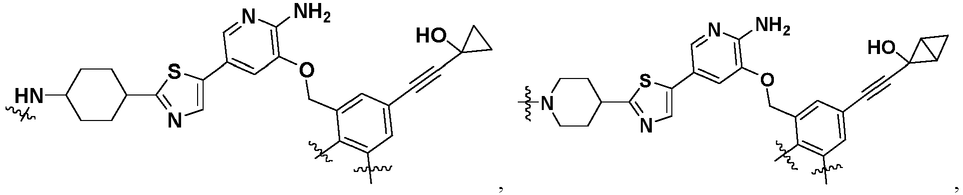

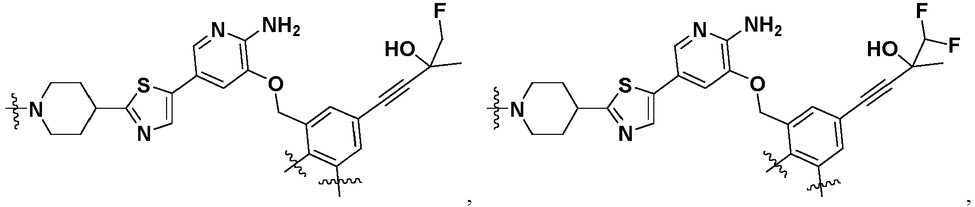

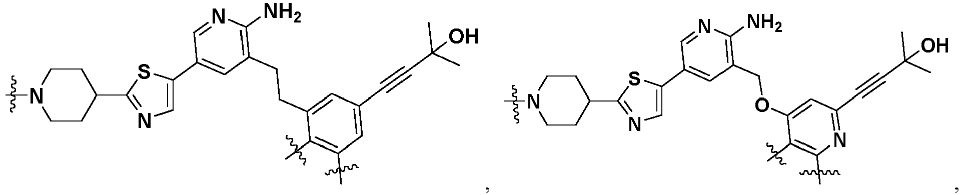

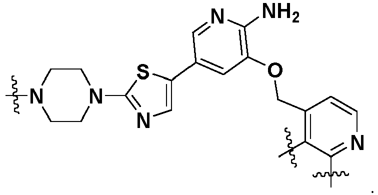

The HPK1 inhibitor described herein further comprises compounds disclosed in the prior art, comprising the following general formulas: 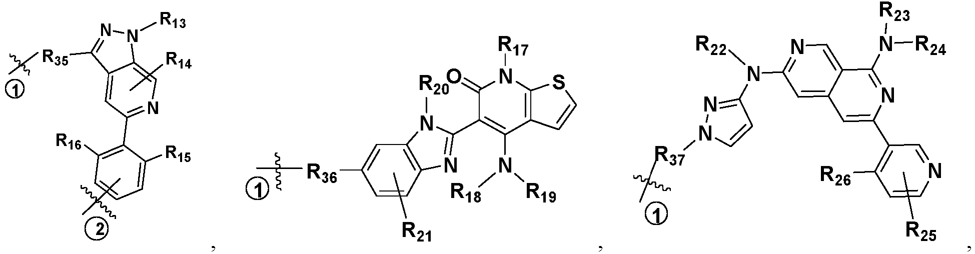 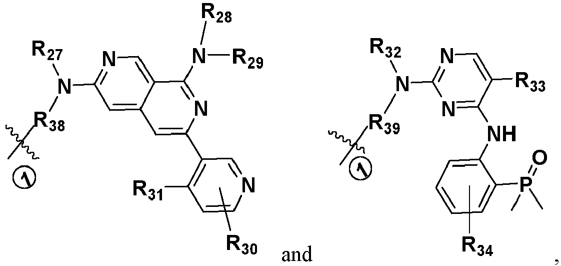 wherein R₃₅, R₃₆, R₃₈ and R₃₉ are selected from C₁₋₁₀ linear/branched alkyl, -CON(C₀₋₁₀ alkyl)-, -CO(C₀₋₁₀ alkyl)-, C₃₋₁₀ cyclohydrocarbyl, -O heterocycloalkyl, -N heterocycloalkyl, phenyl, -N heterocycloaryl and -O heterocycloaryl, and R₃₇, together with an N atom attached thereto and a C atom adjacent to the N atom, forms 5-8 membered cyclolactam.

Preferably, R₃₅, R₃₆, R₃₈ and R₃₉ are selected from -CO(C₀₋₁₀ alkyl)-, -N heterocycloalkyl, phenyl and -N heterocycloaryl, and R₃₇, together with an N atom attached thereto and a C atom adjacent to the N atom, forms 7-membered cyclolactam.

More preferably, the HPK1 inhibitor moiety has a general formula as follows: 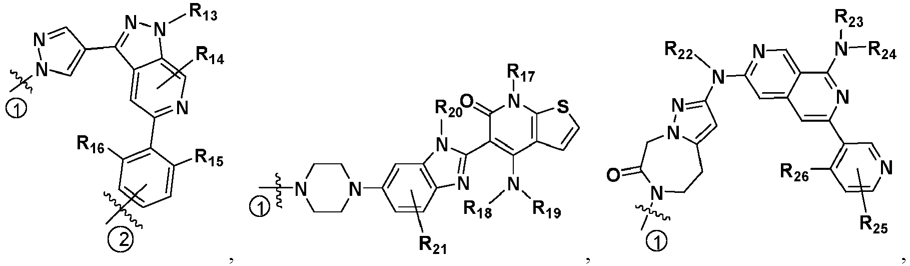 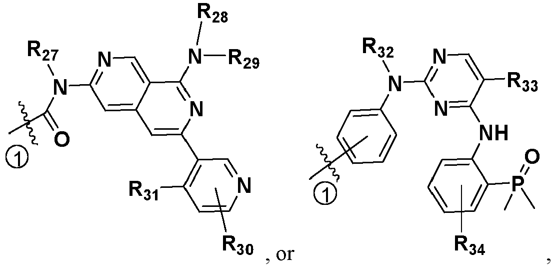 wherein a position ① and a position ② are attachment sites of the Cereblon protein ligands, or, the position ① is an attachment site of the Cereblon protein ligand and the position ② is attached to R‴, or, the position ② is an attachment site of the Cereblon protein ligand and the position ① is attached to R‴; R₁₃-R₃₄ are independently selected from: -H, halogen, -CN, -OC₀₋₁₀ alkyl, C₁₋₁₀ linear/branched alkyl, -N(C₀₋₁₀ alkyl)(C₀₋₁₀ alkyl), C₃₋₁₀ cyclohydrocarbyl, -O heterocycloalkyl, -N heterocycloalkyl, -S heterocycloalkyl, phenyl, -N heterocycloaryl, -O heterocycloaryl and -S heterocycloaryl.

Preferably, R₁₃-R₃₄ are independently selected from: -H, halogen, -CN, C₁₋₄ linear/branched alkyl, -N(C₀₋₁₀ alkyl)(C₀₋₁₀ alkyl) and C₃₋₅ cycloalkyl.

In a preferred embodiment of the present invention, R₁₃-R₃₄ are independently selected from: -H, -F, -Cl, -CN, -CH₃, -CH₂CH₃, -NH₂,

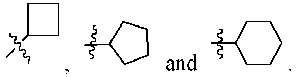

Preferably, the HPK1 inhibitor moiety has a structural formula as follows:

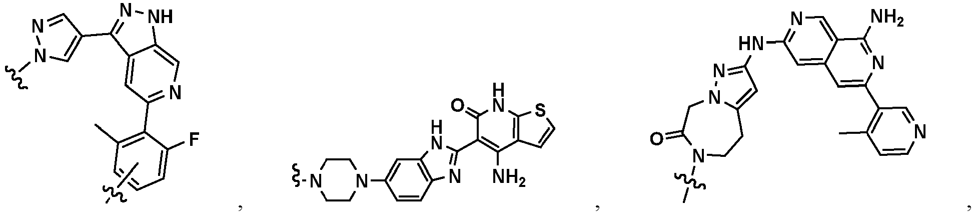

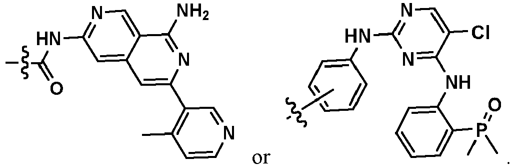

In a preferred embodiment of the present invention, the compound has a specific structural formula as follows: 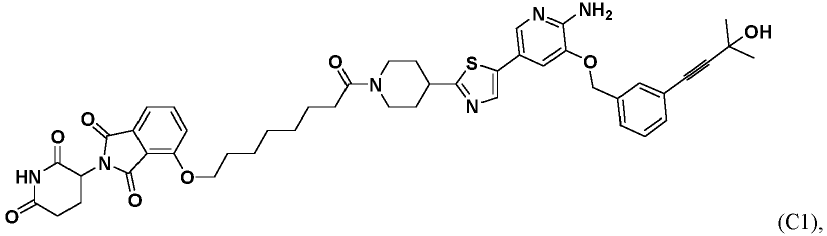 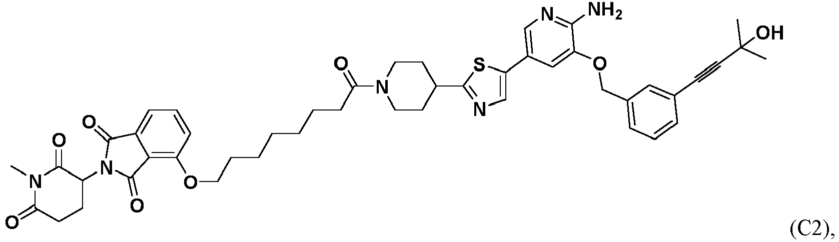 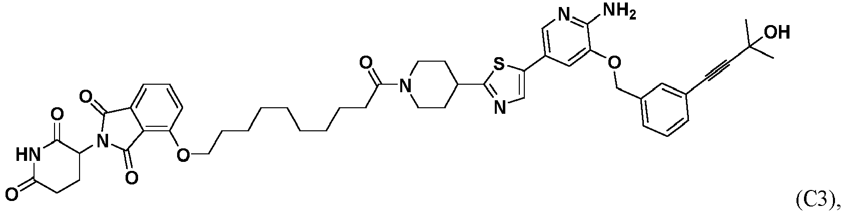 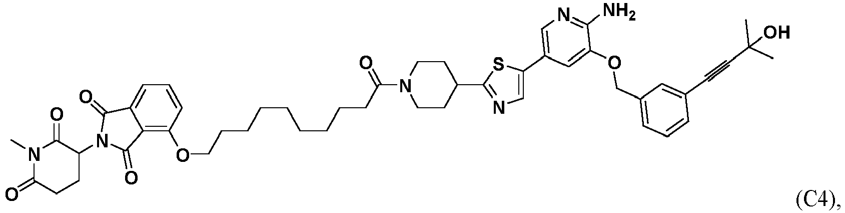 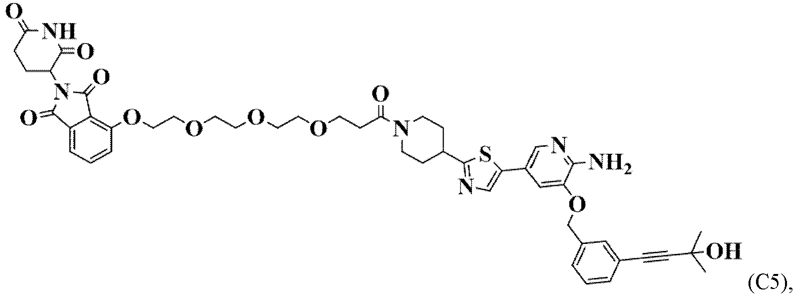 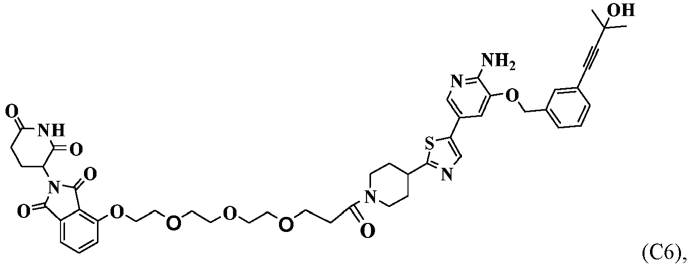 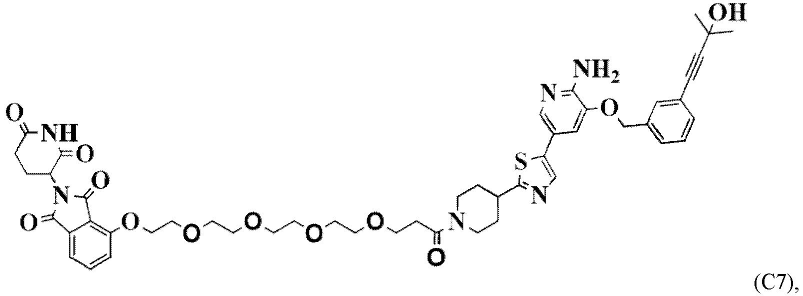 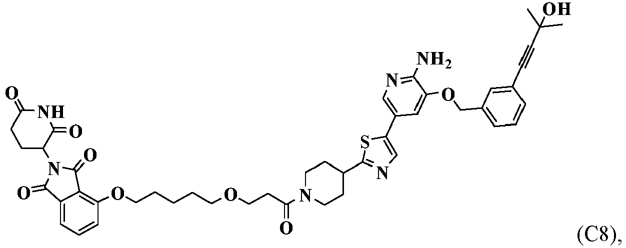 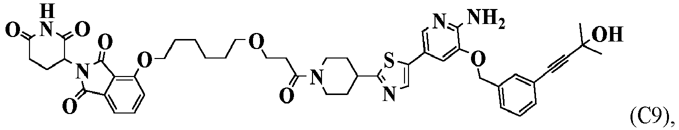 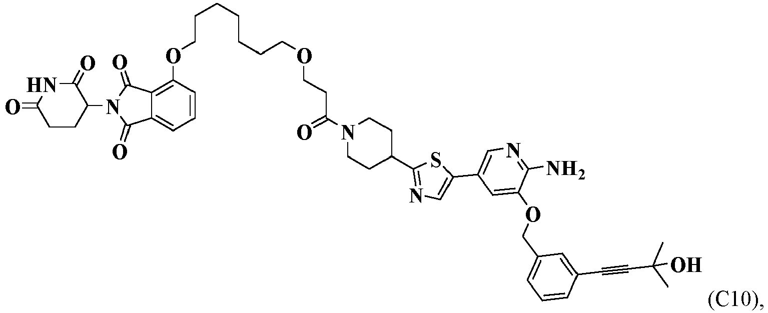 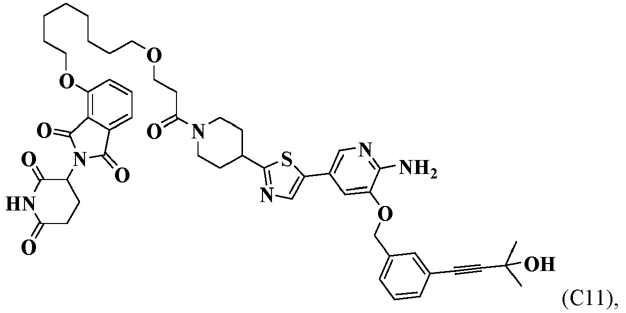 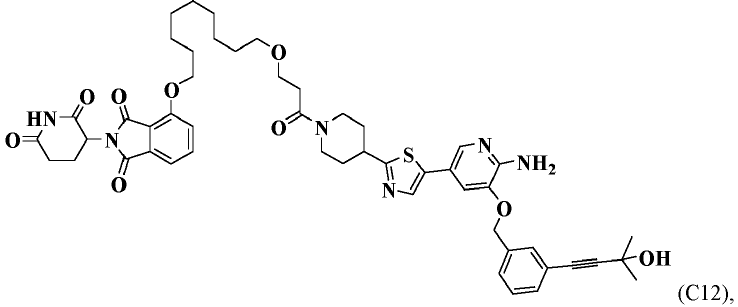 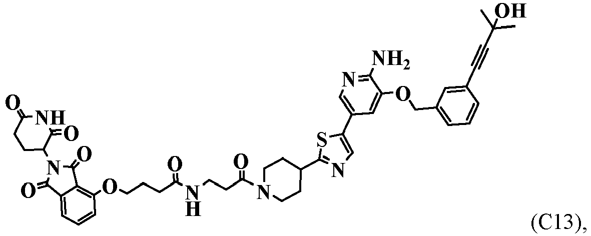 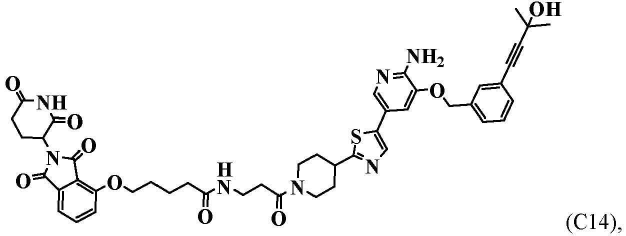 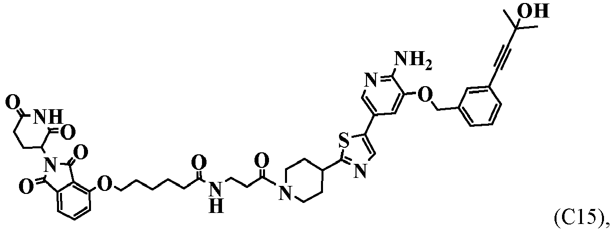 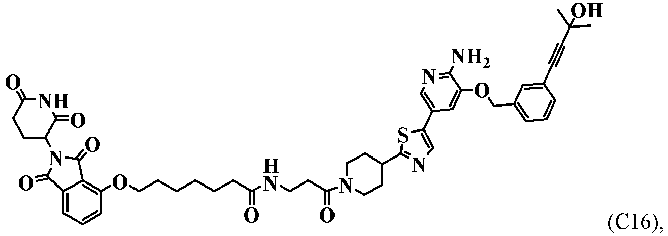 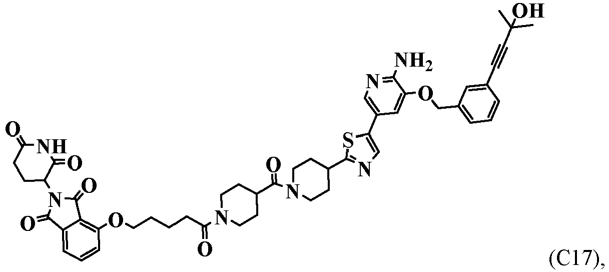 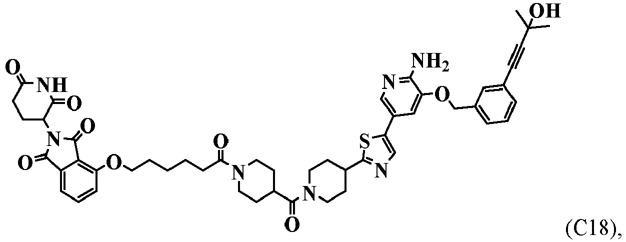 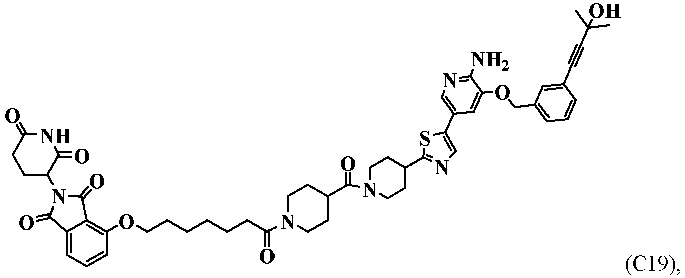 or

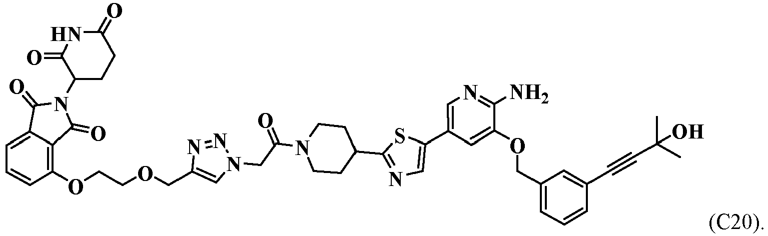

The compound of general formula I disclosed herein further encompasses a pharmaceutically acceptable salt, a stereoisomer, an ester, a prodrug, a solvate and a deuterated compound thereof.

The present invention provides a pharmaceutical composition, which comprises the compound of general formula I, or the pharmaceutically acceptable salt, the stereoisomer, the ester, the prodrug, the solvate and the deuterated compound thereof, and which further comprises a pharmaceutically acceptable excipient, wherein the excipient may be selected from a filler, a binder and a lubricant. Preferably, the pharmaceutical composition comprises a therapeutically effective amount of the compound of general formula I. In certain embodiments, the pharmaceutical composition can be used alone or in combination with other pharmaceutical formulations.

The other pharmaceutical formulations include: PD-1, PD-L1, CTLA-4, TIM-3, TGF-β and receptors thereof, LAG3 antagonists or TLR4, TLR7, TLR8, TLR9, and STING agonists.

The filler is selected from one of or a combination of two or more of lactose, microcrystalline cellulose, methylcellulose, carboxymethylcellulose sodium, ethylcellulose, hydroxypropylcellulose and hydroxypropyl methylcellulose.

The binder is selected from one of or a combination of two or more of starch, dextrin, cellulose derivatives, polyvidone, gelatin, polyethylene glycol, polyvinyl alcohol and sucrose.

The lubricant is selected from one of or a combination of two or more of distilled water, ethanol and starch slurry.

Preferably, the pharmaceutical composition further comprises an antioxidant, a buffer, a bacteriostat, a solute which renders a formulation isotonic with the blood of a subject, and aqueous and non-aqueous sterile suspensions which may comprise a suspending agent, a solubilizer, a thickening agent, a stabilizer and a preservative.

The pharmaceutical composition is suitable for gastrointestinal or parenteral administration, for example by intravenous, intramuscular, intradermal and subcutaneous routes of administration.

The pharmaceutical composition may be prepared into pharmaceutical formulations in the form of: injections, syrups, elixirs, suspensions, powders, granules, tablets, capsules, lozenges, creams, ointments, lotions, gels, emulsions and the like.

In the preparation of injections, any carrier commonly used in the art can be used, such as water, ethanol, propylene glycol, ethoxylated isostearyl alcohol, polyethoxylated isostearyl alcohol, and fatty acid esters of polyethylene sorbitan. In addition, conventional solvents, buffers, and the like can further be added.

The present invention provides use of the compound of general formula I, and the pharmaceutically acceptable salt, the stereoisomer, the ester, the prodrug, the solvate and the deuterated compound thereof for preparing a medicament for inhibiting the serine/threonine kinase family, and preferably for preparing a medicament for inhibiting hematopoietic progenitor kinase 1.

The present invention provides use of the compound of general formula I, and the pharmaceutically acceptable salt, the stereoisomer, the ester, the prodrug, the solvate and the deuterated compound thereof for preparing a medicament for treating a disease associated with the serine/threonine kinase family, and preferably for preparing a medicament for treating a disease associated with hematopoietic progenitor kinase 1 (HPK1).

The disease associated with hematopoietic progenitor kinase 1 (HPK1) is selected from: inflammation, immune disease and cancer.

The immune disease is selected from: lupus erythematosus, glomerulonephritis, rheumatoid arthritis, psoriasis, inflammatory bowel disease and autoimmune diabetes.

The cancer is selected from: lymphoma, blastoma, medulloblastoma, retinoblastoma, sarcoma, liposarcoma, synovial cell sarcoma, neuroendocrine tumor, carcinoid tumor, gastrinoma, islet cell carcinoma, mesothelioma, schwannoma, acoustic neuroma, meningioma, adenocarcinoma, melanoma, leukemia and lymphoid malignancy, squamous cell carcinoma, epithelial squamous cell carcinoma, lung carcinoma, small cell lung carcinoma, non-small cell lung carcinoma, adenocarcinoma lung carcinoma, squamous lung carcinoma, peritoneal carcinoma, hepatocellular carcinoma, gastric carcinoma, intestinal carcinoma, pancreatic carcinoma, glioblastoma, cervical carcinoma, ovarian carcinoma, liver carcinoma, bladder carcinoma, liver carcinoma, breast carcinoma, metastatic breast carcinoma, colon carcinoma, rectal carcinoma, colorectal carcinoma, uterine carcinoma, salivary gland carcinoma, kidney carcinoma, prostate carcinoma, vulval carcinoma, thyroid carcinoma, liver carcinoma, anal carcinoma, penile carcinoma, Merkel cell carcinoma, esophageal carcinoma, biliary tract carcinoma, head and neck carcinoma, and hematological malignancies.

Preferably, the compound of general formula I, and the pharmaceutically acceptable salt, the stereoisomer, the ester, the prodrug, the solvate and the deuterated compound thereof can be used alone or in combination with other pharmaceutical formulations and/or therapeutic methods.

The other pharmaceutical formulations are selected from: PD-1, PD-L1, CTLA-4, TIM-3, TGF-β and receptors thereof, LAG3 antagonists and TLR4, TLR7, TLR8, TLR9, and STING agonists.

The other therapeutic methods are selected from: radiotherapy and immunotherapy.

The present invention provides use of the compound of general formula I, and the pharmaceutically acceptable salt, the stereoisomer, the ester, the prodrug, the solvate and the deuterated compound thereof for preventing and/or treating cancer, immune disease and inflammation.

The present invention provides use of the compound of general formula I, and the pharmaceutically acceptable salt, the stereoisomer, the ester, the prodrug, the solvate and the deuterated compound thereof for preparing a medicament for preventing and/or treating cancer, immune disease and inflammation.

The present invention further provides use of the compound of general formula I, and the pharmaceutically acceptable salt, the stereoisomer, the ester, the prodrug, the solvate and the deuterated compound thereof in cancer immunotherapy in combination with PD-1, PD-L1, CTLA-4, TIM-3, TGF-β and receptors thereof, LAG3 antagonists or TLR4, TLR7, TLR8, TLR9, and STING agonists.

The present invention further provides use of the compound of general formula I, and the pharmaceutically acceptable salt, the stereoisomer, the ester, the prodrug, the solvate and the deuterated compound thereof in cancer immunotherapy in combination with CAR-T immunotherapy.

The CAR-T immunotherapy refers to the chimeric antigen receptor T cell immunotherapy which is one of the effective therapeutic methods for malignant tumors at present, and the basic principle is to eliminate cancer cells by utilizing the patient's own immune cells, which belongs to a cell therapy.

The cancer of the present invention is selected from: lymphoma, blastoma, medulloblastoma, retinoblastoma, sarcoma, liposarcoma, synovial cell sarcoma, neuroendocrine tumor, carcinoid tumor, gastrinoma, islet cell carcinoma, mesothelioma, schwannoma, acoustic neuroma, meningioma, adenocarcinoma, melanoma, leukemia and lymphoid malignancy, squamous cell carcinoma, epithelial squamous cell carcinoma, lung carcinoma, small cell lung carcinoma, non-small cell lung carcinoma, adenocarcinoma lung carcinoma, squamous lung carcinoma, peritoneal carcinoma, hepatocellular carcinoma, gastric carcinoma, intestinal carcinoma, pancreatic carcinoma, glioblastoma, cervical carcinoma, ovarian carcinoma, liver carcinoma, bladder carcinoma, liver carcinoma, breast carcinoma, metastatic breast carcinoma, colon carcinoma, rectal carcinoma, colorectal carcinoma, uterine carcinoma, salivary gland carcinoma, kidney carcinoma, prostate carcinoma, vulval carcinoma, thyroid carcinoma, liver carcinoma, anal carcinoma, penile carcinoma, Merkel cell carcinoma, esophageal carcinoma, biliary tract carcinoma, head and neck carcinoma, and hematological malignancies.

The pharmaceutically acceptable salts of the present invention include acid addition salts and base addition salts.

The acid addition salts include, but are not limited to, salts derived from inorganic acids, such as hydrochloric acid, nitric acid, phosphoric acid, sulfuric acid, hydrobromic acid, hydroiodic acid, and phosphonic acid, and salts derived from organic acids, such as aliphatic mono-carboxylic acid and aliphatic dicarboxylic acid, phenyl-substituted alkanoic acid, hydroxyalkanoic acid, alkanedioic acids, aromatic acid, aliphatic sulfonic acid and aromatic sulfonic acid. Thus, these salts include, but are not limited to, sulfate, pyrosulfate, bisulfate, sulfite, bisulfite, nitrate, phosphate, monohydrogenphosphate, dihydrogenphosphate, metaphosphate, pyrophosphate, hydrochloride, hydrobromide, iodate, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, tosylate, phenylacetate, citrate, lactate, maleate, tartrate, and methanesulfonate, and salts comprising amino acids such as arginate, gluconate and galacturonate. Acid addition salts can be prepared by contacting a free base form with a sufficient amount of the desired acid to form the salt in a conventional manner. The free base form can be regenerated by contacting the salt form with a base and isolating the free base in a conventional manner.

The base addition salts according to the present invention are salts with metals or amines, such as hydroxides of alkali metals and alkaline earth metals, or with organic amines. Examples of metals useful as cations include, but are not limited to, sodium, potassium, magnesium, and calcium. Examples of suitable amines include, but are not limited to, *N*,*N*'-dibenzylethylenediamine, chloroprocaine, choline, diethanolamine, ethylenediamine(ethane-1,2-diamine), *N*-methylglucamine and procaine. Base addition salts can be prepared by contacting the free acid form with a sufficient amount of the desired base to form the salt in a conventional manner. The free acid form can be regenerated by contacting the salt form with an acid and isolating the free acid in a conventional manner.

The stereoisomer described herein includes enantiomeric, diastereomeric and geometric forms. Some of the compounds of the present invention have cyclohydrocarbyl which may be substituted on more than one carbon atom, in which case all geometric forms thereof, including cis and trans, and mixtures thereof, are within the scope of the present invention. The cyclohydrocarbyl includes aliphatic cyclohydrocarbyl and aryl, wherein the alicyclic cyclohydrocarbyl may be non-aromatic, monocyclic, fused, bridged or spiro, saturated or unsaturated cyclic hydrocarbyl, and the aryl may be phenyl, naphthyl, phenanthryl, biphenyl and the like.

The solvate described herein refers to a physical association of the compound of the present invention with one or more solvent molecules. The physical association includes various degrees of ionic and covalent bonding, including hydrogen bonding. In some cases, the solvate can be isolated, for example, when one or more solvent molecules are incorporated into the crystal lattice of a crystalline solid. Solvates include both solution phases and isolatable solvates. Representative solvates include ethanolates, methanolates, and the like.

The prodrug described herein refers to forms of the compound of formula I (including acetals, esters, and zwitterions) which are suitable for administration to patients without undue toxicity, irritation, allergic response and the like, and which are effective for the intended use thereof. The prodrug is converted *in vivo*, e.g., by hydrolysis in blood, to give the parent compound of the above formula.

For the term C₀₋₁₀ alkyl described herein, C₀ alkyl refers to H, and thus C₀₋₁₀ alkyl comprises H, C₁ alkyl, C₂ alkyl, C₃ alkyl, C₄ alkyl, C₅ alkyl, C₆ alkyl, C₇ alkyl, C₈ alkyl, C₉ alkyl and C₁₀ alkyl.

The term C₁₋₁₀ linear/branched alkyl described herein comprises methyl, ethyl, C₃ linear/branched alkyl, C₄ linear/branched alkyl, C₅ linear/branched alkyl, C₆ linear/branched alkyl, C₇ linear/branched alkyl, C₈ linear/branched alkyl, C₉ linear/branched alkyl and C₁₀ linear/branched alkyl.

The term C₃₋₁₀ branched alkyl described herein comprises isopropyl, isobutyl, *tert*-butyl and isoamyl.

The term C₃₋₁₀ cyclohydrocarbyl described herein comprises C₃ cyclohydrocarbyl, C₄ cyclohydrocarbyl, C₅ cyclohydrocarbyl, C₆ cyclohydrocarbyl, C₇ cyclohydrocarbyl, C₈ cyclohydrocarbyl, C₉ cyclohydrocarbyl and C₁₀ cyclohydrocarbyl.

The term halogen described herein comprises fluorine, chlorine, bromine and iodine.

The term heterocyclohydrocarbyl described herein comprises aliphatic heterocyclyl and aromatic heterocyclyl, and preferably aromatic heterocyclyl and aliphatic heterocyclyl having 1 to 3 monocyclic and/or fused rings and 3 to about 18 ring atoms.

The term heterocycloalkyl described herein refers to a non-aromatic saturated monocyclic or polycyclic ring system containing 3 to 10 ring atoms, preferably 5 to 10 ring atoms, wherein one or more ring atoms are not carbon atoms, but are, for example, nitrogen, oxygen or sulfur atoms. Preferred heterocycloalkyl contains 5 to 6 ring atoms. The prefix aza, oxa or thia before heterocycloalkyl means that there is at least one nitrogen, oxygen or sulfur atom as a ring atom.

The term heterocycloaryl described herein refers to an aromatic monocyclic or polycyclic ring system containing 5 to 14 ring atoms, preferably 5 to 10 ring atoms, wherein one or more ring atoms are not carbon atoms, but are, for example, nitrogen, oxygen or sulfur atoms. Preferred heterocycloaryl contains 5 to 6 ring atoms. Representative heterocycloaryl comprises pyrazinyl, furyl, thienyl, pyridyl, pyrimidinyl, isoxazolyl, isothiazolyl, oxazolyl, thiazolyl, pyrazolyl, pyrrolyl, triazolyl, pyridazinyl, quinoxalinyl, 2,3-naphthyridinyl, imidazo[1,2-a]pyridine, imidazo[2,1-b]thiazolyl, benzofurazanyl, indolyl, azaindolyl, benzimidazolyl, benzothienyl, quinolyl, imidazolyl, thienopyridyl, quinazolinyl, thienopyrimidinyl, pyrrolopyridyl, imidazopyridinyl, isoquinolyl, 1,2,4-triazinyl, benzothiazolyl, and the like.

### BRIEF DESCRIPTION OF THE DRAWINGS

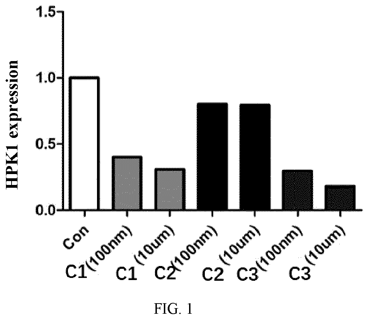

FIG. 1 is a graph showing expression of HPK1 when treated with Compounds C1, C2 and C3.

### DETAILED DESCRIPTION

The technical solutions in the examples of the present invention will be described clearly and completely below, and it is apparent that the examples described herein are only some examples of the present invention, but not all of them. Based on the examples of the present invention, all other examples obtained by those of ordinary skill in the art without creative work shall fall within the protection scope of the present invention.

### Example 1: Preparation of Compound C1

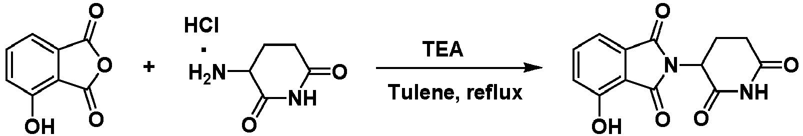

To a 250-mL round-bottom flask were added 3-hydroxyphthalic anhydride (2 g, 12.2 mmol, 1.0 equiv), 3-amino-2,6-piperidinedione hydrochloride (2 g, 12.2 mmol, 1.0 equiv) and an appropriate amount of toluene, and after thorough mixing, the resulting mixture was added with triethylamine (1860 uL, 13.4 mmol, 1.1 equiv), heated to reflux for 12 h, and then the heating was stopped. After the reaction system was returned to room temperature, toluene was removed on a rotary evaporator. The resulting crude product was purified by silica gel column chromatography (methanol:dichloromethane = 1:40, v/v) to give a final product 2-(2,6-dioxopiperidin-3-yl)-4-hydroxyiso-1,3-dione (1.9032 g, yield: 57%) in the form of a pale yellow solid.

NMR: ¹H NMR (400 MHz, DMSO) δ 11.15 (s, 1H), 11.07 (s, 1H), 7.62 (t, *J* = 7.7 Hz, 1H), 7.29 (d, *J* = 7.1 Hz, 1H), 7.22 (d, *J* = 8.4 Hz, 1H), 5.05 (dd, *J* = 12.8, 5.1 Hz, 1H), 3.04 - 2.73 (m, 1H), 2.68 - 2.34 (m, 1H), 2.17 - 1.73 (m, 1H). ¹³C NMR (100 MHz, DMSO) δ 173.28, 170.49, 167.50, 166.30, 155.94, 136.86, 133.63, 124.03, 114.84, 114.77, 49.11, 31.60, 22.80. 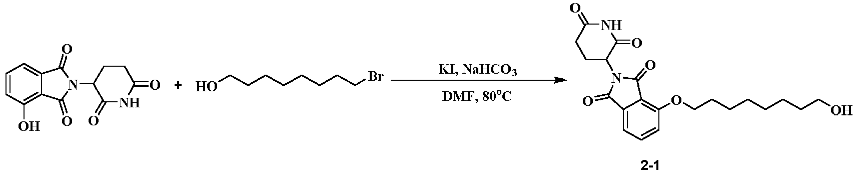 2-(2,6-dioxopiperidin-3-yl)-4-hydroxyiso-1,3-dione (100 mg, 0.4 mmol, 1.0 equiv), potassium iodide (6.1 mg, 0.03 mmol, 0.1 equiv) and sodium hydrogencarbonate (61.3 mg, 0.4 mmol, 2.0 equiv) were dissolved in DMF, 8-bromo-1-octanol (100.4 mg, 0.48 mmol, 1.2 equiv) was added to the reaction system, and then the reaction system was heated to 80°C and reacted overnight. The heating was stopped, the reaction system was cooled to room temperature, diluted with an appropriate amount of ethyl acetate, and extracted with saturated saline for 5 times. The resulting organic phase was dried over anhydrous sodium sulfate, filtered, and concentrated by rotary evaporation. The resulting crude product was purified by silica gel column chromatography (petroleum ether:ethyl acetate = 1:2, v/v) to give a final product (103.6 mg, yield: 64%); LC-MS: m/z = 403. 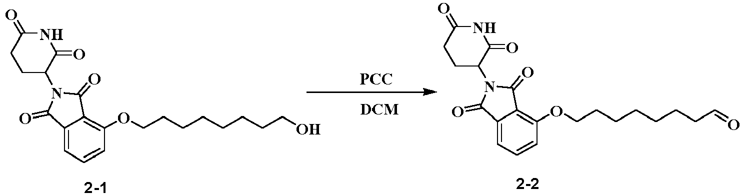 2-1 (92.6 mg, 0.23 mmol, 1.0 equiv) and a Sarrett reagent PCC (99.2 mg, 0.46 mmol, 2.0 equiv) were dissolved in an appropriate amount of dichloromethane, and the resulting mixture was stirred at room temperature for 6 h. The resulting reaction system was added with an appropriate amount of silica gel, concentrated by rotary evaporation, and purified by silica gel column chromatography (petroleum ether:ethyl acetate = 1:2, v/v) to give a final product 2-2 (91 mg, yield: 99%); LC-MS: m/z = 401. 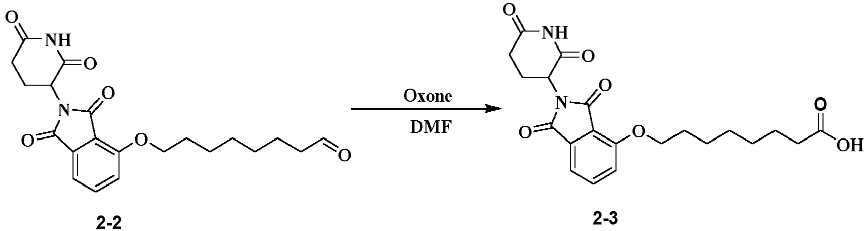 2-2 (54.3 mg, 0.14 mmol, 1.0 equiv) and potassium monopersulfate compound (Oxone) (83.4 mg, 0.14 mol, 1.0 equiv) were dissolved in an appropriate amount of DMF, and the resulting mixture was stirred at room temperature overnight. The resulting reaction system was diluted with ethyl acetate and extracted with water and saturated brine for 3 times. The resulting organic phase was dried over anhydrous sodium sulfate, filtered and concentrated by rotary evaporation to give a final product 2-3 (47.9 mg, yield: 82%); LC-MS: m/z = 417. 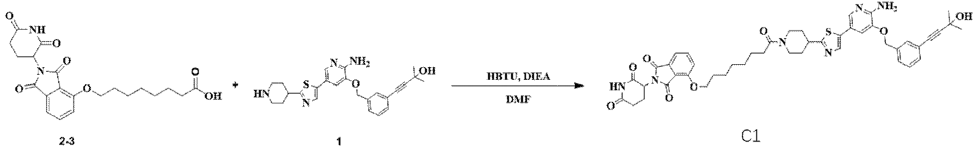 2-3 (10 mg, 0.024 mmol, 1.1equiv) was dissolved in an appropriate amount of DMF, and the resulting mixture was stirred at 0°C, added with benzotriazole-*N*,*N*,*N*',*N*'-tetramethyluronium hexafluorophosphate (HBTU) (18.3 mg, 0.048 mmol, 2.0 equiv), after 5 min, sequentially added with DIEA (9.3 mg, 0.072 mmol, 3.0 equiv) and Compound 1 (10 mg, 0.022 mmol, 1.0 equiv), (for the preparation method of Compound 1, refer to Patent Application NOs. CN 201810945948.2 and CN 201810946081.2), then heated to room temperature, and stirred overnight. The resulting reaction system was diluted with ethyl acetate and extracted with water and saturated brine for 3 times. The resulting organic phase was dried over anhydrous sodium sulfate, filtered under vacuum with a sand-core funnel, and concentrated by rotary evaporation. The resulting crude product was purified by silica gel column chromatography (methanol:dichloromethane = 1:20, v/v) to give a final product 2-4 (17 mg, yield: 91.3%); LC-MS: m/z = 847.

NMR: ¹H NMR (400 MHz, DMSO) δ 11.13 (s, 1H), 7.89 (s, 1H), 7.85 - 7.72 (m, 2H), 7.63 - 7.46 (m, 3H), 7.41 (dd, J = 16.7, 7.8 Hz, 2H), 7.34 (m, J = 7.5 Hz, 2H), 6.10 (s, 2H), 5.48 (s, 1H), 5.22 (s, 2H), 5.09 (dd, J = 12.8, 5.2 Hz, 1H), 4.43 (d, J = 12.7 Hz, 1H), 4.19 (s, 2H), 3.93 (d, J = 13.0 Hz, 1H), 3.20 (dt, J = 24.9, 11.5 Hz, 2H), 3.01 - 2.80 (m, 1H), 2.72 (t, J = 12.1 Hz, 1H), 2.66 - 2.43 (m, 2H), 2.33 (t, J = 7.1 Hz, 2H), 2.04 (dd, J = 21.1, 13.0 Hz, 3H), 1.85 - 1.70 (m, 2H), 1.69 - 1.58 (m, 1H), 1.58 - 1.49 (m, 4H), 1.47 (s, 6H), 1.32 (m, 4H), 1.24 - 1.11 (m, 1H). ¹³C NMR (101 MHz, DMSO) δ 173.24, 171.83, 170.95, 170.42, 167.31, 165.77, 156.47, 151.49, 140.68, 137.66, 137.46, 137.09, 136.93, 135.88, 133.70, 131.14, 130.71, 129.25, 128.00, 123.19, 120.21, 116.67, 116.33, 115.56, 115.06, 96.78, 80.67, 69.25, 69.19, 64.07, 49.20, 45.10, 41.14, 33.07, 32.77, 32.40, 32.06, 31.44, 29.21, 28.99, 28.86, 25.70, 25.30, 22.48. 13C NMR (101 MHz, DMSO) δ 173.24, 171.83, 170.95, 170.42, 167.31, 165.77, 156.47, 151.49, 140.68, 137.66, 137.46, 137.09, 136.93, 135.88, 133.70, 131.14, 130.71, 129.25, 128.00, 123.19, 120.21, 116.67, 116.33, 115.56, 115.06, 96.78, 80.67, 69.25, 69.19, 64.07, 49.20, 45.10, 41.14, 33.07, 32.77, 32.40, 32.06, 31.44, 29.21, 28.99, 28.86, 25.70, 25.30, 22.48, 21.78, 14.55.

### Example 2: Preparation of Compound C2

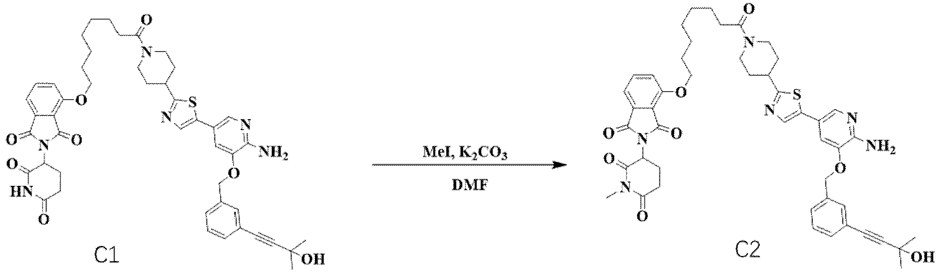

C1 (12 mg, 0.014 mmol,1.0equiv) was dissolved in an appropriate amount of DMF, potassium carbonate (3.9 mg, 0.021 mmol,1.5 equiv) and iodomethane (2.4 mg, 0.017 mmol,1.2 equiv) were sequentially added, and the resulting mixture was stirred at room temperature overnight. When the reaction was completed, the resulting reaction system was diluted with an appropriate amount of ethyl acetate, and extracted with saturated saline for 3 times. The resulting organic phase was dried over anhydrous sodium sulfate, filtered under vacuum with a sand-core funne, concentrated by rotary evaporation, and purified by silica gel column chromatography purification (methanol:dichloromethane = 1:20, v/v) to give a final product 2-5 (8.2 mg, yield: 68%); LC-MS: m/z = 861.

NMR: ¹H NMR (400 MHz, DMSO) δ 7.88 (s, 1H), 7.84 - 7.77 (m, 1H), 7.76 (d, J = 1.5 Hz, 1H), 7.56 - 7.48 (m, 3H), 7.45 - 7.36 (m, 2H), 7.36 - 7.30 (m, 2H), 6.09 (s, 2H), 5.46 (s, 1H), 5.21 (s, 2H), 5.15 (dd, J = 13.0, 5.4 Hz, 1H), 4.42 (d, J = 12.9 Hz, 1H), 4.20 (t, J = 6.3 Hz, 2H), 3.93 (d, J = 13.5 Hz, 1H), 3.29 - 3.09 (m, 3H), 2.98 (s, 3H), 2.96 - 2.87 (m, 1H), 2.81 - 2.65 (m, 2H), 2.62 - 2.51 (m, 2H), 2.33 (t, J = 7.3 Hz, 2H), 2.12 - 1.97 (m, 3H), 1.83 - 1.69 (m, 2H), 1.69 - 1.56 (m, 1H), 1.57 - 1.47 (m, 3H), 1.45 (s, 6H), 1.44 - 1.40 (m, 1H), 1.30 - 1.15 (m, 3H). ¹³C NMR (101 MHz, DMSO) δ 172.24, 171.87, 170.97, 170.17, 167.30, 165.78, 156.53, 151.50, 140.70, 137.68, 137.52, 137.08, 136.96, 135.89, 133.71, 131.15, 130.73, 129.27, 128.02, 123.20, 120.28, 116.68, 116.34, 115.60, 115.13, 96.80, 80.68, 69.29, 69.21, 64.08, 49.78, 45.11, 41.14, 33.08, 33.07, 32.77, 32.40, 32.07, 31.58, 29.20, 28.98, 28.86, 27.07, 25.71, 25.30, 21.68.

### Example 3: Preparation of Compound C3

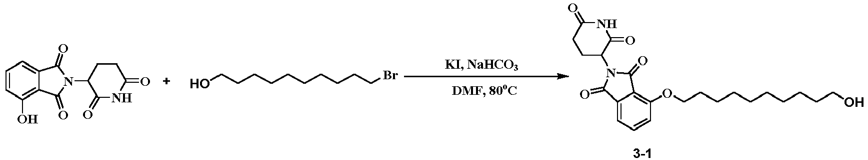

2-(2,6-dioxopiperidin-3-yl)-4-hydroxyiso-1,3-dione (300 mg, 1.1 mmol, 1.0 equiv), potassium iodide (18.2 mg, 0.1 mmol, 0.1 equiv) and sodium bicarbonate (183.8 mg, 2.2 mmol, 2.0 equiv) were dissolved in DMF, and the resulting mixture was added with 10-bromo-1-decanol (314 mg, 1.32 mmol, 1.2 equiv), heated to 80°C and reacted overnight. The heating was stopped, the resulting reaction system was cooled to room temperature, diluted with an appropriate amount of ethyl acetate, and extracted with saturated saline for 5 times. The resulting organic phase was dried over anhydrous sodium sulfate, filtered, and concentrated by rotary evaporation. The resulting crude product was purified by silica gel column chromatography (petroleum ether:ethyl acetate = 1:2, v/v) to give a final product (253.3 mg, yield: 53%); LC-MS: m/z = 431. 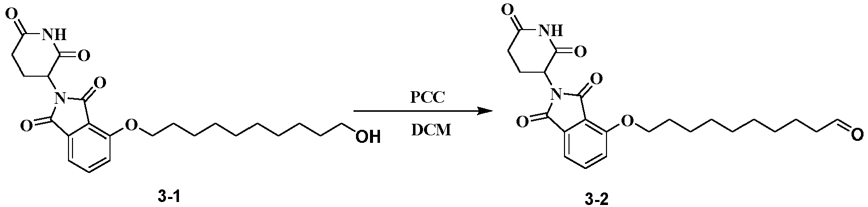 3-1 (190 mg, 0.32 mmol, 1.0 equiv) and PCC (190 mg, 0.64 mmol, 2.0 equiv) were dissolved in an appropriate amount of dichloromethane, and the resulting mixture was stirred at room temperature for 6 h. The resulting reaction system was added with an appropriate amount of silica gel, concentrated by rotary evaporation, and purified by silica gel column chromatography (petroleum ether:ethyl acetate = 1:2, v/v) to give a final product 3-2 (135.5 mg, yield: 99%); LC-MS: m/z =429. 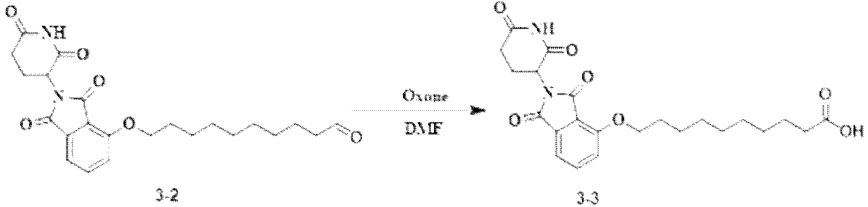 3-2 (135.5 mg, 0.32 mmol, 1.0 equiv) and Oxone (197 mg, 0.32 mmol, 1.0 equiv) were dissolved in an appropriate amount of DMF, and the resulting mixture was stirred at room temperature overnight. The resulting reaction system was diluted with ethyl acetate and extracted with water and saturated brine for 3 times. The resulting organic phase was dried over anhydrous sodium sulfate, filtered, and concentrated by rotary evaporation to give a final product 3-3 (112.6 mg, yield: 79%); LC-MS: m/z = 445. 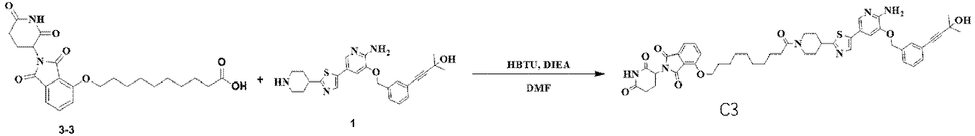 3-3 (12.2 mg, 0.028 mmol, 1.1 equiv) was dissolved in an appropriate amount of DMF, and the resulting mixture was stirred at 0°C, added with HBTU (19 mg, 0.05 mmol, 2.0 equiv), after 5 min, sequentially added with DIEA (9.7 mg, 0.075 mmol, 3.0 equiv) and Compound 1 (11 mg, 0.025 mmol, 1.0 equiv), then heated to room temperature, and stirred overnight. The resulting reaction system was diluted with ethyl acetate, and extracted with water and saturated brine for 3 times. The resulting organic phase was dried over anhydrous sodium sulfate, filtered under vacuum with a sand-core funnel, and concentrated by rotary evaporation. The resulting crude product was purified by silica gel column chromatography (methanol:dichloromethane = 1:20, v/v) to give a final product 3-4 (15.7 mg, yield: 72%); LC-MS: m/z = 875.

NMR: ¹H NMR(400 MHz, DMSO) δ 11.13 (s, 1H), 7.89 (s, 1H), 7.84 - 7.77 (m, 1H), 7.76 (s, 1H), 7.53 (s, 2H), 7.47 (dd, J = 25.3, 7.9 Hz, 2H), 7.42 - 7.36 (m, 1H), 7.34 (d, J = 7.4 Hz, 2H), 6.11 (s, 2H), 5.49 (s, 1H), 5.22 (s, 2H), 5.09 (dd, J = 12.9, 5.2 Hz, 1H), 4.43 (d, J = 12.2 Hz, 1H), 4.19 (t, J = 6.2 Hz, 2H), 3.93 (d, J = 13.5 Hz, 1H), 3.30 - 3.11 (m, 4H), 2.99 - 2.78 (m, 2H), 2.78 - 2.65 (m, 1H), 2.64 - 2.53 (m, 2H), 2.35 - 2.25 (m, 2H), 2.14 - 1.92 (m, 4H), 1.83 - 1.48 (m, 6H), 1.47 (s, 6H), 1.43 - 1.32 (m, 3H), 1.24 - 1.15 (m, 4H). ¹³C NMR (101 MHz, DMSO) δ 173.28, 171.84, 170.96, 170.44, 167.32, 165.77, 156.47, 151.49, 140.65, 137.67, 137.49, 137.08, 136.93, 135.89, 133.69, 131.13, 130.71, 129.26, 128.10, 128.02, 123.16, 120.21, 116.65, 116.29, 115.58, 115.06, 115.03, 96.77, 80.66, 69.22, 69.14, 64.06, 49.18, 45.11, 41.13, 33.08, 32.80, 32.42, 32.05, 31.42, 29.37, 29.27, 29.24, 29.09, 28.87, 25.73, 25.37, 22.46.

### Example 4: Preparation of Compound C4

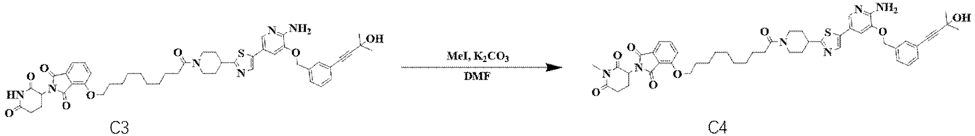

C3 (15.7 mg, 0.018 mmol, 1.0 equiv) was dissolved in an appropriate amount of DMF, potassium carbonate (3.7 mg, 0.027 mmol, 1.5 equiv) and iodomethane (3.1 mg, 0.022 mmol, 1.2 equiv) were sequentially added, and the resulting mixture was stirred at room temperature overnight. When the reaction was completed, the resulting reaction system was diluted with an appropriate amount of ethyl acetate, and extracted with saturated saline for 3 times. The resulting organic phase was dried over anhydrous sodium sulfate, filtered under vacuum with a sand-core funnel, concentrated by rotary evaporation, and purified by silica gel column chromatography (methanol:dichloromethane = 1:20, v/v) to give a final product (10.4 mg, yield: 65%); LC-MS: m/z = 889.

### Example 5: Preparation of Compound C5

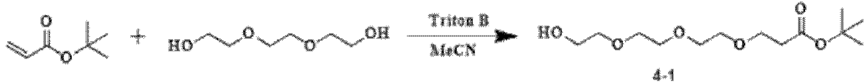

To a solution of triethylene glycol (5.9 g, 39 mmol, 5.0 equiv) in acetonitrile were added *tert-butyl* acrylate (1 g, 7.8 mmol, 1.0 equiv) and benzyltrimethylammonium hydroxide (Triton B) (121 mg, 0.29 mmol, 1.0 equiv, 40% aqueous solution), and the resulting mixture was stirred at room temperature for 48 h. Color was developed with KMnO₄ to determine whether the reaction was completed or not. The solvent was removed on a rotary evaporator. The resulting residue was purified by silica gel column chromatography (petroleum ether:ethyl acetate = 2:1, v/v) to give a target product 4-1 (1.6632 g, yield: 77%) in the form of an oily liquid.

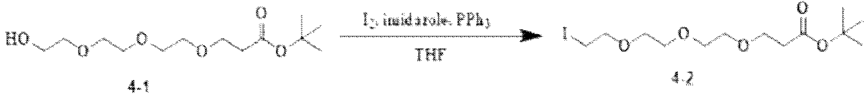

Triphenylphosphine (1007.2 mg, 3.84 mmol, 1.2 equiv), imidazole (261.4 mg, 3.84 mmol, 1.2 equiv) and iodine (1218.3 mg, 4.8 mmol, 1.5 equiv) were dissolved in tetrahydrofuran (extra dry) under argon atmosphere, after thorough mixing, the resulting mixture was added dropwise with 4-1 (900 mg, 3.2 mmol, 1.0 equiv), and stirred at room temperature for 3 h, followed by TLC detection and color development with KMnO₄. After the reaction was completed, the white precipitate was removed by filtration, and the residue was concentrated on a rotary evaporator and purified by silica gel column chromatography (petroleum ether:ethyl acetate = 1:2, v/v) to give a final product 4-2 (1208.8 mg, yield: 97%) in the form of a brown oily liquid.

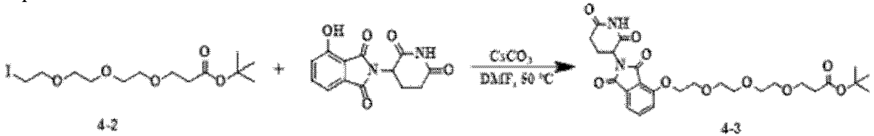

2-(2,6-dioxopiperidin-3-yl)-4-hydroxyiso-1,3-dione (181 mg, 0.66 mmol, 1.0 equiv) and cesium carbonate (644 mg, 1.98 mmol, 3.0 equiv) were dissolved in DMF, and the resulting mixture was added dropwise with 4-2 (307.5 mg, 0.79 mmol, 1.2 equiv), heated to 50°C and stirred overnight. When the reaction was completed, the resulting reaction system was diluted with an appropriate amount of ethyl acetate, and sequentially extracted with a mixed solution of water and a saturated sodium bicarbonate solution (water: saturated sodium bicarbonate solution = 1:5, v/v) and saturated saline. The resulting organic phase was dried over anhydrous sodium sulfate, filtered under vacuum with a sand-core funnel, concentrated by rotary evaporation, and purified by silica gel column chromatography (acetone:petroleum ether = 1:2, v/v) to give a final product 4-3 (194 mg, yield: 55%); LC-MS: m/z = 535.

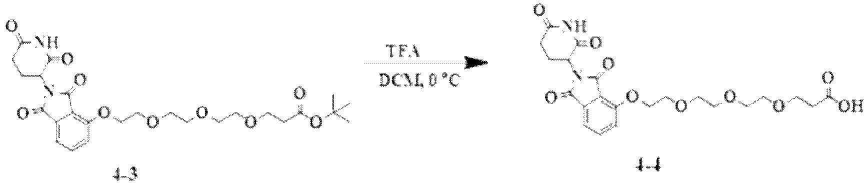

4-3 (40 mg, 0.075 mmol, 1.0 equiv) was dissolved in an appropriate amount of dichloromethane, and the resulting mixture was added with 2-fold volume of trifluoroacetic acid at 0°C and stirred for 2 h. After the reaction was completed as monitored by LC-MS, dichloromethane and trifluoroacetic acid were removed on a rotary evaporator, and the residue was dried in a vacuum pump for 2 h to remove the residual solvent and acid to give a crude product 4-4 (35.4 mg) which was used in the next step directly without further purification; LC-MS: m/z = 479.

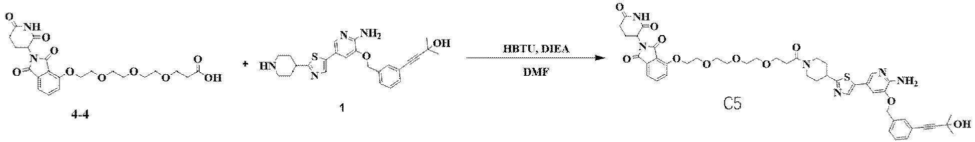

4-4 (23.7 mg, 0.05 mmol, 1.1 equiv) was dissolved in an appropriate amount of DMF, and the resulting mixture was stirred at 0°C, added with HBTU (34 mg, 0.09 mmol, 2.0 equiv), after 5 min, sequentially added with DIEA (17.2 mg, 0.13 mmol, 3.0 equiv) and Compound 1 (20 mg, 0.045 mmol, 1.0 equiv), then heated to room temperature, and stirred overnight. The reaction system was diluted with ethyl acetate, and extracted with water and saturated brine for 3 times. The resulting organic phase was dried over anhydrous sodium sulfate, filtered under vacuum with a sand-core funnel, and concentrated by rotary evaporation. The resulting crude product was purified by silica gel column chromatography (methanol:dichloromethane = 1:20, v/v) to give a final product (20.1 mg, yield: 51%); LC-MS: m/z = 909.

NMR: ¹H NMR (400 MHz, DMSO) δ 7.89 (s, 1H), 7.77 (s, 1H), 7.71 - 7.61 (m, 1H), 7.53 (s, 2H), 7.39 (m, 3H), 7.29 (dd, J = 25.0, 7.8 Hz, 2H), 6.10 (s, 2H), 5.48 (s, 1H), 5.22 (s, 2H), 5.16 (dd, J = 13.0, 5.3 Hz, 1H), 4.42 (d, J = 13.2 Hz, 1H), 3.97 (d, J = 13.1 Hz, 1H), 3.83 (m, 2H), 3.64 (t, J = 6.6 Hz, 2H), 3.50 (d, J = 6.0 Hz, 6H), 3.43 (t, J = 6.6 Hz, 2H), 3.27 - 3.08 (m, 2H), 3.07 - 2.85 (m, 2H), 2.72 (dd, J = 24.3, 12.1 Hz, 2H), 2.60 (m, 2H), 2.13 - 1.92 (m, 4H), 1.76 - 1.53 (m, 2H), 1.47 (s, 6H). ¹³C NMR (101 MHz, DMSO) δ 171.98, 171.85, 170.00, 169.10, 167.44, 165.41, 156.04, 151.49, 148.07, 140.68, 137.89, 137.67, 137.10, 136.93, 136.85, 135.88, 133.59, 132.78, 131.13, 130.71, 130.11, 129.25, 128.01, 124.09, 123.18, 121.86, 118.15, 117.33, 116.33, 115.08, 114.79, 114.72, 96.78, 95.65, 88.52, 80.67, 70.24, 70.14, 70.07, 69.18, 67.36, 67.18, 64.07, 49.71, 33.28, 32.06, 31.59, 29.55, 29.49, 29.44, 22.56, 21.73.

### Example 6: Preparation of Compound C6

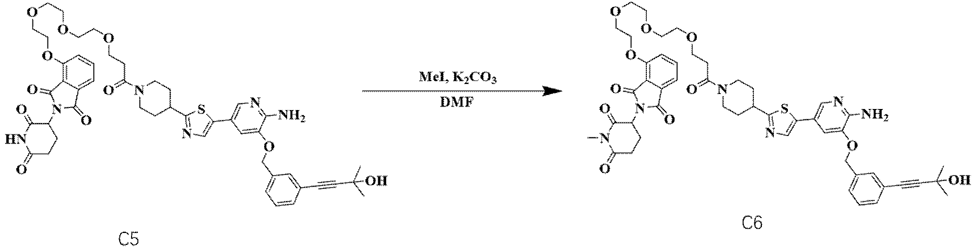

C5 (10 mg, 0.011 mmol, 1.0 equiv) was dissolved in an appropriate amount of DMF, potassium carbonate (2.3 mg, 0.017 mmol, 1.5 equiv) and iodomethane (2 mg, 0.013 mmol, 1.2 equiv) were sequentially added, and the resulting mixture was stirred at room temperature overnight. When the reaction was completed, the resulting reaction system was diluted with an appropriate amount of ethyl acetate, and extracted with saturated saline for 3 times. The resulting organic phase was dried over anhydrous sodium sulfate, filtered under vacuum with a sand-core funnel, concentrated by rotary evaporation, and purified by silica gel column chromatography (methanol:dichloromethane = 1:20, v/v) to give a final product (10.2 mg, yield: 81%); LC-MS: m/z = 923.

NMR: 1H NMR (400 MHz, CDCl3) δ 7.83 (s, 1H), 7.72 - 7.65 (m, 1H), 7.64 (s, 1H), 7.50 (s, 1H), 7.45 (d, J = 7.3 Hz, 1H), 7.42 - 7.30 (m, 3H), 7.22 (d, J = 8.5 Hz, 1H), 7.06 (d, J = 1.4 Hz, 1H), 5.34 (t, J = 4.7 Hz, 1H), 5.08 (s, 2H), 4.97 (dd, J = 12.1, 5.7 Hz, 1H), 4.92 (s, 2H), 4.65 (d, J = 13.3 Hz, 1H), 4.08 - 3.97 (m, 5H), 3.79 (t, J = 6.8 Hz, 2H), 3.66 - 3.55 (m, 9H), 3.25 - 3.13 (m, 2H), 3.01 - 2.87 (m, 1H), 2.83 - 2.71 (m, 3H), 2.71 - 2.64 (m, 2H), 2.24 - 1.99 (m, 5H), 1.82 - 1.70 (m, 2H), 1.62 (s, 6H). ¹³C NMR (101 MHz, CDCl₃) δ 172.58, 171.10, 169.57, 168.71, 167.23, 166.12, 157.09, 150.22, 141.28, 137.24, 136.88, 136.73, 136.10, 135.78, 133.97, 131.83, 131.08, 130.05, 128.93, 127.62, 123.51, 118.09, 117.92, 117.20, 115.98, 115.24, 94.83, 81.61, 70.62, 70.12, 67.64, 67.58, 65.63, 56.51, 45.61, 41.50, 40.76, 39.42, 36.06, 33.78, 32.87, 32.11, 31.61, 29.46, 27.35, 25.67, 22.83, 22.12.

### Example 7: Preparation of Compound C7

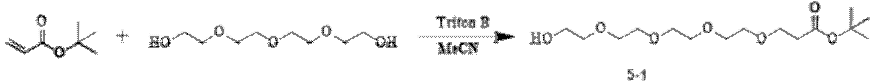

To a solution of tetraethylene glycol (5.9 g, 39 mmol, 5.0 equiv) in acetonitrile were added *tert-butyl* acrylate (1 g, 7.8 mmol, 1.0 equiv) and Triton B (121 mg, 0.29 mmol, 1.0 equiv, 40% aqueous solution), and the resulting was stirred at room temperature for 48 h. Color was developed with KMnO₄ to determine whether the reaction was completed or not. The solvent was removed on a rotary evaporator. The resulting residue was purified by silica gel column chromatography (petroleum ether:ethyl acetate = 2:1, v/v) to give a target product (1.8954 g, yield: 75%) in the form of an oily liquid.

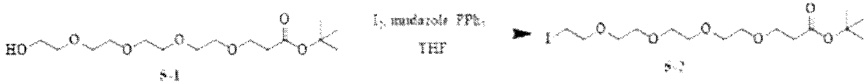

Triphenylphosphine (879.7 mg, 3.4 mmol, 1.2 equiv), imidazole (228.3 mg, 3.4 mmol, 1.2 equiv) and iodine (1064 mg, 4.2 mmol, 1.5 equiv) were dissolved in tetrahydrofuran (extra dry) under argon atmosphere, after thorough mixing, the resulting mixture was added dropwise with 4-1 (900 mg, 2.8 mmol, 1.0 equiv), and stirred at room temperature for 3 h, followed by TLC detection and color development with KMnO₄. After the reaction was completed, the white precipitate was removed by filtration, and the residue was concentrated on a rotary evaporator, and purified by silica gel column chromatography (petroleum ether:ethyl acetate = 1:2, v/v) to give a final product (1150.6 mg, yield: 95%) in the form of a brown oily liquid.

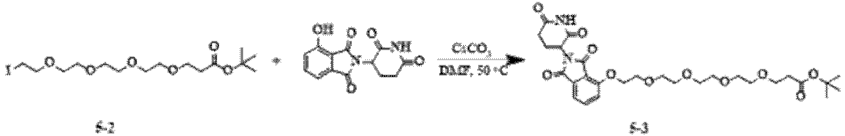

2-(2,6-dioxopiperidin-3-yl)-4-hydroxyiso-1,3-dione (470.7 mg, 1.7 mmol, 1.0 equiv) and cesium carbonate (1678 mg, 5.2 mmol, 3.0 equiv) were dissolved in DMF, and the resulting mixture was added dropwise with 5-2 (307.5 mg, 0.79 mmol, 1.2 equiv), heated to 50°C and stirred overnight. When the reaction was completed, the resulting reaction system was diluted with an appropriate amount of ethyl acetate, and sequentially extracted with a mixed solution of water and a saturated sodium bicarbonate solution (water: saturated sodium bicarbonate solution = 1:5, v/v) and saturated saline. The resulting organic phase was dried over anhydrous sodium sulfate, filtered under vacuum with a sand-core funnel, concentrated by rotary evaporation, and purified by silica gel column chromatography (acetone:petroleum ether = 1:2, v/v) to give a final product (463.1 mg, yield: 47%); LC-MS: m/z = 579.

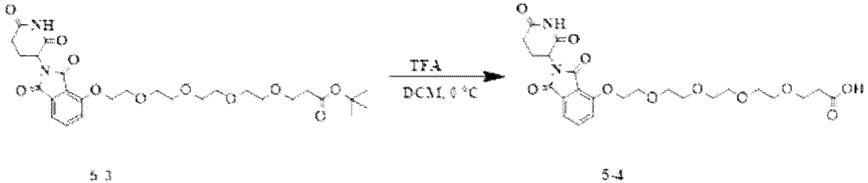

5-3 (40 mg, 0.084 mmol, 1.0 equiv) was dissolved in an appropriate amount of dichloromethane, and the resulting mixture was added with 2-fold volume of trifluoroacetic acid at 0°C and stirred for 2 h. After the reaction was completed as monitored by LC-MS, dichloromethane and trifluoroacetic acid were removed on a rotary evaporator, and the residue was dried in a vacuum pump for 2 h to remove the residual solvent and acid to give a crude product 5-4 (43.6 mg) which was used in the next step directly without further purification; LC-MS: m/z = 523.

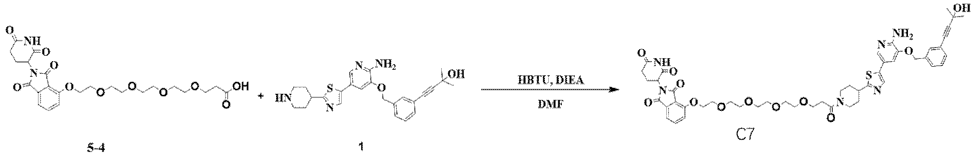

5-4 (26 mg, 0.05 mmol, 1.1 equiv) was dissolved in an appropriate amount of DMF, and the resulting mixture was stirred at 0°C, added with HBTU (34 mg, 0.09 mmol, 2.0 equiv), after 5 min, sequentially added with DIEA (17.2 mg, 0.13 mmol, 3.0 equiv) and compound 1 (20 mg, 0.045 mmol, 1.0 equiv), then heated to room temperature, and stirred overnight. The resulting reaction system was diluted with ethyl acetate, and extracted with water and saturated brine for 3 times. The resulting organic phase was dried over anhydrous sodium sulfate, filtered under vacuum with a sand-core funnel, and concentrated by rotary evaporation, and the crude product was purified by silica gel column chromatography (methanol:dichloromethane = 1:20, v/v) to give a final product 5-5 (22.6 mg, yield: 44%); LC-MS: m/z = 953.

NMR: ¹H NMR (400 MHz, DMSO) δ 7.91 (d, J = 12.6 Hz, 1H), 7.77 (d, J = 1.7 Hz, 1H), 7.70 - 7.60 (m, 1H), 7.54 (m, 2H), 7.47 - 7.32 (m, 3H), 7.27 (dd, J = 22.9, 7.8 Hz, 2H), 6.10 (s, 2H), 5.22 (s, 2H), 5.16 (dd, J = 13.0, 5.4 Hz, 1H), 4.42 (d, J = 12.8 Hz, 1H), 3.97 (d, J = 13.4 Hz, 1H), 3.84 (m, 2H), 3.63 (t, J = 6.7 Hz, 2H), 3.46 - 3.12 (m, 12H), 3.06 - 2.89 (m, 1H), 2.86 - 2.66 (m, 2H), 2.66 - 2.52 (m, 2H), 2.02 (dd, J = 19.6, 16.5 Hz, 3H), 1.75 - 1.51 (m, 2H), 1.47 (s, 6H). ¹³C NMR (101 MHz, DMSO) δ 172.03, 171.93, 170.02, 169.20, 167.50, 166.38, 156.38, 151.47, 140.70, 137.65, 137.07, 136.94, 136.85, 135.87, 133.58, 131.17, 130.70, 129.28, 128.02, 124.27, 123.18, 116.38, 115.09, 114.73, 114.56, 99.99, 96.76, 80.69, 70.24, 70.22, 70.14, 70.12, 70.06, 69.20, 67.34, 67.19, 64.10, 49.70, 45.21, 41.15, 39.11, 33.28, 32.97, 32.27, 32.04, 31.60, 21.73.

### Example 8: Preparation of Compound C8

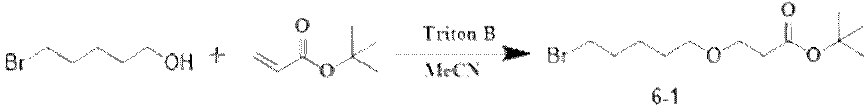

To a solution of 5-bromo-1-pentanol (260 mg, 0.78 mmol, 1.0 equiv) in acetonitrile were added *tert-butyl* acrylate (200 mg, 1.56 mmol, 2.0 equiv) and Triton B (10 mg, 0.09 mmol, 1.0 equiv, 40% aqueous solution), and the resulting mixture was stirred at room temperature for 48 h. Color was developed with KMnO₄ to determine whether the reaction was completed or not. The solvent was removed on a rotary evaporator. The resulting residue was purified by silica gel column chromatography (petroleum ether:ethyl acetate = 50:1, v/v) to give a target product (178 mg, yield: 77%) in the form of an oily liquid.

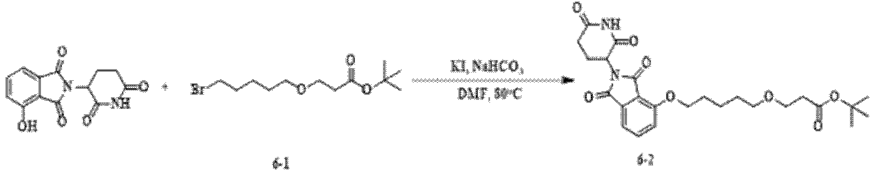

2-(2,6-dioxopiperidin-3-yl) -4-hydroxyiso-1,3-dione (150 mg, 0.55 mmol, 1.0 equiv), potassium iodide (9 mg, 0.054 mmol, 0.1 equiv) and sodium bicarbonate (92 mg, 1.1 mmol, 2.0 equiv) were dissolved in DMF, and the resulting mixture was added with 6-1 (162 mg, 0.55 mmol, 1.2 equiv), heated to 80°C and reacted overnight, and then the heating was stopped. The resulting reaction system was cooled to room temperature, diluted with an appropriate amount of ethyl acetate, and extracted with saturated brine for 5 times. The resulting organic phase was dried over anhydrous sodium sulfate, filtered, and concentrated by rotary evaporation. The resulting crude product was purified by silica gel column chromatography (petroleum ether:acetone = 3:1, v/v) to give a final product 6-2 (164.7 mg, yield: 61%); LC-MS: m/z = 489.

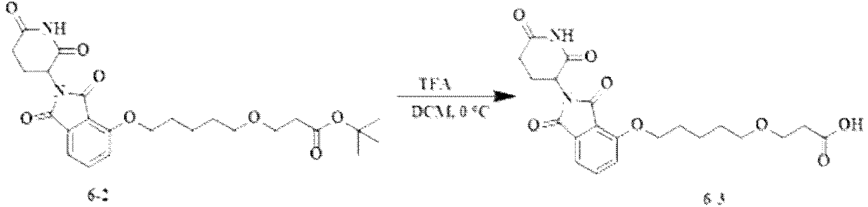

6-2 (164.7 mg, 0.34 mmol, 1.0 equiv) was dissolved in an appropriate amount of dichloromethane, and the resulting mixture was added with 2-fold volume of trifluoroacetic acid at 0°C and stirred for 2 h. After the reaction was completed as monitored by LC-MS, dichloromethane and trifluoroacetic acid were removed on a rotary evaporator. The resulting residue was dried in a vacuum pump for 2 h to remove the residual solvent and acid to give a crude product 6-3 (145 mg) which was used in the next step directly without further purification; LC-MS: m/z = 433. 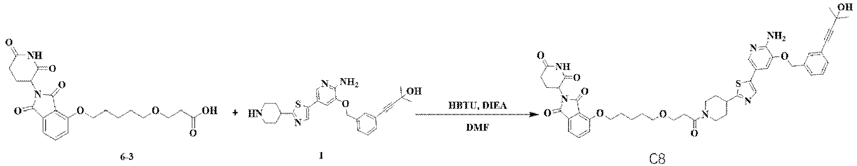 6-3 (21.6 mg, 0.05 mmol, 1.0 equiv) was dissolved in an appropriate amount of DMF, and the resulting mixture was stirred at 0°C, added with HBTU (34 mg, 0.09 mmol, 2.0 equiv), after 5 min, sequentially added with DIEA (17.2 mg, 0.13 mmol, 3.0 equiv) and compound 1 (20 mg, 0.045 mmol, 1.0 equiv), then heated to room temperature, and stirred overnight. The reaction system was diluted with ethyl acetate, and extracted with water and saturated brine for 3 times. The resulting organic phase was dried over anhydrous sodium sulfate, filtered under vacuum with a sand-core funnel, and concentrated by rotary evaporation. The resulting crude product was purified by silica gel column chromatography (methanol:dichloromethane = 1:20, v/v) to give a final product (10.5 mg, yield: 27%); LC-MS: m/z = 863. NMR: ¹H NMR (400 MHz, CDCl₃) δ 7.82 (s, 1H), 7.71 - 7.59 (m, 2H), 7.51 (s, 1H), 7.46 - 7.38 (m, 2H), 7.35 (d, J = 5.7 Hz, 2H), 7.18 (d, J = 8.4 Hz, 1H), 7.07 (s, 1H), 5.42 - 5.32 (m, 1H), 5.09 (s, 2H), 4.93 (d, J = 9.7 Hz, 1H), 4.68 (t, J = 5.7 Hz, 2H), 4.38 - 3.96 (m, 4H), 3.75 (t, J = 6.6 Hz, 2H), 3.48 (d, J = 8.1 Hz, 3H), 3.34 - 3.07 (m, 2H), 3.02 - 2.48 (m, 7H), 2.43 - 2.07 (m, 6H), 2.07 - 1.95 (m, 3H), 1.62 (s, 6H).

### Example 9: Preparation of Compound C9

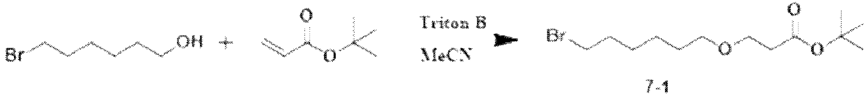

To a solution of 6-bromo-1-hexanol (260 mg, 0.78 mmol, 1.0 equiv) in acetonitrile were added *tert*-butyl acrylate (200 mg, 1.56 mmol, 2.0 equiv) and Triton B (10 mg, 0.09 mmol, 1.0 equiv, 40% aqueous solution), and the resulting mixture was stirred at room temperature for 48 h. Color was developed with KMnO₄ to determine whether the reaction was completed or not. The solvent was removed on a rotary evaporator. The residue was purified by silica gel column chromatography (petroleum ether:ethyl acetate = 50:1, v/v) to give a target product 7-1 (174.4 mg, yield: 72%) in the form of an oily liquid.

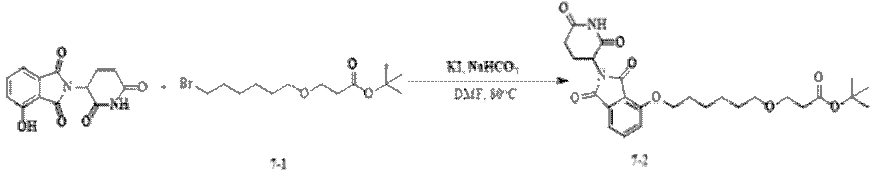

2-(2,6-dioxopiperidin-3-yl) -4-hydroxyiso-1,3-dione (150 mg, 0.55 mmol, 1.0 equiv), potassium iodide (9 mg, 0.054 mmol, 0.1 equiv) and sodium bicarbonate (92 mg, 1.1 mmol, 2.0 equiv) were dissolved in DMF, and the resulting mixture was added with 7-1 (169 mg, 0.55 mmol, 1.2 equiv), heated to 80°C and reacted overnight, and then the heating was stopped. The resulting reaction system was cooled to room temperature, diluted with an appropriate amount of ethyl acetate, and extracted with saturated brine for 5 times. The resulting organic phase was dried over anhydrous sodium sulfate, filtered, and concentrated by rotary evaporation. The resulting crude product was purified by silica gel column chromatography (petroleum ether:acetone = 3:1, v/v) to give a final product 7-2 (163 mg, yield: 59%); LC-MS: m/z = 503.

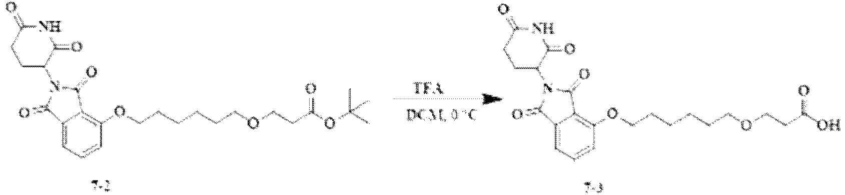

7-2 (163 mg, 0.32 mmol,1.0 equiv) was dissolved in an appropriate amount of dichloromethane, and the resulting mixture was added with 2-fold volume of trifluoroacetic acid at 0°C and stirred for 2 h. After the reaction was completed as monitored by LC-MS, dichloromethane and trifluoroacetic acid were removed on a rotary evaporator. The residue was dried in a vacuum pump for 2 h to remove the residual solvent and acid to give a crude product 7-3 (144.5 mg) which was used in the next step directly without further purification; LC-MS: m/z = 447.

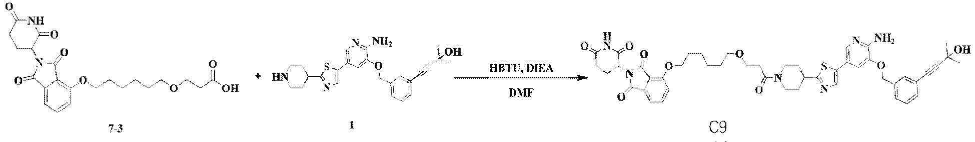

7-3 (22.3 mg, 0.05 mmol, 1.0 equiv) was dissolved in an appropriate amount of DMF, and the resulting mixture was stirred at 0°C, added with HBTU (34 mg, 0.09 mmol, 2.0 equiv), after 5 min, sequentially added with DIEA (17.2 mg, 0.13 mmol, 3.0 equiv) and compound 1 (20 mg, 0.045 mmol, 1.0 equiv), then heated to room temperature, and stirred overnight. The reaction system was diluted with ethyl acetate, and extracted with water and saturated brine for 3 times. The resulting organic phase was dried over anhydrous sodium sulfate, filtered under vacuum with a sand-core funnel, and concentrated by rotary evaporation. The resulting crude product was purified by silica gel column chromatography (methanol:dichloromethane = 1:20, v/v) to give a final product (26mg, yield: 65%); LC-MS: m/z = 877.

NMR: ¹H NMR (400 MHz, CDCl₃) δ 7.76 (d, J = 1.2 Hz, 1H), 7.69 - 7.58 (m, 2H), 7.46 (s, 1H), 7.43 - 7.35 (m, 2H), 7.35 - 7.28 (m, 2H), 7.16 (dd, J = 8.5, 3.7 Hz, 1H), 7.04 (d, J = 1.4 Hz, 1H), 5.52 (s, 1H), 5.03 (s, 2H), 4.98 - 4.84 (m, 1H), 4.65 (d, J = 13.4 Hz, 1H), 4.12 (d, J = 2.3 Hz, 3H), 3.99 (d, J = 13.7 Hz, 1H), 3.84 - 3.62 (m, 2H), 3.54 - 3.37 (m, 4H), 3.18 (dd, J = 27.3, 12.0 Hz, 2H), 2.98 - 2.52 (m, 7H), 2.11 (dd, J = 20.1, 10.2 Hz, 3H), 1.93 - 1.65 (m, 3H), 1.61 (s, 6H), 1.54 - 1.29 (m, 5H). ¹³C NMR (101 MHz, CDCl₃) δ 172.64, 171.94, 169.76, 168.68, 167.16, 165.69, 156.59, 150.37, 141.22, 136.72, 136.44, 135.89, 135.64, 135.41, 133.79, 131.67, 130.90, 128.78, 127.46, 123.41, 118.90, 117.38, 117.08, 115.65, 115.06, 94.87, 81.41, 71.09, 70.02, 69.25, 67.14, 66.23, 65.40, 49.12, 45.63, 41.50, 40.56, 35.32, 33.70, 32.81, 32.12, 31.47, 29.47, 28.78, 25.76, 25.59, 22.66.

### Example 10: Preparation of Compound C10

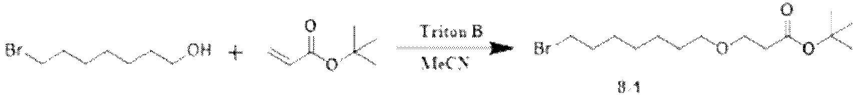

To a solution of 7-bromo-1-heptanol (260 mg, 0.78 mmol, 1.0 equiv) in acetonitrile were added *tert-butyl* acrylate (200 mg, 1.56 mmol, 2.0 equiv) and Triton B (10 mg, 0.09 mmol, 1.0 equiv, 40% aqueous solution), and the resulting mixture was stirred at room temperature for 48 h. Color was developed with KMnO₄ to determine whether the reaction was completed or not. The solvent was removed on a rotary evaporator. The residue was purified by silica gel column chromatography (petroleum ether:ethyl acetate = 50:1, v/v) to give a target product 8-1 (258.6 mg, yield: 80%) in the form of an oily liquid. 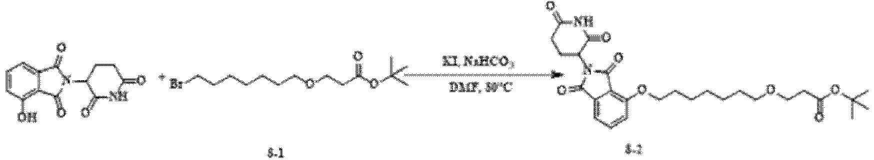 2-(2,6-dioxopiperidin-3-yl) -4-hydroxyiso-1,3-dione (150 mg, 0.55 mmol,1.0 equiv), potassium iodide (9 mg, 0.054 mmol,0.1 equiv) and sodium bicarbonate (92 mg, 1.1 mmol, 2.0 equiv) were dissolved in DMF, and the resulting mixture was added with 8-1 (177 mg, 0.55 mmol, 1.0 equiv), heated to 80°C and reacted overnight, and then the heating was stopped. The reaction system was cooled to room temperature, diluted with an appropriate amount of ethyl acetate, and extracted with saturated brine for 5 times. The resulting organic phase was dried over anhydrous sodium sulfate, filtered, and concentrated by rotary evaporation. The crude product was purified by silica gel column chromatography (petroleum ether: acetone = 3:1, v/v) to give a final product 8-2 (179 mg, yield: 63%); LC-MS: m/z = 517. 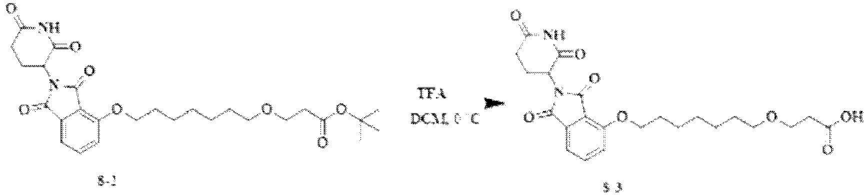 8-2 (179 mg, 0.35 mmol, 1.0 equiv) was dissolved in an appropriate amount of dichloromethane, and the resulting mixture was added with 2-fold volume of trifluoroacetic acid at 0°C and stirred for 2 h. After the reaction was completed as monitored by LC-MS, dichloromethane and trifluoroacetic acid were removed on a rotary evaporator. The residue was dried in a vacuum pump for 2 h to remove the residual solvent and acid to give a crude product 8-3 (158.3 mg) which was used in the next step directly without further purification; LC-MS: m/z = 461. 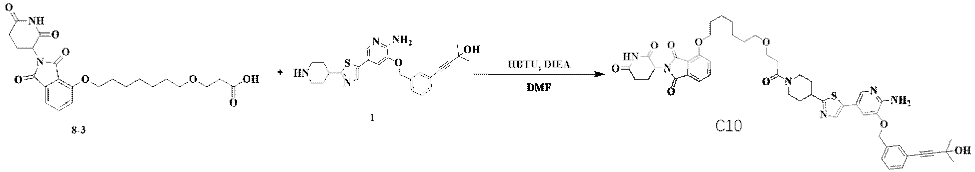 8-3 (23 mg, 0.05 mmol, 1.0 equiv) was dissolved in an appropriate amount of DMF, and the resulting mixture was stirred at 0°C, added with HBTU (34 mg, 0.09 mmol, 2.0 equiv), after 5 min, sequentially added with DIEA (17.2 mg, 0.13 mmol, 3.0 equiv) and compound 1 (20 mg, 0.045 mmol, 1.0 equiv), then heated to room temperature, and stirred overnight. The reaction system was diluted with ethyl acetate, and extracted with water and saturated brine for 3 times. The resulting organic phase was dried over anhydrous sodium sulfate, filtered under vacuum with a sand-core funnel, and concentrated by rotary evaporation. The resulting crude product was purified by silica gel column chromatography (methanol:dichloromethane = 1:20, v/v) to give a final product (28 mg, yield: 70%); LC-MS: m/z = 891.

NMR: ¹H NMR (400 MHz, CDCl₃) δ 7.75 (s, 1H), 7.70 - 7.55 (m, 2H), 7.47 (s, 1H), 7.44 - 7.35 (m, 2H), 7.36 - 7.28 (m, 2H), 7.16 (dd, J = 8.4, 5.1 Hz, 1H), 7.05 (d, J = 1.5 Hz, 1H), 5.68 (s, 2H), 5.04 (s, 2H), 4.97 - 4.87 (m, 2H), 4.66 (d, J = 13.2 Hz, 1H), 4.28 - 4.05 (m, 3H), 4.00 (d, J = 12.9 Hz, 1H), 3.82 - 3.62 (m, 3H), 3.54 - 3.35 (m, 4H), 3.31 - 3.08 (m, 2H), 2.91 - 2.45 (m, 9H), 2.32 - 1.95 (m, 4H), 1.90 - 1.63 (m, 5H), 1.61 (s, 6H). ¹³C NMR (101 MHz, CDCl₃) δ 172.76, 171.96, 169.74, 168.64, 167.15, 165.69, 156.61, 150.36, 141.31, 136.77, 136.44, 135.80, 135.29, 133.79, 131.70, 130.91, 128.79, 127.46, 123.43, 118.85, 117.27, 117.05, 115.64, 115.11, 94.89, 81.39, 71.20, 70.08, 69.36, 67.11, 66.15, 65.41, 49.10, 45.64, 41.48, 40.58, 35.27, 33.73, 32.78, 32.14, 31.46, 29.53, 29.37, 29.04, 28.78, 26.03, 25.78, 22.64.

### Example 11: Preparation of Compound C11

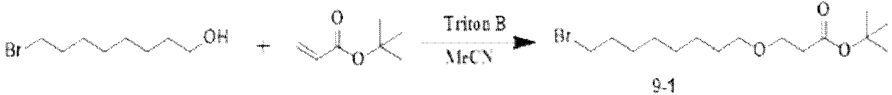

To a solution of 8-bromo-1-octanol (260 mg, 0.78 mmol, 1.0 equiv) in acetonitrile were added *tert-butyl* acrylate (200 mg, 1.56 mmol, 2.0 equiv) and Triton B (10 mg, 0.09 mmol, 1.0 equiv, 40% aqueous solution), and the resulting mixutre was stirred at room temperature for 48 h. Color was developed with KMnO₄ to determine whether the reaction was completed or not. Then the solvent was removed on a rotary evaporator. The residue was purified by silica gel column chromatography (petroleum ether:ethyl acetate = 50:1, v/v) to give a target product 9-1 (198.4 mg, yield: 75%) in the form of an oily liquid.

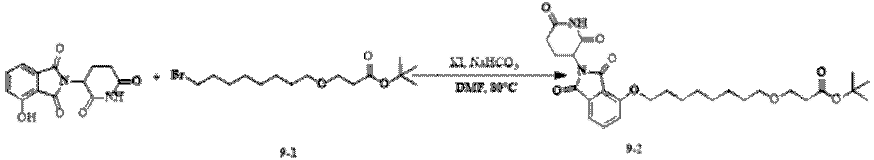

2-(2,6-dioxopiperidin-3-yl) -4-hydroxyiso-1,3-dione (150 mg, 0.55 mmol, 1.0 equiv), potassium iodide (9 mg, 0.054 mmol, 0.1 equiv) and sodium bicarbonate (92 mg, 1.1 mmol, 2.0 equiv) were dissolved in DMF, and the resulting mixture was added with 9-1 (184 mg, 0.55 mmol, 1.0 equiv), heated to 80°C and reacted overnight, and then the heating was stopped. The reaction system was cooled to room temperature, diluted with an appropriate amount of ethyl acetate, and extracted with saturated brine for 5 times. The resulting organic phase was dried over anhydrous sodium sulfate, filtered, and concentrated by rotary evaporation. The resulting crude product was purified by silica gel column chromatography (petroleum ether: acetone = 3:1, v/v) to give a final product 9-2 (160.5 mg, yield: 55%); LC-MS: m/z = 531.

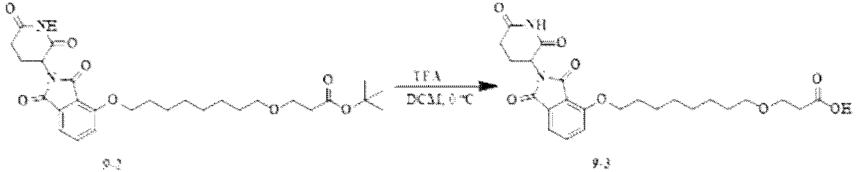

9-2 (160.5 mg, 0.30 mmol, 1.0 equiv) was dissolved in an appropriate amount of dichloromethane, and the resulting mixture was added with 2-fold volume of trifluoroacetic acid at 0°C and stirred for 2 h. After the reaction was completed as monitored by LC-MS, dichloromethane and trifluoroacetic acid were removed on a rotary evaporator. The residue was dried in a vacuum pump for 2 h to remove the residual solvent and acid to give a crude product 9-3 (143 mg) which was used in the next step directly without further purification; LC-MS: m/z = 475.

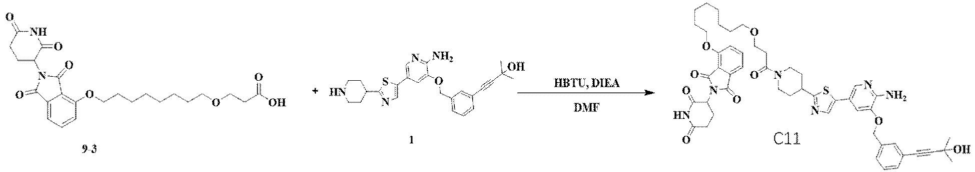

9-3 (23.7 mg, 0.05 mmol, 1.0 equiv) was dissolved in an appropriate amount of DMF, and the resulting mixture was stirred at 0°C, added with HBTU (34 mg, 0.09 mmol, 2.0 equiv), after 5 min, sequentially added with DIEA (17.2 mg, 0.13 mmol, 3.0 equiv) and compound 1 (20 mg, 0.045 mmol, 1.0 equiv), then heated to room temperature, and stirred overnight. The reaction system was diluted with ethyl acetate, and extracted with water and saturated brine for 3 times. The resulting organic phase was dried over anhydrous sodium sulfate, filtered under vacuum with a sand-core funnel, and concentrated by rotary evaporation. The resulting crude product was purified by silica gel column chromatography (methanol:dichloromethane = 1:20, v/v) to give a final product (21.6mg, yield: 53%); LC-MS: m/z = 905.

NMR: ¹H NMR(400 MHz, CDCl₃) δ 10.12 (s, 1H), 7.85 (d, J = 1.2 Hz, 1H), 7.78 - 7.59 (m, 2H), 7.50 (s, 1H), 7.46 - 7.39 (m, 2H), 7.38 - 7.30 (m, 2H), 7.20 (d, J = 8.5 Hz, 1H), 7.07 (d, J = 1.4 Hz, 1H), 5.25 (s, 1H), 5.07 (s, 2H), 4.95 (dd, J = 12.0, 5.2 Hz, 1H), 4.68 (d, J = 13.4 Hz, 1H), 4.15 (t, J = 6.4 Hz, 2H), 4.02 (d, J = 13.5 Hz, 1H), 3.75 (dd, J = 10.9, 6.6 Hz, 2H), 3.45 (t, J = 6.6 Hz, 2H), 3.21 (dd, J = 23.6, 11.8 Hz, 2H), 3.05 - 2.40 (m, 8H), 2.33 - 2.00 (m, 4H), 2.00 - 1.68 (m, 4H), 1.64 (s, 6H), 1.60 - 1.40 (m, 5H), 1.33 (s, 4H). ¹³C NMR (101 MHz, CDCl₃) δ 172.50, 171.98, 171.93, 169.67, 167.16, 165.70, 156.67, 150.28, 141.12, 136.74, 136.60, 136.41, 136.08, 135.97, 135.58, 133.82, 131.66, 130.89, 128.76, 127.43, 123.43, 118.92, 117.62, 117.12, 117.10, 115.64, 115.07, 94.86, 81.42, 71.30, 70.01, 69.39, 67.10, 65.34, 49.12, 45.60, 41.46, 40.61, 33.76, 32.80, 32.09, 31.46, 29.60, 29.23, 29.12, 28.83, 26.02, 25.68, 22.66.

### Example 12: Preparation of Compound C12

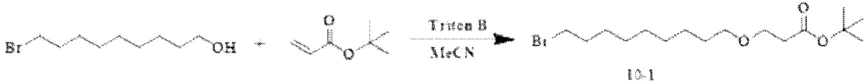

To a solution of 9-bromo-1-nonanol (260 mg, 0.78 mmol, 1.0 equiv) in acetonitrile were added *tert-butyl* acrylate (200 mg, 1.56 mmol, 2.0 equiv) and Triton B (10 mg, 0.09 mmol, 1.0 equiv, 40% aqueous solution), and the resulting mixture was stirred at room temperature for 48 h. Color was developed with KMnO₄ to determine whether the reaction was completed or not. The solvent was removed on a rotary evaporator. The residue was purified by silica gel column chromatography (petroleum ether:ethyl acetate = 50:1, v/v) to give a target product 10-1 (274 mg, yield: 73%) in the form of an oily liquid.

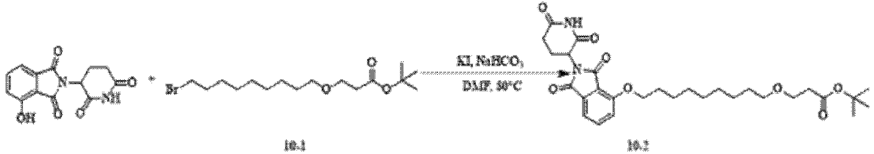

2-(2,6-dioxopiperidin-3-yl) -4-hydroxyiso-1,3-dione (150 mg, 0.55 mmol, 1.0 equiv), potassium iodide (9 mg, 0.054 mmol, 0.1 equiv) and sodium bicarbonate (92 mg, 1.1 mmol, 2.0 equiv) were dissolved in DMF, and the resulting mixture was added with 10-1 (192 mg, 0.55 mmol, 1.0 equiv), heated to 80°C and reacted overnight, and then the heating was stopped. The reaction system was cooled to room temperature, diluted with an appropriate amount of ethyl acetate, and extracted with saturated brine for 5 times. The resulting organic phase was dried over anhydrous sodium sulfate, filtered, and concentrated by rotary evaporation. The resulting crude product was purified by silica gel column chromatography (petroleum ether: acetone = 3:1, v/v) to give a final product 10-2 (176.7 mg, yield: 59%); LC-MS: m/z = 545. 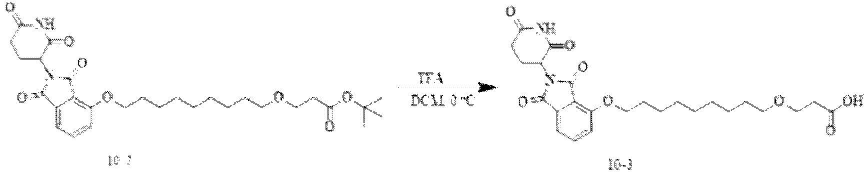 10-2 (176.7 mg, 0.32 mmol, 1.0 equiv) was dissolved in an appropriate amount of dichloromethane, and the resulting mixture was added with 2-fold volume of trifluoroacetic acid at 0°C and stirred for 2 h. After the reaction was completed as monitored by LC-MS, dichloromethane and trifluoroacetic acid were removed on a rotary evaporator. The residue was dried in a vacuum pump for 2 h to remove the residual solvent and acid to give a crude product 10-3 (158.5 mg) which was used in the next step directly without further purification; LC-MS: m/z = 489.

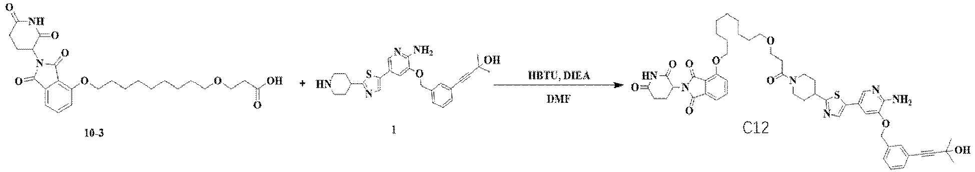

10-3 (24.2 mg, 0.05 mmol, 1.0 equiv) was dissolved in an appropriate amount of DMF, and the resulting mixture was stirred at 0°C, added with HBTU (34 mg, 0.09 mmol, 2.0 equiv), after 5 min, sequentially added with DIEA (17.2 mg, 0.13 mmol, 3.0 equiv) and compound 1 (20 mg, 0.045 mmol, 1.0 equiv), then heated to room temperature, and stirred overnight. The reaction system was diluted with ethyl acetate, and extracted with water and saturated brine for 3 times. The resulting organic phase was dried over anhydrous sodium sulfate, filtered under vacuum with a sand-core funnel, and concentrated by rotary evaporation. The resulting crude product was purified by silica gel column chromatography (methanol:dichloromethane = 1:20, v/v) to give a final product 10-4 (27.7 mg, yield: 67%); LC-MS: m/z = 919.

NMR: ¹H NMR (400 MHz, CDCl₃) δ 7.77 (s, 1H), 7.70 - 7.58 (m, 2H), 7.48 (s, 1H), 7.45 - 7.36 (m, 2H), 7.36 - 7.30 (m, 1H), 7.19 (d, J = 8.4 Hz, 2H), 7.07 (s, 1H), 5.72 (s, 2H), 5.06 (d, J = 3.6 Hz, 2H), 5.01 - 4.87 (m, 2H), 4.66 (d, J = 12.7 Hz, 1H), 4.12 (dd, J = 12.1, 5.1 Hz, 4H), 4.01 (d, J = 12.1 Hz, 1H), 3.73 (m, 3H), 3.45 (dd, J = 12.5, 6.3 Hz, 3H), 3.34 - 3.07 (m, 2H), 2.76 (m, 11H), 2.27 - 1.96 (m, 5H), 1.91 - 1.66 (m, 5H), 1.62 (s, 6H). ¹³C NMR (101 MHz, CDCl₃) δ 172.69, 171.92, 169.72, 168.68, 167.15, 165.69, 156.66, 150.46, 141.31, 136.77, 136.43, 135.83, 135.31, 133.78, 131.67, 130.86, 128.77, 127.40, 123.44, 118.93, 118.89, 117.21, 117.06, 115.62, 115.16, 94.95, 81.36, 71.30, 71.19, 70.07, 69.43, 69.39, 67.07, 66.11, 65.38, 65.35, 49.10, 45.59, 41.45, 40.53, 36.54, 35.21, 33.73, 32.75, 32.08, 31.46, 29.61, 29.49, 29.36, 29.29, 29.24, 29.12, 28.84, 26.07, 26.00, 25.73, 25.71, 22.63.

### Example 13: Preparation of Compound C13

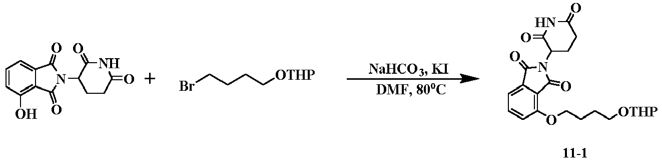

2-(2,6-dioxopiperidin-3-yl)-4-hydroxyiso-1,3-dione (100 mg, 0.4 mmol, 1.0 equiv), potassium iodide (6.1 mg, 0.03 mmol, 0.1 equiv) and sodium bicarbonate (61.3 mg, 0.4 mmol, 2.0 equiv) were dissolved in DMF, and the resulting mixture was added with 2-(4-bromobutoxy) tetrahydro-2*H*-pyran (113.8 mg, 0.48 mmol, 1.2 equiv), then heated to 80°C and reacted overnight, and then the heating was stopped. The reaction system was cooled to room temperature, diluted with an appropriate amount of ethyl acetate, and extracted with saturated saline for 5 times. The resulting organic phase was dried over anhydrous sodium sulfate, filtered, and concentrated by rotary evaporation. The resulting crude product was purified by silica gel column chromatography (petroleum ether:ethyl acetate = 1:2, v/v) to give a final product (113.6 mg, yield: 66%); LC-MS: m/z = 431.2.

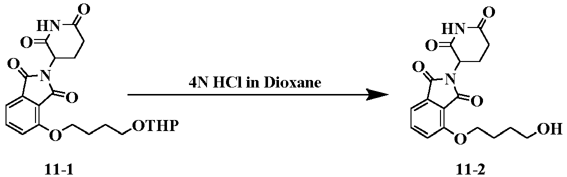

11-1 (113.6 mg, 0.26 mmol) was weighed into a round-bottom flask, a 4 M dioxane hydrochloride solution was added, and the resulting mixture was stirred at room temperature until the reaction was completed. The redundant solvent was removed by rotary evaporation to give a final product (84 mg, yield: 93%); LC-MS: m/z = 347.1.

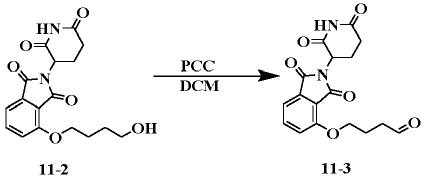

11-2 (84 mg, 0.24 mmol, 1.0 equiv) and PCC (103.5 mg, 0.48 mmol, 2.0 equiv) were dissolved in an appropriate amount of dichloromethane, and the resulting mixture was stirred at room temperature for 6 h. The reaction system was added with an appropriate amount of silica gel, concentrated by rotary evaporation, and purified by silica gel column chromatography (petroleum ether:ethyl acetate = 1:2, v/v) to give a final product (81 mg, yield: 98%); LC-MS: m/z = 345.1.

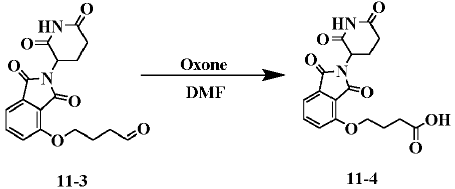

11-3 (81 mg, 0.24 mmol, 1.0 equiv) and Oxone (140.1 mg, 0.24 mol, 1.0 equiv) were dissolved in an appropriate amount of DMF, and the resulting mixture was stirred at room temperature overnight. The reaction system was diluted with ethyl acetate and extracted with water and saturated brine for 3 times. The resulting organic phase was dried over anhydrous sodium sulfate, filtered, and concentrated by rotary evaporation to give a final product (70.9 mg, yield: 82%); LC-MS: m/z = 361.1.

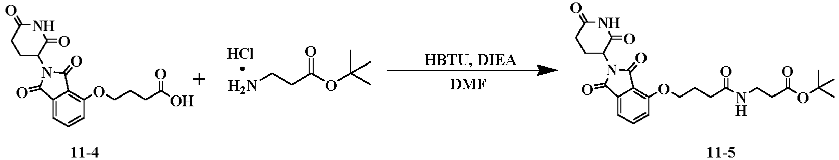

11-4 (50 mg, 0.14 mmol, 1.0 equiv) was dissolved in an appropriate amount of DMF, the resulting mixture was stirred at 0°C, added with HBTU (105.8 mg, 0.28 mmol, 2.0 equiv), after 5 min, sequentially added with DIEA (71.5 mg, 0.56 mmol, 4.0 equiv) and β-alanine *tert-*butyl ester hydrochloride (25.4 mg, 0.14 mmol,1.0 equiv), then heated to room temperature, and stirred overnight. The reaction system was diluted with ethyl acetate, and extracted with water and saturated saline for 3 times. The resulting organic phase was dried over anhydrous sodium sulfate, filtered under vacuum with a sand-core funnel, and concentrated by rotary evaporation. The resulting crude product was purified by silica gel column chromatography to give a final product (44.4 mg, yield: 65%); LC-MS: m/z = 488.2.

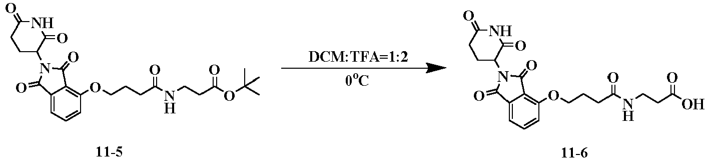

11-5 (44.4 mg, 0.09 mmol) was dissolved in an appropriate amount of dichloromethane, and the resulting mixture was added with 2-fold volume of trifluoroacetic acid at 0°C and stirred for 2 h. After the reaction was completed as monitored by LC-MS, dichloromethane and trifluoroacetic acid were removed on a rotary evaporator. The residue was dried in a vacuum pump for 2 h to remove the residual solvent and acid to give a crude product (38.8 mg) which was used in the next step directly without further purification; LC-MS: m/z = 432.1.

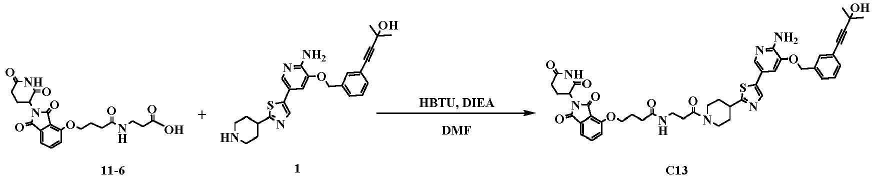

11-6 (10.8 mg, 25 mmol, 1.0 equiv) was dissolved in an appropriate amount of DMF, the resulting mixture was stirred at 0°C, added with HBTU (17 mg, 0.045 mmol, 2.0 equiv), after 5 min, sequentially added with DIEA (8.6 mg, 0.065 mmol, 3.0 equiv) and compound 1 (10 mg, 0.023 mmol, 1.0 equiv), then heated to room temperature, and stirred overnight. The reaction system was diluted with ethyl acetate, and extracted with water and saturated saline for 3 times. The resulting organic phase was dried over anhydrous sodium sulfate, filtered under vacuum with a sand-core funnel, and concentrated by rotary evaporation. The resulting crude product was purified by silica gel column chromatography (methanol:dichloromethane = 1:20, v/v) to give a final product (7.9 mg, yield: 40%); LC-MS: m/z = 862.3.

NMR: ¹H NMR(400 MHz, CDCl3) δ 7.86 (d*, J* = 1.8 Hz, 1H), 7.76 - 7.64 (m, 2H), 7.54 (s, 1H), 7.51 - 7.42 (m, 2H), 7.41 - 7.35 (m, 2H), 7.24 (dd, *J* = 8.4, 4.4 Hz, 1H), 7.11 (d*, J* = 7.9 Hz, 1H), 5.33 (s, 1H), 5.13 (s, 2H), 5.02 - 4.93 (m, 1H), 4.76 - 4.57 (m, 2H), 4.34 - 4.15 (m, 2H), 4.13 - 3.92 (m, 2H), 3.85 - 3.66 (m, 1H), 3.39 - 3.20 (m, 2H), 3.20 - 3.00 (m, 2H), 2.92 - 2.64 (m, 6H), 2.29 - 2.11 (m, 4H), 1.79 - 1.72 (m, 5H), 1.66 (s, 6H).

### Example 14: Preparation of Compound C14

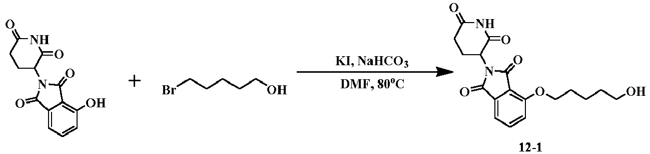

2-(2,6-dioxopiperidin-3-yl)-4-hydroxyiso-1,3-dione (100 mg, 0.4 mmol, 1.0 equiv), potassium iodide (6.1 mg, 0.03 mmol, 0.1 equiv) and sodium bicarbonate (61.3 mg, 0.4 mmol, 2.0 equiv) were dissolved in DMF, and the resulting mixture was added with 5-bromo-1-pentanol (80.2 mg, 0.48 mmol, 1.2 equiv), heated to 80°C and reacted overnight, and then the heating was stopped. The reaction system was cooled to room temperature, diluted with an appropriate amount of ethyl acetate, and extracted with saturated saline for 5 times. The resulting organic phase was dried over anhydrous sodium sulfate, filtered, and concentrated by rotary evaporation. The resulting crude product was purified by silica gel column chromatography (petroleum ether:ethyl acetate = 1:2, v/v) to give a final product (110.7 mg, yield: 64%); LC-MS: m/z = 361.1.

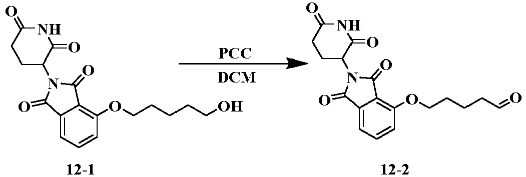

12-1 (110.7 mg, 0.31 mmol, 1.0 equiv) and PCC (133.7 mg, 0.62 mmol, 2.0 equiv) were dissolved in an appropriate amount of dichloromethane, and the resulting mixture was stirred at room temperature for 6 h. The reaction system was added with an appropriate amount of silica gel, concentrated by rotary evaporation, and purified by silica gel column chromatography (petroleum ether:ethyl acetate = 1:2, v/v) to give a final product (108.9 mg, yield: 98%); LC-MS: m/z = 359.1.

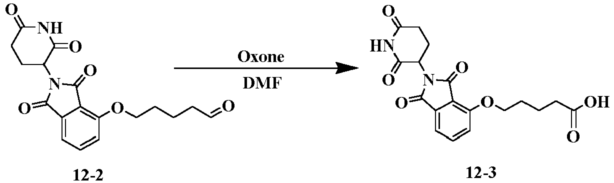

12-2 (108.9 mg, 0.3 mmol, 1.0 equiv) and Oxone (175.1 mg, 0.3 mol, 1.0 equiv) were dissolved in an appropriate amount of DMF, and the resulting mixture was stirred at room temperature overnight. The reaction system was diluted with ethyl acetate and extracted with water and saturated brine for 3 times. The resulting organic phase was dried over anhydrous sodium sulfate, filtered, and concentrated by rotary evaporation to give a final product (88.7 mg, yield: 79%); LC-MS: m/z = 375.1.

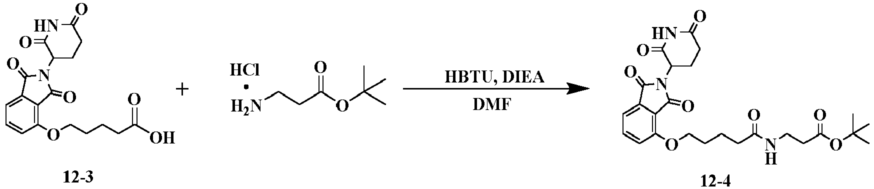

12-3 (52.4 mg, 0.14 mmol, 1.0 equiv) was dissolved in an appropriate amount of DMF, the resulting mixture was stirred at 0°C, added with HBTU (105.8 mg, 0.28 mmol, 2.0 equiv), after 5 min, sequentially added with DIEA (71.5 mg, 0.56 mmol, 4.0 equiv) and β-alanine *tert-butyl* ester hydrochloride (25.4 mg, 0.14 mmol,1.0 equiv), then heated to room temperature, and stirred overnight. The reaction system was diluted with ethyl acetate and extracted with water and saturated brine for 3 times. The resulting organic phase was dried over anhydrous sodium sulfate, filtered under vacuum with a sand-core funnel, and concentrated by rotary evaporation. The resulting crude product was purified by silica gel column chromatography to give a final product (47 mg, yield: 67%); LC-MS: m/z = 502.2.

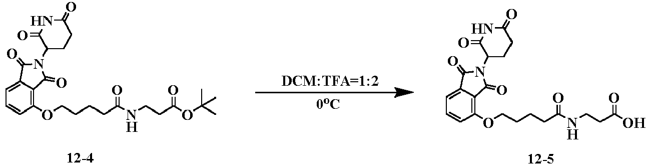

12-4 (47 mg, 0.09 mmol) was dissolved in an appropriate amount of dichloromethane, and the resulting mixture was added with 2-fold volume of trifluoroacetic acid at 0°C and stirred for 2 h. After the reaction was completed as monitored by LC-MS, dichloromethane and trifluoroacetic acid were removed on a rotary evaporator. The residue was dried in a vacuum pump for 2 h to remove the residual solvent and acid to give a crude product (40.1 mg) which was used in the next step directly without further purification; LC-MS: m/z = 446.2.

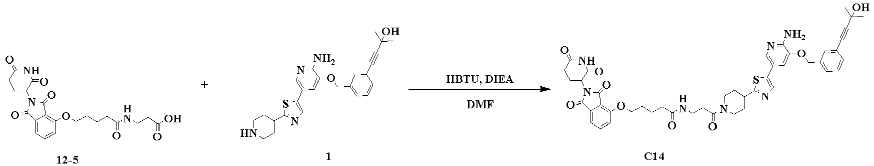

12-5 (11.1 mg, 0.025 mmol, 1.0 equiv) was dissolved in an appropriate amount of DMF, the resulting mixture was stirred at 0°C, added with HBTU (17 mg, 0.045 mmol, 2.0 equiv), after 5 min, sequentially added with DIEA (8.6 mg, 0.065 mmol, 3.0 equiv) and compound 1 (10 mg, 0.023 mmol, 1.0 equiv), then heated to room temperature, and stirred overnight. The reaction system was diluted with ethyl acetate, and extracted with water and saturated saline for 3 times. The resulting organic phase was dried over anhydrous sodium sulfate, filtered under vacuum with a sand-core funnel, and concentrated by rotary evaporation. The resulting crude product was purified by silica gel column chromatography (methanol:dichloromethane = 1:20, v/v) to give a final product (9.4 mg, yield: 43%); LC-MS: m/z = 876.2.

NMR: ¹H NMR (400 MHz, CDCl₃) δ 7.86 (s, 1H), 7.75 - 7.63 (m, 2H), 7.54 (s, 1H), 7.51 - 7.42 (m, 2H), 7.42 - 7.34 (m, 2H), 7.24 (d, *J* = 8.6 Hz, 1H), 7.12 (s, 1H), 5.46 (d, *J* = 16.4 Hz, 1H), 5.12 (s, 2H), 5.04 - 4.94 (m, 1H), 4.68 (dd, *J* = 33.0, 13.7 Hz, 2H), 4.31 - 4.16 (m, 2H), 4.00 (dd, *J* = 26.4, 13.7 Hz, 2H), 3.83 - 3.68 (m, 1H), 3.35 - 3.19 (m, 2H), 3.18 - 3.03 (m, 1H), 3.01 - 2.65 (m, 6H), 2.51 - 2.30 (m, 3H), 2.26 - 2.10 (m, 4H), 1.80 - 1.74 (m, 5H), 1.65 (s, 6H).

### Example 15: Preparation of Compound C15

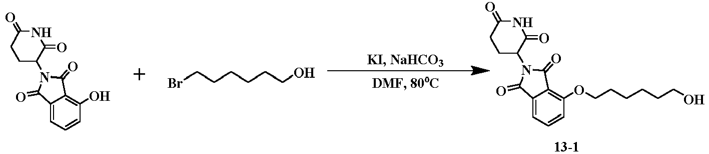

2-(2,6-dioxopiperidin-3-yl)-4-hydroxyiso-1,3-dione (100 mg, 0.4 mmol, 1.0 equiv), potassium iodide (6.1 mg, 0.03 mmol, 0.1 equiv) and sodium bicarbonate (61.3 mg, 0.4 mmol, 2.0 equiv) were dissolved in DMF, and the resulting mixture was added with 6-bromo-1-hexanol (86.9 mg, 0.48 mmol, 1.2 equiv), heated to 80°C and reacted overnight, and then the heating was stopped. The reaction system was cooled to room temperature, diluted with an appropriate amount of ethyl acetate, and extracted with saturated saline for 5 times. The resulting organic phase was dried over anhydrous sodium sulfate, filtered, and concentrated by rotary evaporation. The resulting crude product was purified by silica gel column chromatography (petroleum ether:ethyl acetate = 1:2, v/v) to give a final product (120.4 mg, yield: 67%); LC-MS: m/z = 375.2. 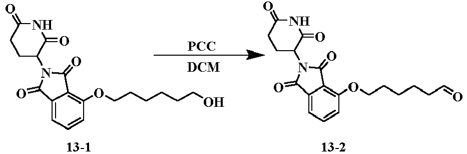 13-1 (120.4 mg, 0.32 mmol, 1.0 equiv) and PCC (138 mg, 0.64 mmol, 2.0 equiv) were dissolved in an appropriate amount of dichloromethane, and the resulting mixture was stirred at room temperature for 6 h. The reaction system was added with an appropriate amount of silica gel, concentrated by rotary evaporation, and purified by silica gel column chromatography (petroleum ether:ethyl acetate = 1:2, v/v) to give a final product (118 mg, yield: 99%); LC-MS: m/z = 373.1. 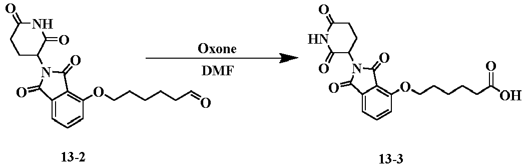 13-2 (118 mg, 0.32 mmol, 1.0 equiv) and Oxone (186.8 mg, 0.32 mol, 1.0 equiv) were dissolved in an appropriate amount of DMF, and the resulting mixture was stirred at room temperature overnight. The reaction system was diluted with ethyl acetate and extracted with water and saturated brine for 3 times. The resulting organic phase was dried over anhydrous sodium sulfate, filtered, and concentrated by rotary evaporation to give a final product (100.7 mg, yield: 81%); LC-MS: m/z = 389.1. 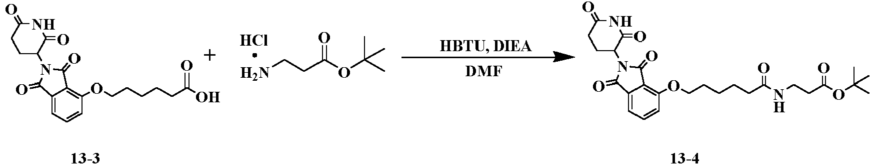 13-3 (54.4 mg, 0.14 mmol, 1.0 equiv) was dissolved in an appropriate amount of DMF, the resulting mixture was stirred at 0°C, added with HBTU (105.8 mg, 0.28 mmol, 2.0 equiv), after 5 min, sequentially added with DIEA (71.5 mg, 0.56 mmol, 4.0 equiv) and β-alanine *tert-*butyl ester hydrochloride (25.4 mg, 0.14 mmol, 1.0 equiv), then heated to room temperature, and stirred overnight. The reaction system was diluted with ethyl acetate and extracted with water and saturated brine for 3 times. The resulting organic phase was dried over anhydrous sodium sulfate, filtered under vacuum with a sand-core funnel, and concentrated by rotary evaporation. The resulting crude product was purified by silica gel column chromatography to give a final product (42.6 mg, yield: 59%); LC-MS: m/z = 516.3. 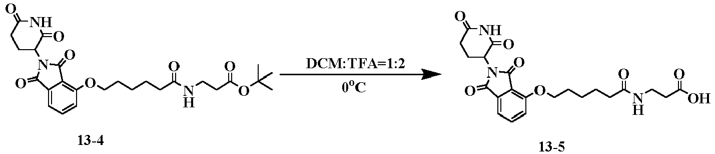 13-4 (42.6 mg, 0.08 mmol) was dissolved in an appropriate amount of dichloromethane, and the resulting mixture was added with 2-fold volume of trifluoroacetic acid at 0°C and stirred for 2 h. After the reaction was completed as monitored by LC-MS, dichloromethane and trifluoroacetic acid were removed on a rotary evaporator. The residue was dried in a vacuum pump for 2 h to remove the residual solvent and acid to give a crude product (36.8 mg) which was used in the next step directly without further purification; LC-MS: m/z = 460.2. 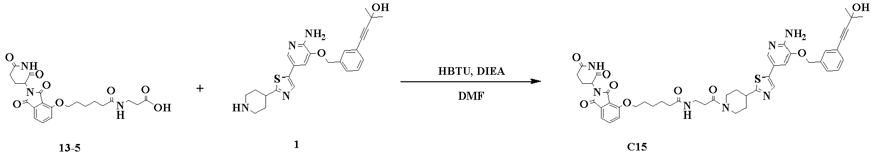 13-5 (11.5 mg, 0.025 mmol, 1.0 equiv) was dissolved in an appropriate amount of DMF, the resulting mixture was stirred at 0°C, added with HBTU (17 mg, 0.045 mmol, 2.0 equiv), after 5 min, sequentially added with DIEA (8.6 mg, 0.065 mmol, 3.0 equiv) and compound 1 (10 mg, 0.023 mmol, 1.0 equiv), then heated to room temperature, and stirred overnight. The reaction system was diluted with ethyl acetate, and extracted with water and saturated saline for 3 times. The resulting organic phase was dried over anhydrous sodium sulfate, and filtered under vacuum with a sand-core funnel. The redundant solvent was removed on a rotary evaporator. The resulting crude product was purified by silica gel column chromatography (methanol:dichloromethane = 1:20, v/v) to give a final product (10.4 mg, yield: 51%); LC-MS: m/z = 890.3.

NMR: ¹H NMR (400 MHz, CDCl₃) δ 7.87 (s, 1H), 7.76 - 7.63 (m, 2H), 7.53 (s, 1H), 7.49 - 7.41 (m, 2H), 7.41 - 7.33 (m, 2H), 7.24 (d*, J* = 8.5 Hz, 1H), 7.10 (s, 1H), 5.32 (s, 1H), 5.24 (d*, J* = 11.7 Hz, 1H), 5.11 (s, 2H), 5.05 (s, 1H), 5.02 - 4.91 (m, 1H), 4.75 - 4.57 (m, 2H), 4.26 - 4.15 (m, 2H), 4.00 (dd, *J* = 27.7, 14.5 Hz, 2H), 3.84 - 3.64 (m, 1H), 3.34 - 3.20 (m, 2H), 3.17 - 3.05 (m, 1H), 2.96 - 2.63 (m, 6H), 2.45 - 2.31 (m, 3H), 2.30 - 2.09 (m, 4H), 1.91 - 1.71 (m, 9H), 1.65 (s, 6H).

### Example 16: Preparation of Compound C16

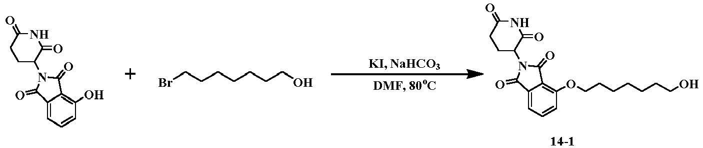

2-(2,6-dioxopiperidin-3-yl)-4-hydroxyiso-1,3-dione (100 mg, 0.4 mmol, 1.0 equiv), potassium iodide (6.1 mg, 0.03 mmol, 0.1 equiv) and sodium bicarbonate (61.3 mg, 0.4 mmol, 2.0 equiv) were dissolved in DMF, and the resulting mixture was added with 7-bromo-1-heptanol (93.6 mg, 0.48 mmol, 1.2 equiv), heated to 80°C and reacted overnight, and then the heating was stopped. The reaction system was cooled to room temperature, diluted with an appropriate amount of ethyl acetate, and extracted with saturated saline for 5 times. The resulting organic phase was dried over anhydrous sodium sulfate, filtered, and concentrated by rotary evaporation. The resulting crude product was purified by silica gel column chromatography (petroleum ether:ethyl acetate = 1:2, v/v) to give a final product (113.7 mg, yield: 61%); LC-MS: m/z = 389.1.

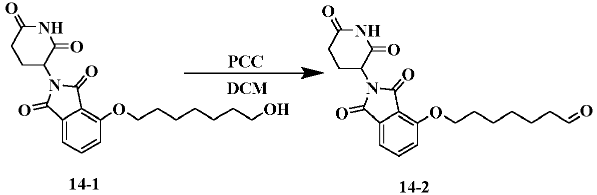

14-1 (113.7 mg, 0.29 mmol, 1.0 equiv) and PCC (125 mg, 0.58 mmol, 2.0 equiv) were dissolved in an appropriate amount of dichloromethane, and the resulting mixture was stirred at room temperature for 6 h. The reaction system was added with an appropriate amount of silica gel, concentrated by rotary evaporation, and purified by silica gel column chromatography (petroleum ether:ethyl acetate = 1:2, v/v) to give a final product (110.9 mg, yield: 99%); LC-MS: m/z = 387.2.

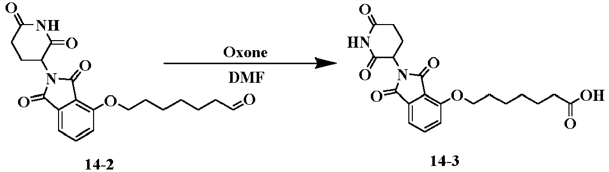

14-2 (110.9 mg, 0.29 mmol, 1.0 equiv) and Oxone (180.6 mg, 0.29 mol, 1.0 equiv) were dissolved in an appropriate amount of DMF, and the resulting mixture was stirred at room temperature overnight. The reaction system was diluted with ethyl acetate and extracted with water and saturated brine for 3 times. The resulting organic phase was dried over anhydrous sodium sulfate, filtered, and concentrated by rotary evaporation to give a final product (99.2 mg, yield: 85%); LC-MS: m/z = 403.2.

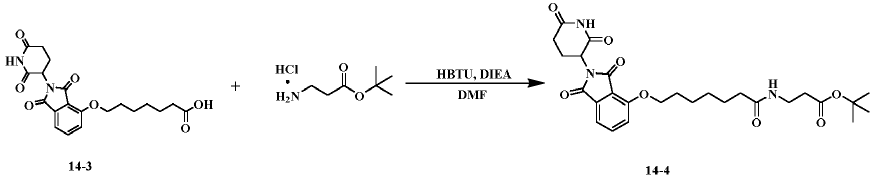

14-3 (54.4 mg, 0.14 mmol, 1.0 equiv) was dissolved in an appropriate amount of DMF, and the resulting mixture was stirred at 0°C, added with HBTU (105.8 mg,0.28 mmol, 2.0 equiv), after 5 min, sequentially added with DIEA (71.5 mg, 0.56 mmol, 4.0 equiv) and β-alanine *tert*-butyl ester hydrochloride (25.4 mg, 0.14 mmol, 1.0 equiv), then heated to room temperature, and stirred overnight. The reaction system was diluted with ethyl acetate, and extracted with water and saturated saline for 3 times. The resulting organic phase was dried over anhydrous sodium sulfate, filtered under vacuum with a sand-core funnel, and concentrated by rotary evaporation. The resulting crude product was purified by silica gel column chromatography to give a final product (47.5 mg, yield: 64%); LC-MS: m/z = 530.3.

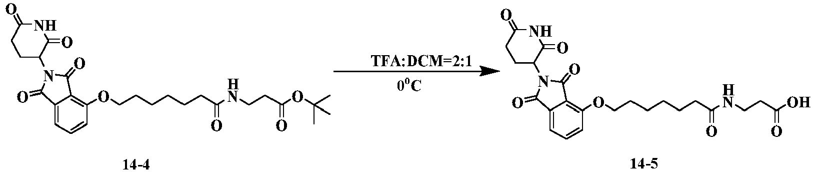

14-4 (47.5 mg, 0.09 mmol) was dissolved in an appropriate amount of dichloromethane, and the resulting mixture was added with 2-fold volume of trifluoroacetic acid at 0°C and stirred for 2 h. After the reaction was completed as monitored by LC-MS, dichloromethane and trifluoroacetic acid were removed on a rotary evaporator. The residue was dried in a vacuum pump for 2 h to remove the residual solvent and acid to give a crude product (42.6 mg) which was used in the next step directly without further purification; LC-MS: m/z = 474.2.

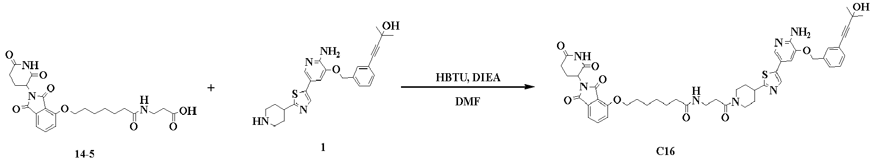

14-5 (11.8 mg, 0.025 mmol, 1.0 equiv) was dissolved in an appropriate amount of DMF, and the resulting mixture was stirred at 0°C, added with HBTU (17 mg, 0.045 mmol, 2.0 equiv), after 5 min, sequentially added with DIEA (8.6 mg, 0.065 mmol, 3.0 equiv) and compound 1 (10 mg, 0.023 mmol, 1.0 equiv), then heated to room temperature, and stirred overnight. The reaction system was diluted with ethyl acetate, and extracted with water and saturated saline for 3 times. The resulting organic phase was dried over anhydrous sodium sulfate, and filtered under vacuum with a sand-core funnel. The redundant solvent was removed on a rotary evaporator. The resulting crude product was purified by silica gel column chromatography (methanol:dichloromethane = 1:20, v/v) to give a final product (8.1 mg, yield: 39%); LC-MS: m/z = 904.3.

NMR: ¹H NMR (400 MHz, MeOD) δ 7.76 (s, 1H), 7.71 (d, *J* = 8.4 Hz, 2H), 7.55 (s, 1H), 7.52 - 7.43 (m, 2H), 7.42 - 7.35 (m, 3H), 7.30 (s, 1H), 5.37 - 5.31 (m, 2H), 5.20 (s, 2H), 5.08 (dd, *J* = 12.1, 4.9 Hz, 2H), 4.61 - 4.51 (m, 2H), 4.42 (dd, *J* = 9.7, 3.9 Hz, 1H), 4.25 - 4.19 (m, 2H), 4.05 - 3.98 (m, 1H), 3.81 - 3.72 (m, 2H), 3.64 - 3.56 (m, 3H), 3.50 - 3.45 (m, 2H), 2.87 - 2.62 (m, 6H), 2.60 - 2.52 (m, 2H), 2.49 - 2.41 (m, 2H), 2.15 - 2.03 (m, 5H), 1.56 (s, 6H).

### Example 17: Preparation of Compound C17

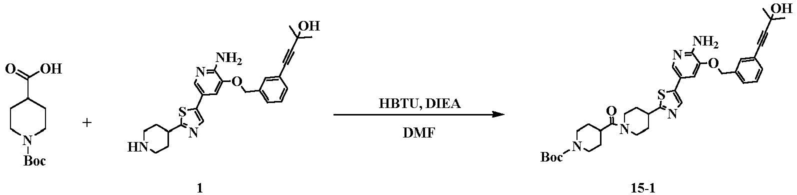

1-Boc-4-piperidineacetic acid (50.4 mg, 0.22 mmol, 1.0 equiv) was dissolved in an appropriate amount of DMF, and the resulting mixture was stirred at 0°C, added with HBTU (166.2 mg, 0.44 mmol, 2.0 equiv), after 5 min, sequentially added with DIEA (84.3 mg, 0.66 mmol, 3.0 equiv) and Compound 1 (100 mg, 0.22 mmol, 1.0 equiv), then heated to room temperature, and stirred overnight. The reaction system was diluted with ethyl acetate, and extracted with water and saturated saline for 3 times. The resulting organic phase was dried over anhydrous sodium sulfate, filtered under vacuum with a sand-core funnel, and concentrated by rotary evaporation. The resulting crude product was purified by silica gel column chromatography to give a final product (117.2 mg, yield: 81%); LC-MS: m/z = 660.3. 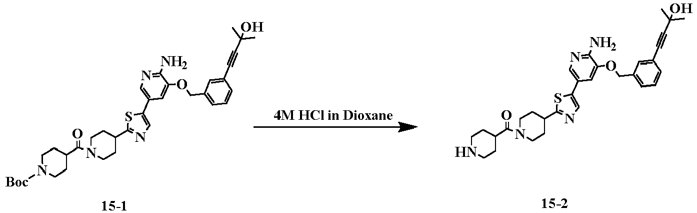 15-1 (117.2 mg, 0.18 mmol) was weighed into a round-bottom flask, a 4 M dioxane hydrochloride solution was added, and the resulting mixture was stirred at room temperature until the reaction was completed. The redundant solvent was removed by rotary evaporation to give a final product in the form of a hydrochloride salt as a yellow solid (103.1 mg, yield: 96%); LC-MS: m/z = 560.3. 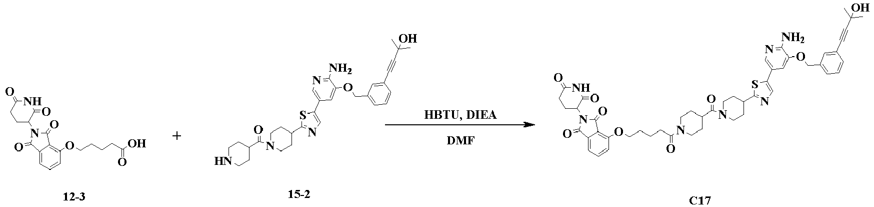 12-3 (9.4 mg, 0.025 mmol, 1.0 equiv) was dissolved in an appropriate amount of DMF, and the resulting mixture was stirred at 0°C, added with HBTU (17 mg, 0.045 mmol, 2.0 equiv), after 5 min, sequentially added with DIEA (8.6 mg, 0.065 mmol, 3.0 equiv) and 15-2 (13 mg, 0.023 mmol, 1.0 equiv), then heated to room temperature, and stirred overnight. The reaction system was diluted with ethyl acetate, and extracted with water and saturated saline for 3 times. The resulting organic phase was dried over anhydrous sodium sulfate, and filtered under vacuum with a sand-core funnel. The redundant solvent was removed on a rotary evaporator. The resulting crude product was purified by silica gel column chromatography (methanol:dichloromethane = 1:20, v/v) to give a final product (7.4 mg, yield 35%); LC-MS: m/z = 916.4. NMR: ¹H NMR (400 MHz, MeOD) δ 7.75 (s, 1H), 7.73 - 7.66 (m, 2H), 7.55 (s, 1H), 7.51 - 7.45 (m, 1H), 7.41 - 7.33 (m, 4H), 7.29 (d, *J* = 1.8 Hz, 1H), 5.20 (s, 2H), 5.09 (dd, *J* = 12.9, 5.0 Hz, 1H), 4.58 (d, *J* = 14.7 Hz, 1H), 4.25 - 4.15 (m, 2H), 4.04 (d, *J* = 13.4 Hz, 1H), 3.49 (t, *J* = 6.5 Hz, 2H), 3.31 - 3.19 (m, 5H), 2.92 - 2.65 (m, 6H), 2.65 - 2.54 (m, 2H), 2.34 (t, *J* = 6.7 Hz, 2H), 2.21 - 2.07 (m, 5H), 1.91 - 1.80 (m, 4H), 1.79 - 1.61 (m, 3H), 1.56 (s, 6H).

### Example 18: Preparation of Compound C18

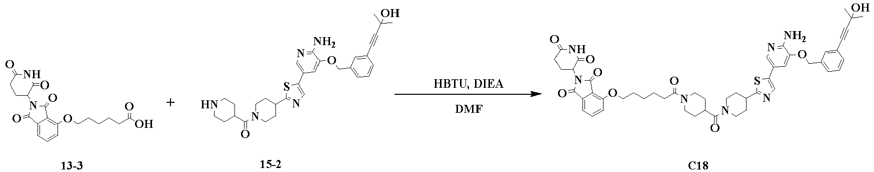

13-3 (11.7 mg, 0.03 mmol, 1.0 equiv) was dissolved in an appropriate amount of DMF, and the resulting mixture was stirred at 0°C, added with HBTU (22.7 mg, 0.045 mmol, 2.0 equiv), after 5 min, sequentially added with DIEA (11.9 mg, 0.065 mmol, 3.0 equiv) and 15-2 (17 mg, 0.03 mmol, 1.0 equiv), then heated to room temperature, and stirred overnight. The reaction system was diluted with ethyl acetate, and extracted with water and saturated saline for 3 times. The resulting organic phase was dried over anhydrous sodium sulfate, and filtered under vacuum with a sand-core funnel. The redundant solvent was removed on a rotary evaporator. The resulting crude product was purified by silica gel column chromatography (methanol:dichloromethane = 1:20, v/v) to give a final product (14.2 mg, yield: 51%); LC-MS: m/z = 930.3.

NMR: ¹H NMR (400 MHz, CDCl₃) δ 10.68 (d, *J* = 59.9 Hz, 1H), 7.83 (s, 1H), 7.68 - 7.59 (m, 2H), 7.48 (s, 1H), 7.45 - 7.28 (m, 4H), 7.19 (d, *J* = 8.5 Hz, 1H), 7.05 (s, 1H), 6.69 (t, *J* = 6.1 Hz, 1H), 5.18 (d, *J* = 8.2 Hz, 2H), 5.05 (s, 2H), 5.00 - 4.92 (m, 1H), 4.69 - 4.56 (m, 1H), 4.26 - 4.09 (m, 2H), 3.89 (d, *J* = 13.8 Hz, 1H), 3.68 - 3.53 (m, 1H), 3.52 - 3.36 (m, 1H), 3.28 - 3.07 (m, 2H), 2.92 - 2.66 (m, 5H), 2.54 (t, *J* = 5.5 Hz, 2H), 2.30 - 2.04 (m, 6H), 1.88 - 1.77 (m, 3H), 1.76 - 1.64 (m, 5H), 1.61 (s, 6H), 1.58 - 1.46 (m, 3H); ¹³C NMR (100 MHz, CDCl₃) δ 173.24, 172.30, 172.19, 170.14, 169.08, 167.15, 165.92, 156.56, 150.34, 141.09, 136.88, 136.67, 136.48, 136.00, 135.72, 133.80, 131.66, 130.88, 128.79, 127.43, 123.43, 119.38, 117.61, 117.41, 115.89, 115.00, 94.86, 81.42, 69.99, 69.30, 65.40, 49.25, 45.14, 41.55, 40.55, 36.54, 35.05, 33.04, 32.76, 32.14, 31.58, 31.49, 28.23, 25.62, 25.09, 22.71.

### Example 19: Preparation of Compound C19

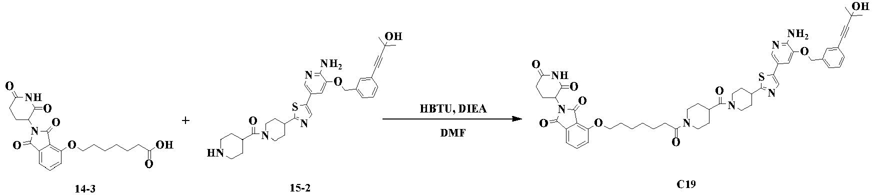

14-3 (12.1 mg, 0.03 mmol, 1.0 equiv) was dissolved in an appropriate amount of DMF, and the resulting mixture was stirred at 0°C, added with HBTU (22.7 mg, 0.045 mmol, 2.0 equiv), after 5 min, sequentially added with DIEA (11.9 mg, 0.065 mmol, 3.0 equiv) and 15-2 (17 mg, 0.03 mmol, 1.0 equiv), then heated to room temperature, and stirred overnight. The reaction system was diluted with ethyl acetate, and extracted with water and saturated saline for 3 times. The resulting organic phase was dried over anhydrous sodium sulfate, and filtered under vacuum with a sand-core funnel. The redundant solvent was removed on a rotary evaporator. The resulting crude product was purified by silica gel column chromatography (methanol:dichloromethane = 1:20, v/v) to give a final product (15.1 mg, yield: 53%); LC-MS: m/z = 944.4.

NMR: ¹H NMR (400 MHz, CDCl₃) δ 10.93 (d, *J* = 86.1 Hz, 2H), 7.79 (s, 1H), 7.69 - 7.55 (m, 2H), 7.44 (s, 1H), 7.42 - 7.27 (m, 4H), 7.16 (d, *J* = 8.5 Hz, 1H), 7.02 (s, 1H), 6.92 - 6.77 (m, 1H), 5.20 (s, 2H), 5.02 (s, 2H), 4.99 - 4.92 (m, 1H), 4.61 (d, *J* = 12.6 Hz, 1H), 4.23 - 4.05 (m, 2H), 3.83 (d, *J* = 13.9 Hz, 1H), 3.62 (dt, *J* = 13.0, 6.5 Hz, 4H), 3.53 - 3.44 (m, 1H), 3.22 - 3.01 (m, 5H), 2.87 - 2.70 (m, 4H), 2.61 - 2.42 (m, 3H), 2.20 - 2.02 (m, 5H), 1.87 - 1.74 (m, 2H), 1.73 - 1.61 (m, 3H), 1.58 (s, 6H), 1.53 - 1.51 (m, 3H); ¹³C NMR (100 MHz, CDCl₃) δ 173.45, 172.27, 172.16, 170.26, 169.32, 167.16, 165.82, 156.59, 150.31, 141.09, 136.85, 136.66, 136.47, 136.04, 135.72, 133.82, 131.65, 130.85, 128.78, 127.44, 123.44, 119.03, 117.62, 117.18, 115.70, 114.97, 94.92, 81.39, 69.95, 69.25, 65.35, 49.21, 45.07, 41.59, 40.44, 35.94, 34.94, 32.98, 32.61, 32.07, 31.59, 31.49, 28.34, 28.20, 25.25, 25.01, 22.77.

### Example 20: Preparation of Compound C20

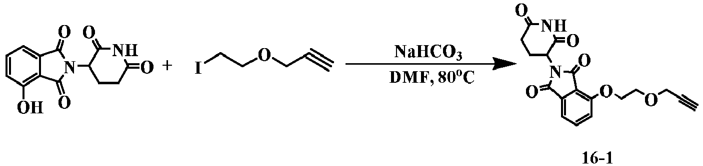

2-(2,6-dioxopiperidin-3-yl)-4-hydroxyiso-1,3-dione (200 mg, 0.73 mmol, 1.0 equiv) and sodium bicarbonate (184 mg, 2.19 mmol, 3.0 equiv) were dissolved in DMF, and the resulting mixture was added dropwise with 3-(2-iodoethoxy)prop-1-yne (184 mg, 0.88 mmol, 1.2 equiv), then heated to 80°C and stirred overnight. When the reaction was completed, the reaction system was diluted with an appropriate amount of ethyl acetate, and sequentially extracted a mixed solution of water and a saturated sodium bicarbonate solution (water: saturated sodium bicarbonate solution = 1:5, v/v) and saturated saline. The resulting organic phase was dried over anhydrous sodium sulfate, filtered under vacuum with a sand-core funnel, concentrated by rotary evaporation, and purified by silica gel column chromatography (acetone:petroleum ether = 1:2, v/v) to give a final product (156.1 mg, yield: 70%); LC-MS: m/z = 357.2.

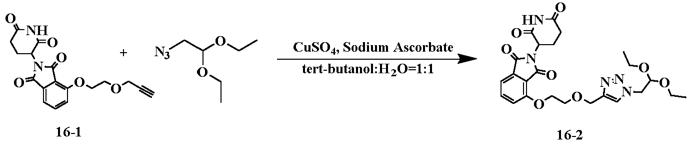

Copper sulfate (14.4 mg, 0.09 mmol, 0.2 equiv) was dissolved in 6 mL of water. 16-1 (156.1 mg, 0.44 mmol, 1 equiv) and 2-azido-1,1-diethoxyethane (70 mg, 0.44 mmol, 1 equiv) were dissolved in 6 mL of *tert*-butanol, respectively, and sequentially added to the copper sulfate solution. Sodium ascorbate (35.6 mg, 0.18 mmol, 0.4 equiv) was dissolved in 6 mL of water and added to the reaction system, and the reaction system was stirred overnight at room temperature. After the reaction was completed as monitored, the reaction system was diluted with saturated saline and extracted twice with chloroform. The organic phases were combined, sequentially extracted with a saturated sodium bicarbonate solution and saturated brine, and dried over anhydrous sodium sulfate. The redundant solvent was removed on a rotary evaporator to give a crude product. The resulting crude product was purified by silica gel column chromatography to give a final product (102.1 mg, yield: 45%); LC-MS: m/z = 516.2.

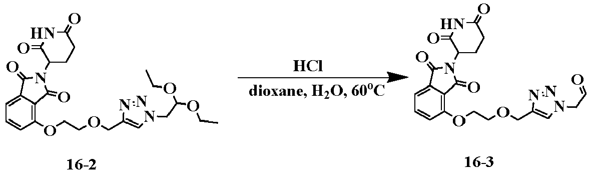

16-2 (100 mg, 0.19 mmol) was added to a 4 M dioxane hydrochloride solution, and the resulting mixture was added dropwise with a small amount of water and heated to 60°C. After the reaction was completed as monitored by TLC, the redundant solvent was removed by rotary evaporation. The reaction system was diluted with an appropriate amount of ethyl acetate, and extracted twice with saturated brine to give a final product (25.1 mg, yield: 30%); LC-MS: m/z = 442.2.

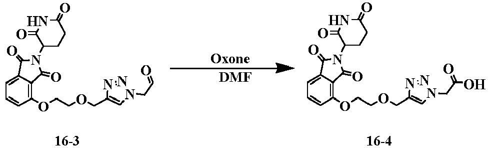

16-3 (25 mg, 0.06 mmol, 1.0 equiv) and Oxone (37 mg, 0.32 mmol, 1.0 equiv) were dissolved in an appropriate amount of DMF, and the resulting mixture was stirred at room temperature overnight. The reaction system was diluted with ethyl acetate and sequentially extracted with water and saturated brine for 3 times. The resulting organic phase was dried over anhydrous sodium sulfate, filtered, and concentrated by rotary evaporation to give a final product (23 mg, yield: 83%); LC-MS: m/z = 458.1.

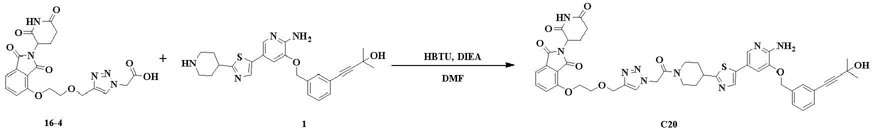

16-4 (22.9 mg, 0.05 mmol, 1.0 equiv) was dissolved in an appropriate amount of DMF, and the resulting mixture was stirred at 0°C, added with HBTU (34 mg, 0.09 mmol, 2.0 equiv), after 5 min, sequentially added with DIEA (17.2 mg, 0.13 mmol, 3.0 equiv) and compound 1 (20 mg, 0.045 mmol, 1.0 equiv), then heated to room temperature, and stirred overnight. The reaction system was diluted with ethyl acetate and extracted with water and saturated brine for 3 times. The resulting organic phase was dried over anhydrous sodium sulfate, filtered under vacuum with a sand-core funnel, and concentrated by rotary evaporation. The resulting crude product was purified by silica gel column chromatography (methanol:dichloromethane = 1:10, v/v) to give a final product (16.0 mg, yield: 40%); LC-MS: m/z = 888.3.

NMR: ¹H NMR (400 MHz, MeOD) δ 7.96 (d, *J* = 3.8 Hz, 1H), 7.78 (d, *J* = 1.8 Hz, 1H), 7.74 - 7.65 (m, 2H), 7.52 (s, 1H), 7.47 (d, *J* = 7.3 Hz, 1H), 7.41 - 7.38 (m, 3H), 7.29 (dd, *J* = 8.5, 1.7 Hz, 1H), 7.12 (d, *J* = 1.8 Hz, 1H), 5.34 (p, *J* = 16.3 Hz, 2H), 5.12 (s, 2H), 5.05 - 4.93 (m, 1H), 4.84 (q, *J* = 12.7 Hz, 2H), 4.57 (d, *J* = 13.4 Hz, 1H), 4.45 - 4.30 (m, 2H), 3.47 - 3.22 (m, 4H), 3.06 - 2.72 (m, 5H), 2.35 - 2.00 (m, 4H), 1.96 - 1.66 (m, 3H), 1.60 (s, 6H); ¹³C NMR (101 MHz, MeOD) δ 171.84, 171.64, 168.66, 166.66, 165.35, 163.22, 155.72, 149.85, 144.35, 140.81, 136.11, 135.86, 135.65, 135.26, 133.01, 131.03, 130.12, 128.13, 126.74, 124.69, 123.01, 118.87, 116.71, 116.50, 115.50, 114.49, 94.15, 80.41, 69.45, 68.50, 67.50, 64.30, 64.02, 50.29, 44.44, 41.62, 39.57, 39.46, 31.64, 31.18, 30.72, 30.50, 22.02.

### Example 21: Biological Experiment

S1: Jurkat cells were resuscitated, and then subjected to adherent culture for 12 h, compounds were added, the compound concentrations were fixed (treatment time was 0 h, 3 h, 6 h, 12 h, 24 h and 72 h) or the treatment time was fixed (compound concentrations were 0 nM, 1 nM, 10 nM, 100 nM and 1000 nM);
S2: the culture solution was poured out, the cells were washed with 3 mL of PBS pre-cooled at 4°C, then the washing solution was discarded, an appropriate amount of lysis solution containing PMSF (100×) was added according to the cell amount, the mixture was uniformly mixed, then placed on ice for lysis for 30 min, and centrifuged at 12000 rpm and at 4°C for 5 min, (a centrifuge was started for pre-cooling in advance), and the centrifuged supernatant was transferred into a new centrifuge tube and prepared for quantification;
S3: an BCA quantitative reagent of Pierce^{™} BCA Protein Assay Kit (23225) was added at 200 uL/well, then 20 uL of BSA with known different concentrations was added to prepare a protein quantitative standard curve and a sample protein to be detected with avoiding generation of bubbles, the mixture was incubated for 30 min in the dark at 37°C, an OD value was measured at 562 nm, then an appropriate amount of SDS-PAGE loading buffer was added to the protein sample, and the reaction mixture was heated for 5 min in a boiling water bath to fully denature the protein, and stored at -20°C for later use;
S4: an SDS-PAGE gel was prepared, wherein the voltage of a concentrated gel was 90 V, and the voltage of a separation gel was 120 V, a membrane was transferred for 1 h at 4°C and at a voltage of 100 V, after the membrane was transferred, the protein membrane was immediately placed in a prepared 5% BSA solution, and the mixture was slowly shaken on a shaker, and sealed for 1 h at room temperature;
S5: the primary antibody was diluted according to an appropriate proportion according to the specifications of HPK1 Antibody (4472S CST), GLK (D1L4G) Rabbit mAb (92427S CST), SLP-76 (D1R1A) Rabbit mAb (70896S CST), Phospho-SLP-76 (Ser376) (D7S1K) XP^{®} Rabbit mAb (92711T CST) and β-actin Mouse Monoclonal antibody (BE0021 easybio), then incubated overnight with slow shaking at 4°C, and washed 3 times with PBST for 10 min/time;
S6: Goat Anti-Rabbit IgG (H&L)-HRP Conjugated (BE0101 easybio) or Goat Anti-Mouse IgG (H&L)-HRP Conjugated (BE0102 easybio) was selected according to the primary antibody, and the secondary antibody was diluted according to an appropriate proportion according to the specification of the secondary antibody, then incubated for 1 h at room temperature with slow shaking, and washed 3 times with PBST for 10 min/time; and
S7: an ECL luminescence was added, the results were observed by a developing machine, Western results were quantified using Image J software, and the degradation rates (HPK1 and GLK) or inhibition rates (SLP76 Ser376 phosphorylation) were calculated by comparison with a control group to which no compounds were added, (degradation rate/inhibition rate = 1 - administration group/control group). The experimental results are shown in the tables below.

**TABLE 1: Degradation Rates of HPK1 with Fixed Treatment Time of 12 h and Varying Treatment Concentrations of Compounds**

| Compound | Degradation rate (10 nM) | Degradation rate (100 nM) |
|---|---|---|
| C1 | 47% | 68% |
| C2 | 39% | 33% |
| C3 | 15% | 75% |
| C7 | 72% | 68% |

**TABLE 2: Degradation Rates of HPK1 at Different Time Points with Treatment Concentration of Compound of 100 nM**

| Compound | Degradation rate (3 h) | Degradation rate (6 h) | Degradation rate (12 h) | Degradation rate (24 h) | Degradation rate (48 h) | Degradation rate (72 h) |
|---|---|---|---|---|---|---|
| C3 | 54% | 52% | 58% | 3% | 11% | |
| C7 | | | 58% | 86% | -40% | -9% |

**TABLE 3: Maximum Degradation Rate of HPK1 and Maximum Inhibition Rate of SLP-76 Phosphorylation at Different Time Points with Treatment Concentration of Compound of 100 nM**

| Compound | Maximum degradation rate of HPK1 | Maximum inhibition rate of SLP76 Ser376 phosphorylation |
|---|---|---|
| C3 | 62% | 81% |

**TABLE 4: Degradation Rates of HPK1 and GLK and Inhibition Rate of SLP-76 Phosphorylation with Treatment Time of 6 h and Treatment Concentration of Compound of 100 nM**

| Compound | HPK1 degradation rate | GLK degradation rate | SLP76 phosphorylation inhibition rate |
|---|---|---|---|
| C8 | 66% | 34% | 51% |
| C9 | 46% | -34% | 61% |
| C10 | 48% | -30% | |
| C11 | 30% | -36% | |
| C12 | 30% | 22% | |
| C13 | 38% | 43% | 4% |
| C14 | 58% | 60% | 26% |
| C15 | -27% | 38% | -7% |
| C16 | 49% | 74% | 24% |
| C17 | -7% | 42% | 22% |
| C18 | 39% | 71% | 34% |
| C19 | -95% | 34% | 3% |
| C20 | -15% (12h 41%) | 15% | 7% |

Finally, it should be noted that, the above examples are only used to illustrate the technical solutions of the present invention, and should not limit the same; although the present invention has been described in detail with reference to the examples described above, it will be understood by those skilled in the art that, the technical solutions in the examples described above can still be modified, or some or all of the technical features can be equivalently replaced; and these modifications or replacements do not make the technical solutions corresponding thereto depart from the scope of the technical solutions in the examples of the present invention.

## Claims

1. A compound of general formula I, or a pharmaceutically acceptable salt, a stereoisomer, an ester, a prodrug, a solvate and a deuterated compound thereof: 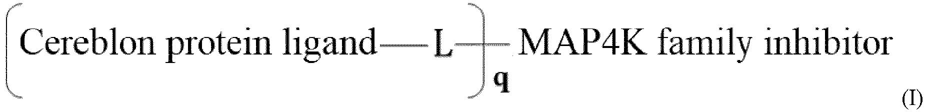 wherein the MAP4Ks family inhibitor is selected from: a HPK1 inhibitor, an MAP4K2 inhibitor, an MAP4K3 inhibitor, an MAP4K4 inhibitor, an MAP4K5 inhibitor, and an MAP4K6 inhibitor;
q is selected from integers between 1 and 5;
the Cereblon protein ligand is a small molecular ligand of Cereblon protein in an E3 ubiquitin ligase complex; L is a linking group between the Cereblon protein ligand and the MAP4K family inhibitor;
preferably, the L is -A₁-A₂......-Aₜ-,
wherein A₁, A₂......Aₜ are independently selected from:
CR^{L1}R^{L2}, O, S, S=O, S(=O)₂, S(O)₂O, OS(O)₂, OS(O)₂O, NR^{L3}, S(=O)₂NR^{L3}, S(=O)NR^{L3}, C(=O)NR^{L3}, OC(O)NR^{L3}, NR^{L3}C(=O), NR^{L3}C(=O)NR^{L4}, NR^{L3}S(=O)₂NR^{L4}, C(=O), CR^{L1}=CR^{L2}, C=C, C≡C, SiR^{L1}R^{L2}, P(=O)R^{L1}, P(=O)OR^{L1}, NR^{L3}C(=N-CN)NR^{L4}, NR^{L3}C(=N-CN), NR^{L3}C(=C-NO₂)NR^{L4}, C₃₋₁₁ cyclohydrocarbyl, C₃₋₁₁ heterocyclohydrocarbyl, succinimide, 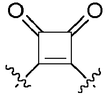 , -CH₂CH₂O-, -OCH₂CH₂-, 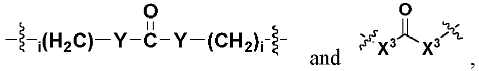 or are absent, wherein any hydrogen atom of the cyclohydrocarbyl and the heterocyclohydrocarbyl may be substituted with 1-6 R^{L1} or R^{L2};
when A₁, A₂......Aₜ are CR^{L1}R^{L2} or SiR^{L1}R^{L2}, R^{L1} and R^{L2} may be independently linked to other A to form cyclohydrocarbyl or heterocyclohydrocarbyl, and preferably, the cyclohydrocarbyl or heterocyclohydrocarbyl is optionally substituted with 1-11 R^{L5} groups;
R^{L1}, R^{L2}, R^{L3}, R^{L4} and R^{L5} are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cyclohydrocarbyl, C₃₋₁₁ heterocyclohydrocarbyl, O(C₁₋₈ cyclohydrocarbyl), S(C₁₋₈ cyclohydrocarbyl), NH(C₁₋₈ cyclohydrocarbyl), N(C₁₋₈ cyclohydrocarbyl)(C₁₋₈ alkyl), OH, NH₂, SH, SO₂(C₁₋₈ alkyl), P(=O)(OC₁₋₈ alkyl)(C₁₋₈ alkyl), P(=O)(OC₁₋₈ alkyl)₂, C₁₋₈ alkynyl, CH=CH(C₁₋₈ alkyl), C(C₁₋₈ alkyl)=CH(C₁₋₈ alkyl), C(C₁₋₈alkyl)=C(C₁₋₈alkyl)₂, Si(OH)₃, Si(C₁₋₈ alkyl)₃, Si(OH)(C₁₋₈ alkyl)₂, C(=O)(C₁₋₈ alkyl), CO₂H, CN, CF₃, CHF₂, CH₂F, NO₂, SF₅, SO₂NH(C₁₋₈ alkyl), SO₂N(C₁₋₈ alkyl)₂, S(=O)N(C₁₋₈ alkyl)₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl), NHSO₂N(C₁₋₈ alkyl)₂ and NHSO₂NH₂;
X³ is selected from O, S, -NR- and -CHR-;
R is selected from H and C₁₋₃ alkyl substituted with 1 or 2 -OH;
i is selected from integers between 1 and 6;
Y is selected from -O-, -S- and -NR-, or is absent; and
t is selected from integers between 1 and 100, and preferably, t is selected from integers between 1 and 50.

2. The compound, or the pharmaceutically acceptable salt, the stereoisomer, the ester, the prodrug, the solvate and the deuterated compound thereof according to claim 1, wherein the MAP4K family inhibitor is selected from a HPK1 inhibitor.

3. The compound, or the pharmaceutically acceptable salt, the stereoisomer, the ester, the prodrug, the solvate and the deuterated compound thereof according to claim 1, wherein the linking group L is 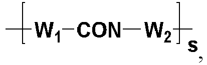 wherein s is selected from integers between 1 and 6;
CON is selected from 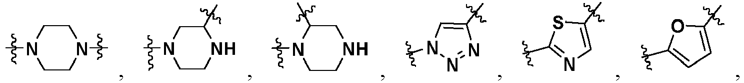 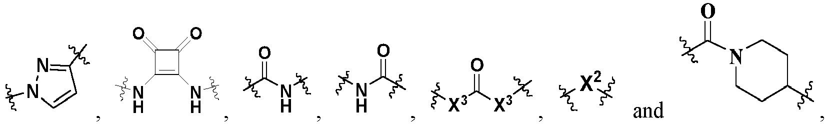 or is absent;
W₁ and W₂ are independently selected from 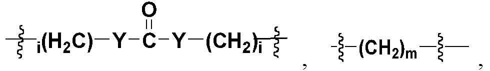 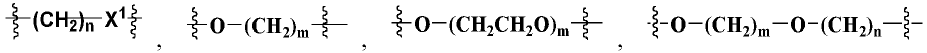 and 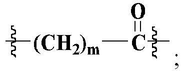 n is selected from integers between 1 and 10, and preferably from integers between 1 and 4;
m is selected from integers between 0 and 10, and preferably from integers between 2 and 7;
X¹ is selected from O, S and -NR-; and
X² is selected from O, S, -NR-, -S(O)-, -S(O)₂O-, -OS(O)₂- and -OS(O)₂O-.

4. The compound, or the pharmaceutically acceptable salt, the stereoisomer, the ester, the prodrug, the solvate and the deuterated compound thereof according to claim 1, wherein the linking group L is selected from 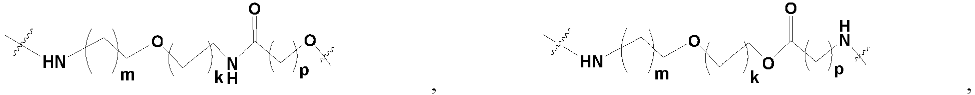 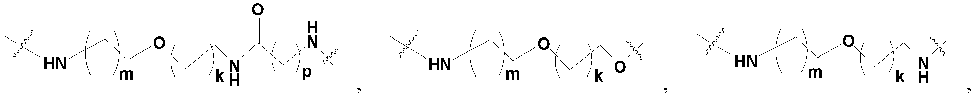 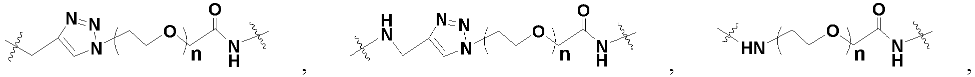 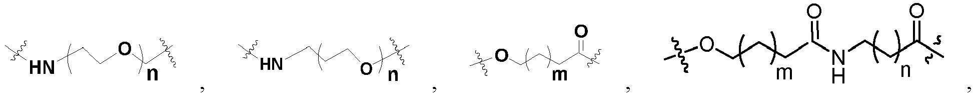 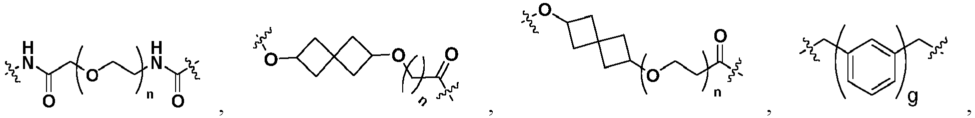 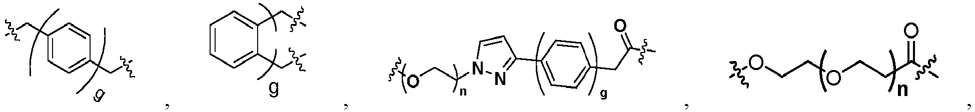 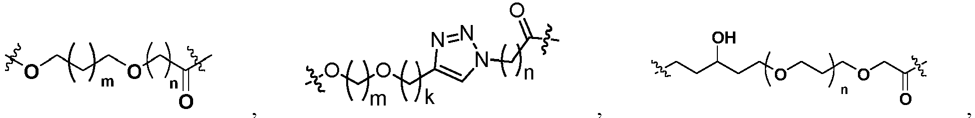 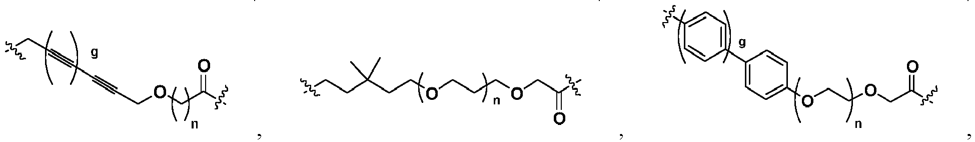 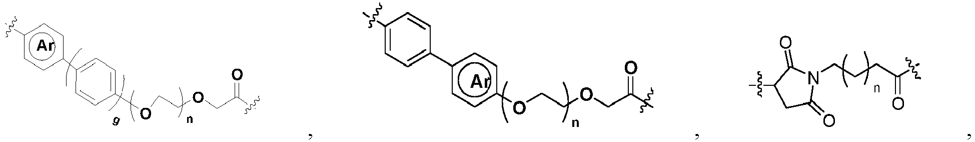 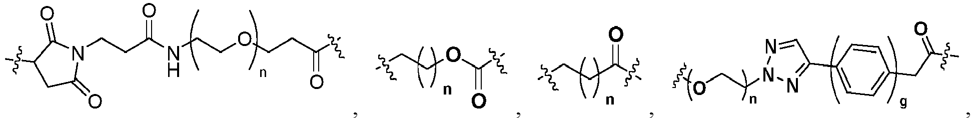 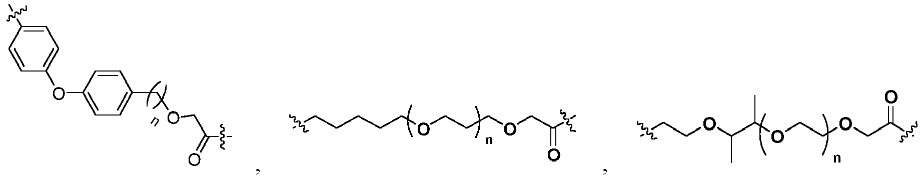 and 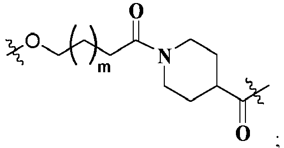 Ar is selected from N-containing six-membered aryl;
g is selected from 0 and 1;
k is selected from integers between 1 and 10, and preferably from integers between 1 and 4; and
p is selected from integers between 1 and 10, and preferably from integers between 1 and 2.

5. The compound, or the pharmaceutically acceptable salt, the stereoisomer, the ester, the prodrug, the solvate and the deuterated compound thereof according to claim 1, wherein the Cereblon protein ligand is selected from: an amide compound, a phthalimide compound, thalidomide and a derivative thereof, lenalidomide and a derivative thereof, and pomalidomide and a derivative thereof, and preferably, the Cereblon protein ligand moiety has a general formula as follows: 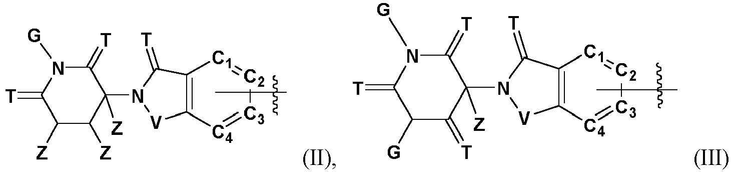 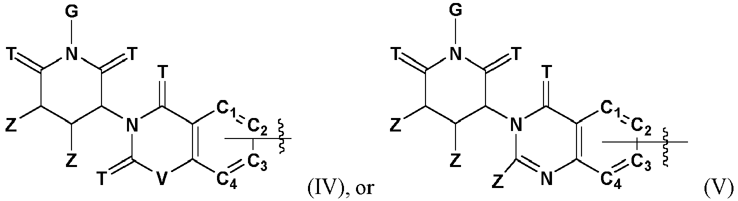 wherein C₁, C₂, C₃ and C₄ are independently selected from C and N;
T is selected from O and S;
V is selected from -O-, -S-, 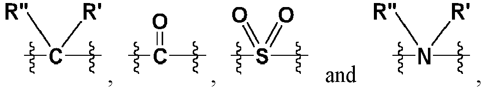 wherein R' and R" are independently selected from C₀₋₁₀ alkyl, cyclohydrocarbyl and heterocyclohydrocarbyl; G and Z are independently selected from H, -OH, C₁₋₁₀ linear/branched alkyl, C₃₋₁₀ cyclohydrocarbyl, O-containing heterocyclohydrocarbyl, N-containing heterocyclohydrocarbyl and S-containing heterocyclohydrocarbyl; and
q is selected from integers between 1 and 3, such as 1, 2 or 3.

6. The compound, or the pharmaceutically acceptable salt, the stereoisomer, the ester, the prodrug, the solvate and the deuterated compound thereof according to claim 5, wherein C₁, C₂, C₃ and C₄ are C; or, when C₁ is C, at least one of C₂, C₃ and C₄ is N; or, when C₂ is C, at least one of C₁, C₃ and C₄ is N; or, when C₃ is C, at least one of C₁, C₂ and C₄ is N; or, when C₄ is C, at least one of C₁, C₂ and C₃ is N.

7. The compound, or the pharmaceutically acceptable salt, the stereoisomer, the ester, the prodrug, the solvate and the deuterated compound thereof according to claim 6, wherein C₁, C₂, C₃ and C₄ are C;
T is O;
V is selected from -CH₂-, 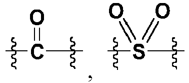 and -NH-; and
G and Z are independently selected from H, -CH₃ and -CH₂CH₃.

8. The compound, or the pharmaceutically acceptable salt, the stereoisomer, the ester, the prodrug, the solvate and the deuterated compound thereof according to claim 7, wherein the Cereblon protein ligand moiety has a structural formula as follows:

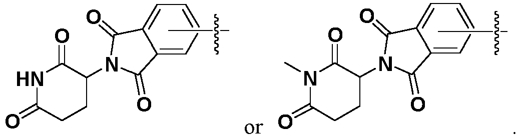

9. The compound, or the pharmaceutically acceptable salt, the stereoisomer, the ester, the prodrug, the solvate and the deuterated compound thereof according to claim 1, wherein the linking group L has a structure as follows:

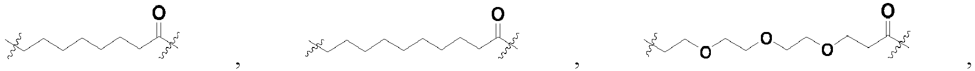

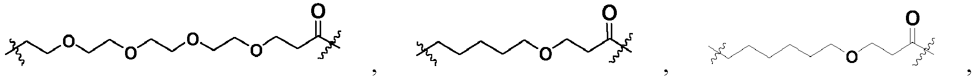

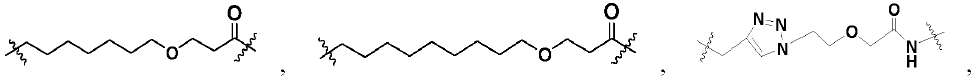

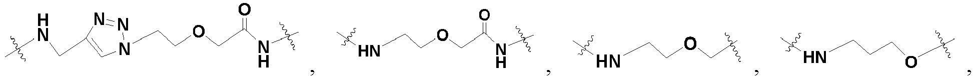

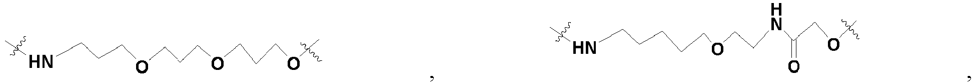

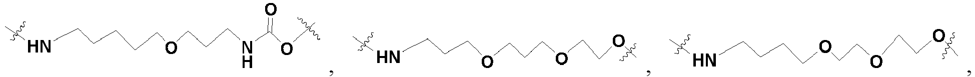

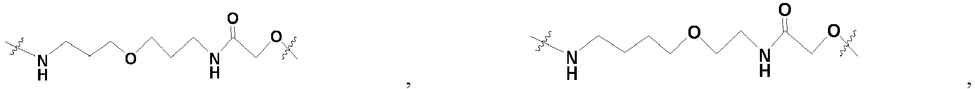

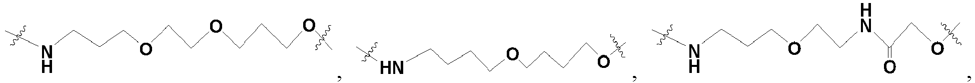

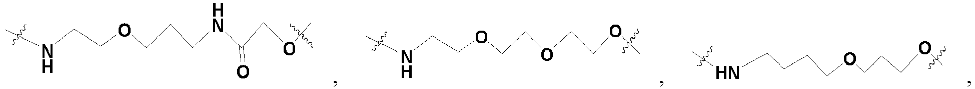

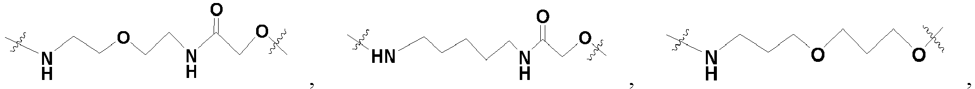

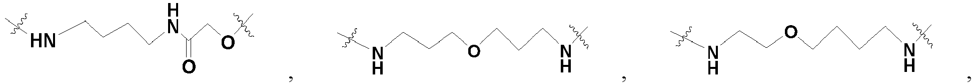

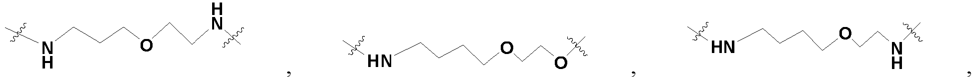

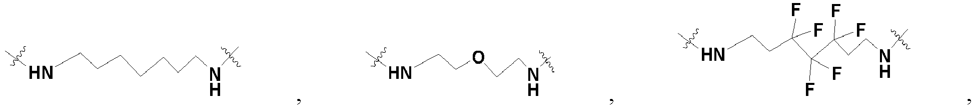

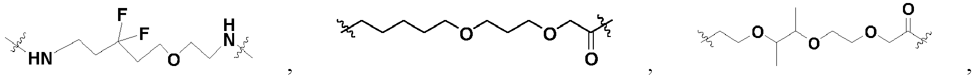

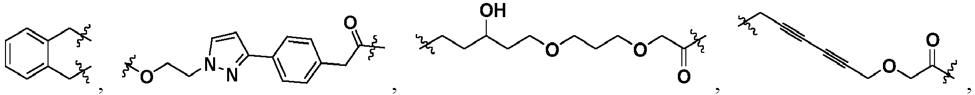

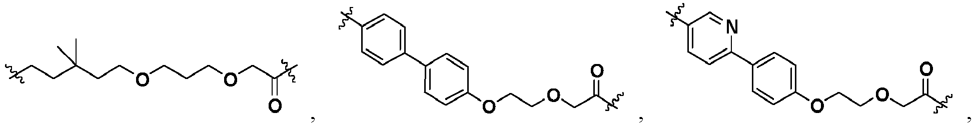

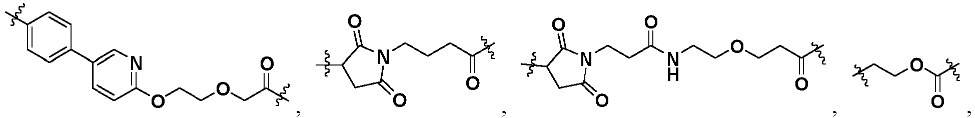

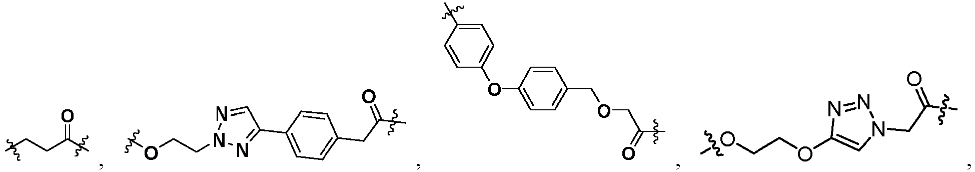

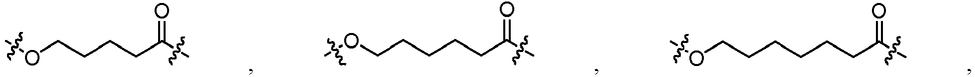

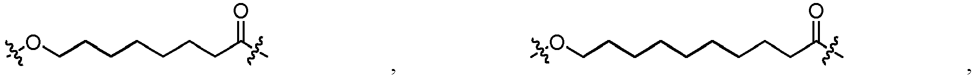

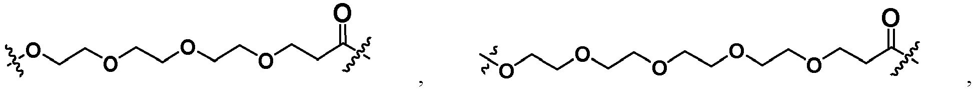

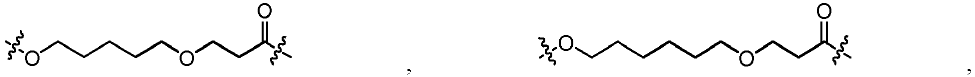

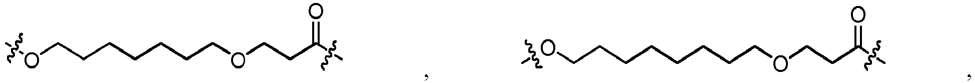

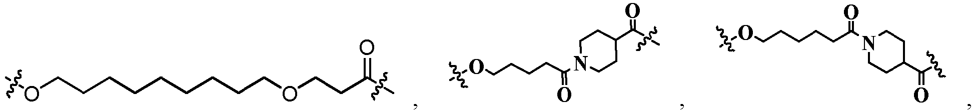

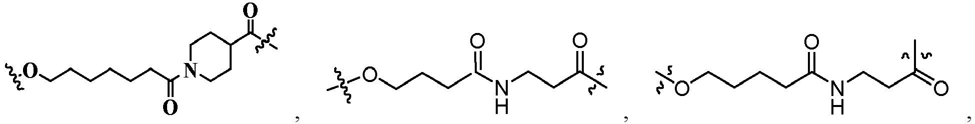

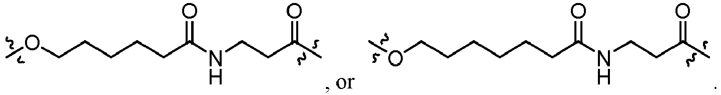

10. The compound, or the pharmaceutically acceptable salt, the stereoisomer, the ester, the prodrug, the solvate and the deuterated compound thereof according to claim 9, wherein the L is 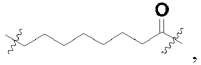 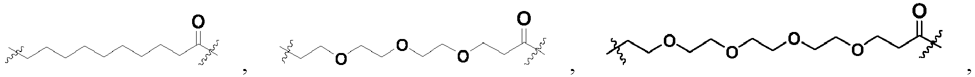 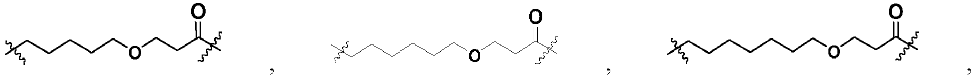 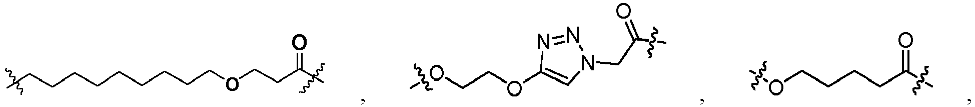 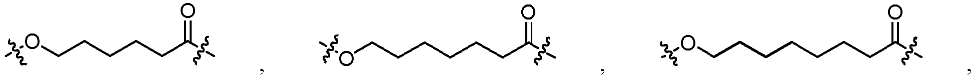 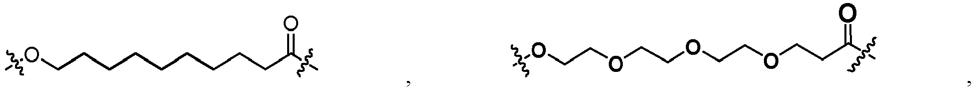 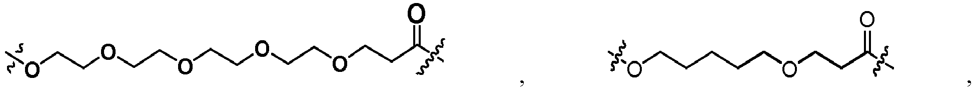 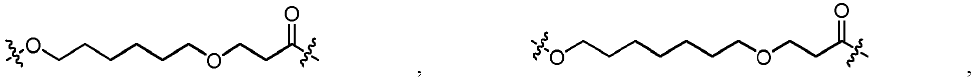 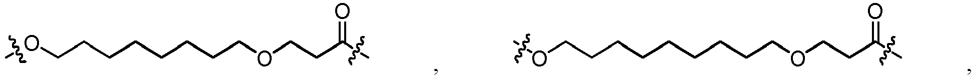 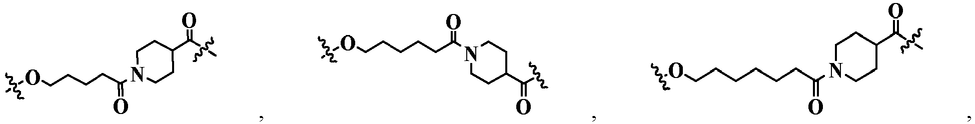 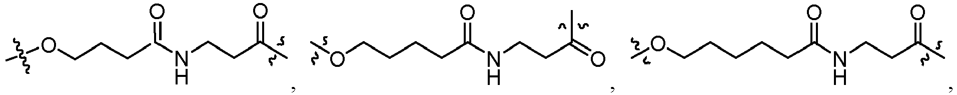 or

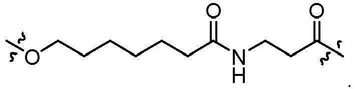

11. The compound, or the pharmaceutically acceptable salt, the stereoisomer, the ester, the prodrug, the solvate and the deuterated compound thereof according to claim 1, wherein the HPK1 inhibitor moiety has a general formula as follows: 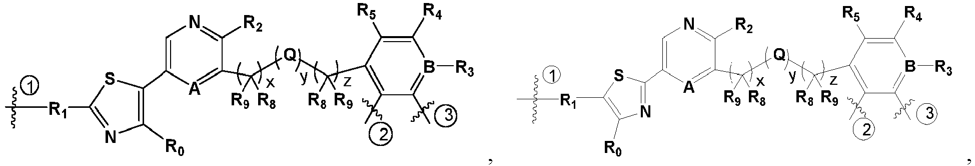 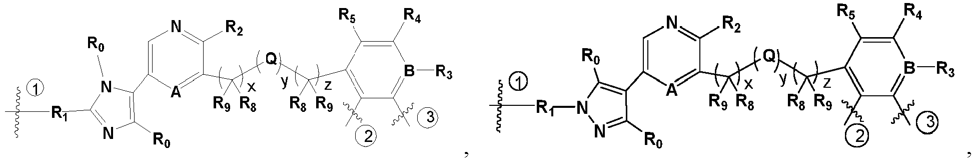 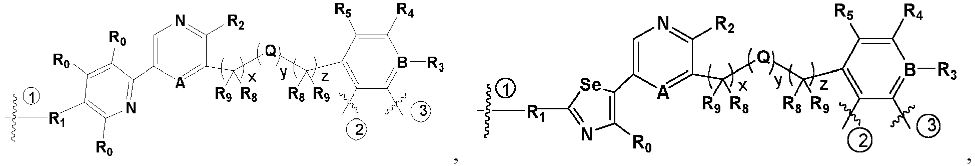 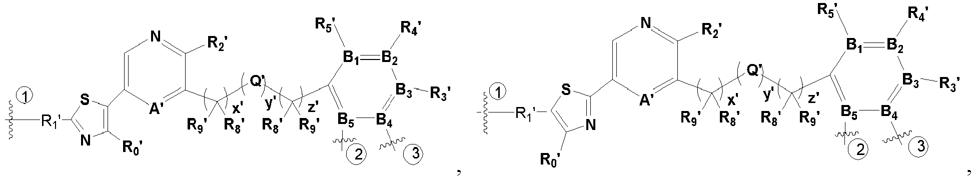 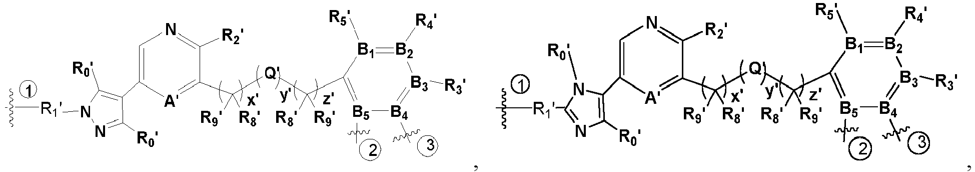 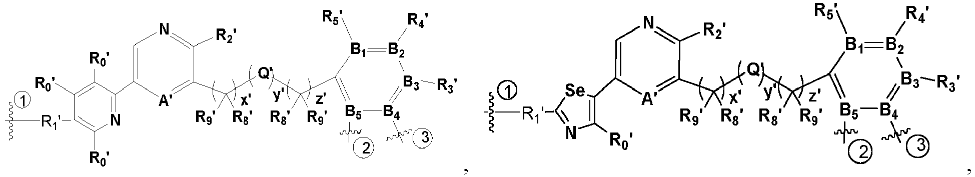 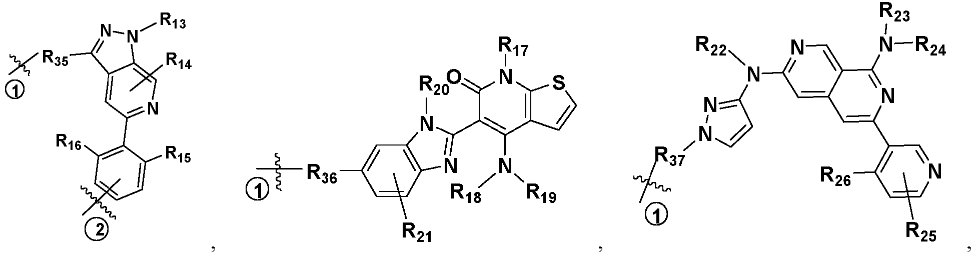 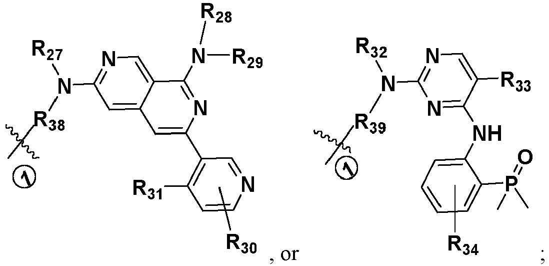 the general formula of HPK1 has 3, 2 or 1 attachment site(s) of Cereblon protein ligands, and may be optionally attached to 3, 2 or 1 Cereblon protein ligand(s), with the rest attachment sites being replaced with -R‴; preferably, a position ①, a position ② and a position ③ in the HPK1 inhibitor are all attachment sites of the Cereblon protein ligands, or the position ① and the position ② are attachment sites of the Cereblon protein ligands while the position ③ is attached to R‴, or the position ② and the position ③ are attachment sites of the Cereblon protein ligands while the position ① is attached to R‴, or the position ① and the position ③ are attachment sites of the Cereblon protein ligands while the position ② is attached to R‴, or the position ① is an attachment site of the Cereblon protein ligand while the position ② and the position ③ are attached to R‴, or the position ② is an attachment site of the Cereblon protein ligand while the position ① and the position ③ are attached to R‴, or the position ③ is an attachment site of the Cereblon protein ligand while the position ① and the position ② are attached to R‴, or the position ① and the position ② are attachment sites of the Cereblon protein ligands, or the position ① is an attachment site of the Cereblon protein ligand while the position ② is attached to R‴, or the position ② is an attachment site of the Cereblon protein ligand while the position ① is attached to R‴;
wherein R'" is selected from -H, halogen, -NO₂, -CN, C₁₋₅ linear/branched alkyl, C₃₋₁₀ cyclohydrocarbyl, -N(C₀₋₁₀ alkyl)(C₀₋₁₀ alkyl), -CF₃, -OCF₃, -OCHF₂, -OCH₂F and -OC₀₋₁₀ alkyl;
A is selected from C and N; B is selected from C and N;
Q is selected from O and S; x and z are independently selected from integers between 0 and 6; y is selected from 0 and 1;
R₀ is independently selected from: -H, C₁₋₁₀ linear/branched alkyl, -N(C₀₋₁₀ alkyl)(C₀₋₁₀ alkyl), -OC₀₋₁₀ alkyl and C₃₋₁₀ cyclohydrocarbyl;
R₁ is selected from -O heterocycloalkyl, -N heterocycloalkyl, C₁₋₁₀ linear/branched alkyl, C₃₋₁₀ cycloalkyl, -OC₀₋₁₀ alkyl, -N(Co-io alkyl)(Co-io alkyl), -O₂(C₀₋₁₀ alkyl), -O(C₀₋₁₀ alkyl), -O-phenyl, -S(C0-10 alkyl), -N heterocycloaryl, -O heterocycloaryl and -S heterocycloaryl, wherein H on a C atom or heteroatom may be substituted with C1-3 linear alkyl, -N(C₀₋₁₀ alkyl)(C₀₋₁₀ alkyl) and -CF₃;
R₂ is selected from: -H, halogen, -NO₂, -CN, C₁₋₅ linear/branched alkyl, C₃₋₁₀ cyclohydrocarbyl, -N(C₀₋₁₀ alkyl)(C₀₋₁₀ alkyl), -CF₃, -OCF₃, -OCHF₂, -OCH₂F and -OC₀₋₁₀ alkyl;
when B is N, R₃ is absent; when B is C, R₃ is selected from: -H, halogen, -OC₀₋₁₀ alkyl, C₁₋₁₀ linear/branched alkyl, -N(C₀₋₁₀ alkyl)(C₀₋₁₀ alkyl) and C₃₋₁₀ cyclohydrocarbyl;
R₄ is selected from: -H, halogen, -OC₀₋₁₀ alkyl, -CN, C₃₋₁₀ cyclohydrocarbyl, -C=C-R₁₀. C₁₋₁₀ linear/branched alkyl, -N(C₀₋₁₀ alkyl)(C₀₋₁₀ alkyl), -O heterocyclohydrocarbyl and -N heterocyclohydrocarbyl;
R₁₀ is selected from: H, C₁₋₅ linear/branched alkyl, C₃₋₁₀ cyclohydrocarbyl and 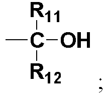
R₁₁ and R₁₂ are independently selected from: -H, -CF₃, -CHF₂H, -CH₂F, C₁₋₁₀ linear/branched alkyl, -CH=C(C₀₋₁₀ alkyl)(C₀₋₁₀ alkyl), -C≡C(C₀₋₁₀ alkyl), C₃₋₁₀ cyclohydrocarbyl, an aromatic five-membered cyclic group and an aromatic six-membered cyclic group, or R₁₁ and R₁₂, together with carbon atoms between R₁₁ and R₁₂, form C₃₋₈ cyclohydrocarbyl or C₃₋₈ heterocyclohydrocarbyl containing -O or -S, C₉ fused cyclohydrocarbyl, C₃₋₇ cyclolactam, C₃₋₇ cyclic lactone, or C₃₋₇ cyclic ketone, wherein H on a C atom may be substituted with alkyl or halogen;
R₅ is selected from: -H, halogen, -CN, -OC₀₋₁₀ alkyl, C₁₋₁₀ linear/branched alkyl, heteroalkyl containing O and N, -N(Co-io alkyl)(C₀₋₁₀ alkyl), C₃₋₁₀ cyclohydrocarbyl, -C≡C-R₁₀, -O heterocyclohydrocarbyl and -N heterocyclohydrocarbyl, wherein H attached to a C atom may be substituted with the following groups: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, C₁₋₁₀ linear/branched alkyl, -N(C₀₋₁₀ alkyl)(C₀₋₁₀ alkyl), -OC₀₋₁₀ alkyl, C₃₋₁₀ cyclohydrocarbyl, -O heterocycloalkyl, -N heterocycloalkyl, -N heterocycloaryl, -O heterocycloaryl and -S heterocycloaryl;
F₈ and R₉ are independently selected from: -H, halogen and C₁₋₁₀ linear/branched alkyl;
A' is selected from C and N; B₁, B₂, B₃, B₄ or B₅ is independently selected from C and N;
Q' is selected from O and S; x' and z' are independently selected from integers between 0 and 6; y' is selected from 0 and 1;
R₀' is independently selected from: -H, C₁₋₁₀ linear/branched alkyl, -N(C₀₋₁₀ alkyl)(C₀₋₁₀ alkyl), -OC₀₋₁₀ alkyl and C₃₋₁₀ cyclohydrocarbyl;
R₁' is selected from: -O heterocycloalkyl, -N heterocycloalkyl, C₁₋₁₀ linear/branched alkyl, C₃₋₁₀ cyclohydrocarbyl, -OC₀₋₁₀ alkyl, -N(C₀₋₁₀ alkyl)(C₀₋₁₀ alkyl), -SO₂(C₀₋₁₀ alkyl), -O(C₀₋₁₀ alkyl), -O-phenyl, -S(Co-io alkyl), -N heterocycloaryl, -O heterocycloaryl and -S heterocycloaryl, wherein H attached to a C atom or heteroatom may be substituted with C₁₋₃ linear alkyl, -N(C₀₋₁₀ alkyl)(C₀₋₁₀ alkyl) or -CF₃;
R₂' is selected from: -H, halogen, -NO₂, -CN, C₁₋₅ linear/branched alkyl, C₃₋₁₀ cyclohydrocarbyl, -N(C₀₋₁₀ alkyl)(C₀₋₁₀ alkyl), -CF₃, -OCF₃, -OCHF₂, -OCH₂F and -OC₀₋₁₀ alkyl;
when B₁, B₂, B₃, B₄ or B₅ is N, the corresponding R₃', R₄' and R₅' is absent; when B₁, B₂, B₃, B₄ or B₅ is C, R₃', R₄' and R₅' are independently selected from: -H, halogen, -CN, -OC₀₋₁₀ alkyl, C₁₋₁₀ linear/branched alkyl, heteroalkyl containing O and N, -N(C₀₋₁₀ alkyl)(C₀₋₁₀ alkyl), C₃₋₁₀ cyclohydrocarbyl, -C≡C-R₁₀'. -O heterocyclohydrocarbyl and -N heterocyclohydrocarbyl, or R₅' and R₄', or R₄' and R₃', together with carbon atoms therebetween, form C₃₋₈ cyclohydrocarbyl or C₃₋₈ heterocycloalkyl containing -O- and -S-, -N heterocycloaryl, -O heterocycloaryl or -S heterocycloaryl, or phenyl, wherein H attached to a C atom may be substituted with the following groups: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, C₁₋₁₀ linear/branched alkyl, -N(C₀₋₁₀ alkyl)(C₀₋₁₀ alkyl), -OC₀₋₁₀ alkyl, C₃₋₁₀ cyclohydrocarbyl, -O heterocycloalkyl, -N heterocycloalkyl, -N heterocycloaryl, -O heterocycloaryl and -S heterocycloaryl;
R₅' and R₉' are independently selected from: -H, halogen and C₁₋₁₀ linear/branched alkyl; R₃₅, R₃₆, R₃₈ and R₃₉ are selected from C₁₋₁₀ linear/branched alkyl, -CON(C₀₋₁₀ alkyl)-, - CO(C₀₋₁₀ alkyl)-, C₃₋₁₀ cyclohydrocarbyl, -O heterocycloalkyl, -N heterocycloalkyl, phenyl, -N heterocycloaryl and -O heterocycloaryl, and R₃₇, together with an N atom attached thereto and a C atom adjacent to the N atom, forms 5-8 membered cyclolactam; and R₁₃-R₃₄ are independently selected from: -H, halogen, -CN, -OC₀₋₁₀ alkyl, C₁₋₁₀ linear/branched alkyl, -N(C₀₋₁₀ alkyl)(C₀₋₁₀ alkyl), C₃₋₁₀ cyclohydrocarbyl, -O heterocycloalkyl, -N heterocycloalkyl, -S heterocycloalkyl, phenyl, -N heterocycloaryl, -O heterocycloaryl and -S heterocycloaryl.

12. The compound, or the pharmaceutically acceptable salt, the stereoisomer, the ester, the prodrug, the solvate and the deuterated compound thereof according to claim 11, wherein x and z are independently selected from integers between 0 and 2, such as 0, 1 or 2;
the HPK1 inhibitor moiety has a structural formula as follows:

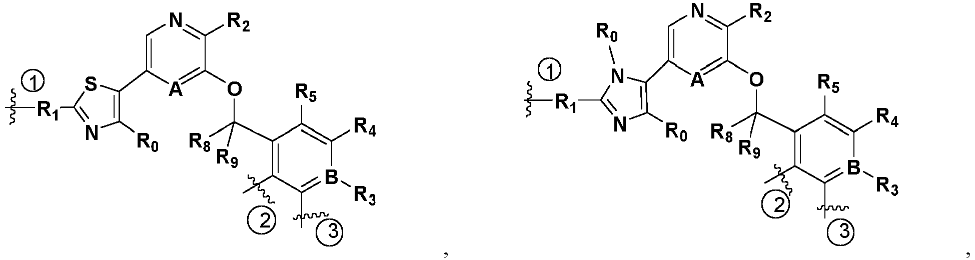

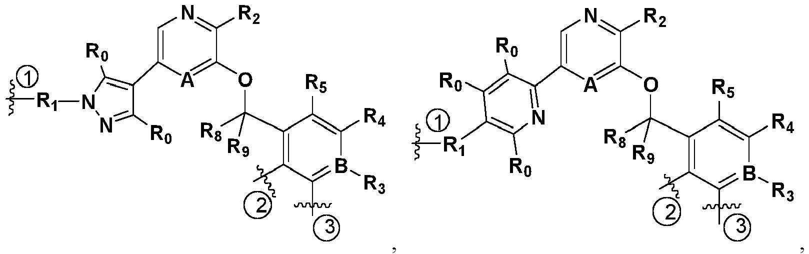

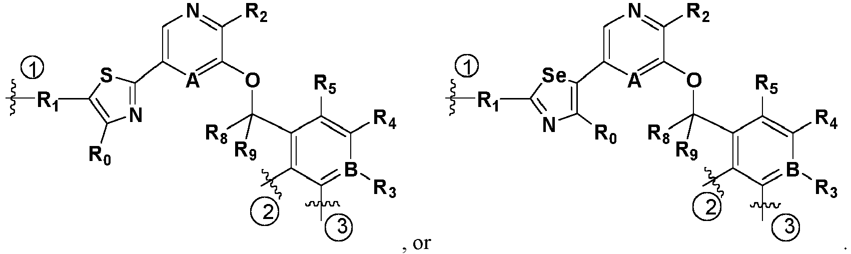

13. The compound, or the pharmaceutically acceptable salt, the stereoisomer, the ester, the prodrug, the solvate and the deuterated compound thereof according to claim 12, wherein R₀ is independently selected from: C₁₋₅ linear/branched alkyl and -N(C₀₋₁₀ alkyl)(C₀₋₁₀ alkyl);
R₁ is selected from: -O heterocycloalkyl and -N heterocycloalkyl, -SO₂(C₀₋₃ alkyl), -O-phenyl, -S(C₀₋₄ alkyl), C₃₋₆ cycloalkyl and C₃₋₅ linear/branched alkyl, wherein H attached to a C atom or heteroatom may be substituted with -CH₃, -NH₂ or -CF₃;
R₂ is selected from: -NO₂, -N(C₀₋₁₀ alkyl)(C₀₋₁₀ alkyl), -OC₀₋₁₀ alkyl and -OCF₃;
R₃ is selected from: -H, halogen, -OC₀₋₁₀ alkyl and C₁₋₁₀ linear/branched alkyl;
R₄ is selected from: -H, halogen, -OC₀₋₁₀ alkyl, -CN, C₃₋₁₀ cycloalkyl and -C≡C-R₁₀;
R₁₁ and R₁₂ are independently selected from: -H, -CF₃, -CHF₂H, -CH₂F, C₁₋₁₀ linear/branched alkyl, -CH=C(C₀₋₁₀ alkyl)(C₀₋₁₀ alkyl), C₃₋₁₀ cycloalkyl and an aromatic six-membered cyclic group, or R₁₁ and R₁₂, together with carbon atoms between R₁₁ and R₁₂, form C₃₋₈ cycloalkyl, C₄₋₇ fused cycloalkyl, C₃₋₇ cyclolactam, C₃₋₇ cyclic lactone, or C₃₋₇ cyclic ketone, wherein H attached to a C atom may be substituted with alkyl or -F;
R₅ is selected from: -H, halogen, C₃₋₆ cycloalkyl, -OC₀₋₅ alkyl, C₁₋₅ linear/branched alkyl and C₁₋₅ linear/branched alkyl containing O and N, wherein H attached to a C atom may be substituted with -F; and
F₈ and R₉ are independently selected from: -H and C₁₋₁₀ linear/branched alkyl.

14. The compound, or the pharmaceutically acceptable salt, the stereoisomer, the ester, the prodrug, the solvate and the deuterated compound thereof according to claim 13, wherein R₀ is independently selected from: -CH₃, -CH₂CH₃ and -NH₂;
R₁ is selected from: 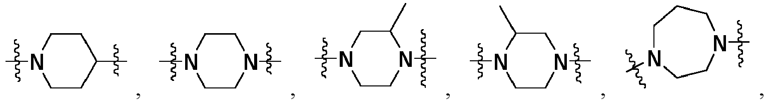 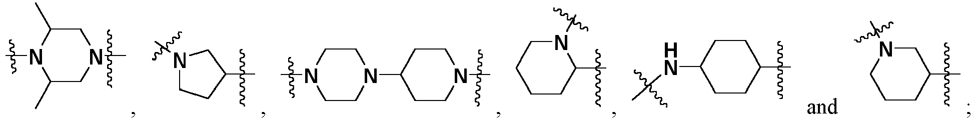 R₂ is selected from: -NH₂ and -NO₂;
R₃ is selected from: -H, -F and -OCH₃;
R₄ is selected from: -H, -F, -Cl, -OCH₃, -CN, 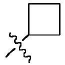 and -C≡C-R₁₀;
R₁₁ and R₁₂ are independently selected from: -H, -CF₃, -CHF₂, -CH₂F, -CH₃, -CH₂CH₃, -CH=CH₂, 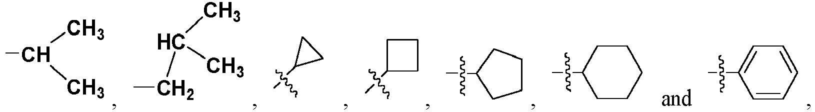 or R₁₁ and R₁₂, together with carbon atoms between R₁₁ and R₁₂, form 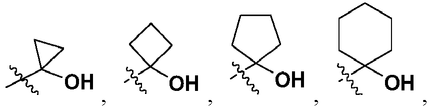 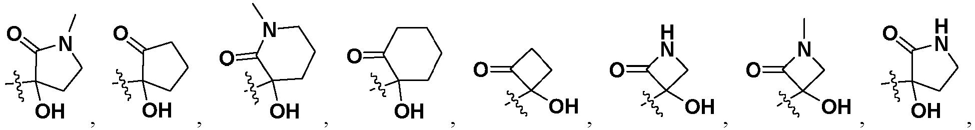 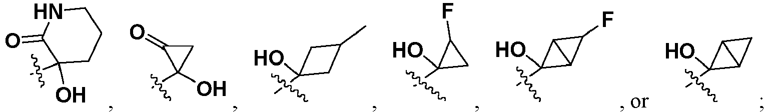 F₈ is selected from: -H, halogen, -OC₀₋₃ alkyl, C₁₋₃ linear/branched alkyl and C₁₋₃ linear/branched alkyl containing N, wherein H attached to a C atom may be substituted with -F, and preferably, R₅ is selected from:
-H, -F, -Cl, -CH₃, -CH₂NH₂, -CN and -OCH₃; and
R₈ and R₉ are independently selected from: -H and -CH₃.

15. The compound, or the pharmaceutically acceptable salt, the stereoisomer, the ester, the prodrug, the solvate and the deuterated compound thereof according to claim 11, wherein B₁, B₂, B₃, B₄ or B₅ is C, or, at least one of B₁, B₂, B₃, B₄ and B₅ is N;
preferably, B₂ is C, at least one of B₁, B₃, B₄ and B₅ is N, or B₂ is C, and B₁ is N; B₂ is C, and B₃ is N; B₂ is C, and B₄ is N; B₂ is C, and B₅ is N, or B₂ is C, B₃ and B₄ are N, or B₃ and B₅ are N;
x' and z' are independently selected from integers between 0 and 2, such as 0, 1 or 2;
the HPK1 inhibitor moiety has a structural formula as follows: 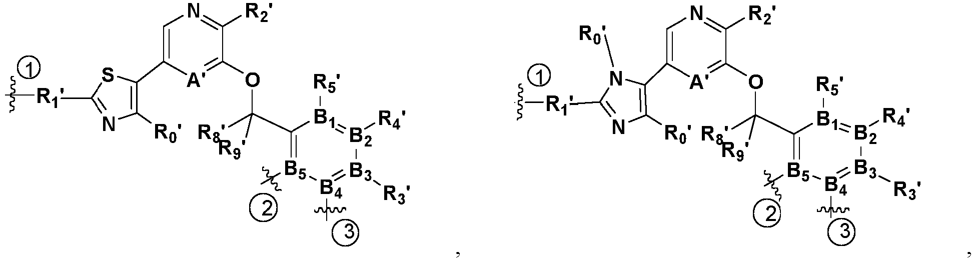 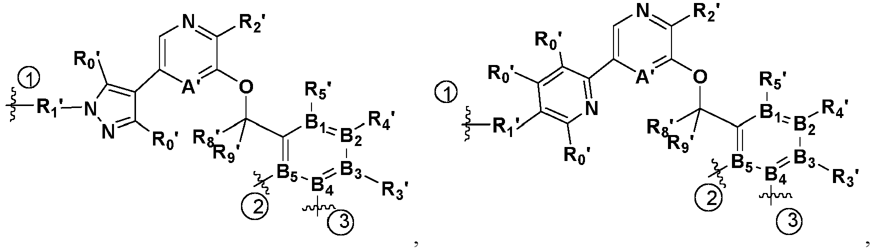 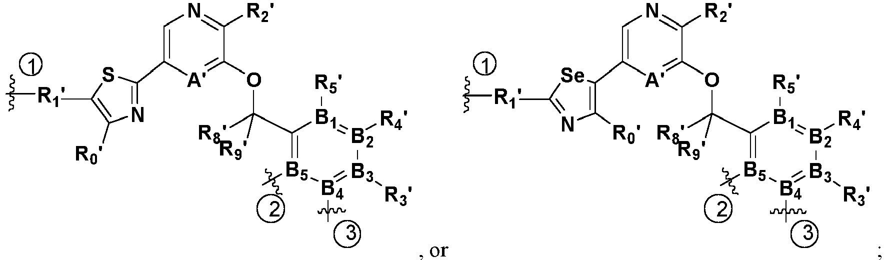
R₀' is independently selected from: C₁₋₅ linear/branched alkyl and -N(C₀₋₁₀ alkyl)(C₀₋₁₀ alkyl);
R₁' is selected from: -O heterocycloalkyl and -N heterocycloalkyl, -SO₂(C₀₋₃ alkyl), -O-phenyl, -S(C₀₋₄ alkyl), C₃₋₆ cycloalkyl and C₃₋₅ linear/branched alkyl, wherein H attached to a C atom or heteroatom may be substituted with -CH₃, -NH₂ or -CF₃;
R₂' is selected from: -NO₂, -N(C₀₋₁₀ alkyl)(C₀₋₁₀ alkyl), -OC₀₋₁₀ alkyl and -OCF₃;
R₃' is selected from: -H, halogen, -OC₀₋₁₀ alkyl, C₁₋₁₀ linear/branched alkyl, -N(C₀₋₁₀ alkyl)(C₀₋₁₀ alkyl) and C₃₋₁₀ cycloalkyl;
R₄' is selected from: -H, halogen, -OC₀₋₁₀ alkyl, -CN, C₃₋₁₀ cycloalkyl, -C≡C-R₁₀'. C₁₋₁₀ linear/branched alkyl, -N(C₀₋₁₀ alkyl)(C₀₋₁₀ alkyl), -O heterocycloalkyl and -N heterocycloalkyl;
R₅' is independently selected from: -H, halogen, -CN, -OC₀₋₁₀ alkyl, C₁₋₁₀ linear/branched alkyl, -N(C₀₋₁₀ alkyl)(C₀₋₁₀ alkyl), C₃₋₁₀ cycloalkyl, -C≡C-R₁₀', -O heterocycloalkyl and -N heterocycloalkyl, and C₁₋₅ linear/branched alkyl containing O and N, wherein H attached to a C atom may be substituted with -F;
R₁₀' is selected from: H, C₁₋₅ linear/branched alkyl, C₃₋₁₀ cycloalkyl and 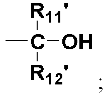
R₁₁' and R₁₂' are independently selected from: -H, -CF₃, -CHF₂H, -CH₂F, C₁₋₁₀ linear/branched alkyl, -CH=C(C₀₋₁₀ alkyl)(Co-io alkyl), -C≡C(C₀₋₁₀ alkyl), C₃₋₁₀ cycloalkyl, an aromatic five-membered cyclic group and an aromatic six-membered cyclic group, or R₁₁' and R₁₂', together with carbon atoms between R₁₁' and R₁₂', form C₃₋₈ cycloalkyl or C₃₋₈ heterocycloalkyl containing -O and -S, C₄₋₉ fused cycloalkyl, C₅₋₁₀ spiro cycloalkyl, C₄₋₉ bridged cycloalkyl, C₃₋₇ cyclolactam, C₃₋₇ cyclic lactone, or C₃₋₇ cyclic ketone, wherein H attached to a C atom may be substituted with the following groups: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, C₁₋₁₀ linear/branched alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -N heterocycloaryl, -O heterocycloaryl and -S heterocycloaryl, wherein the alkyl moieties may be optionally substituted with one or more of the following groups: -SO₂, -SO₂N(C₀₋₁₀ alkyl)(Co-io alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, -N(C₀₋₁₀ alkyl)(C₀₋₁₀ alkyl), -OC₀₋₁₀ alkyl, -N heterocycloaryl, -O heterocycloaryl and -S heterocycloaryl; and
R₅' and R₉' are independently selected from: -H and C₁₋₁₀ linear/branched alkyl.

16. The compound, or the pharmaceutically acceptable salt, the stereoisomer, the ester, the prodrug, the solvate and the deuterated compound thereof according to claim 15, wherein R₀' is independently selected from: -CH₃, -CH₂CH₃ and -NH₂;
R₁' is selected from: 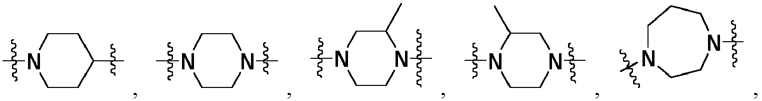 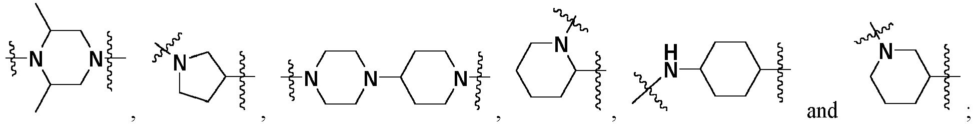 R₂' is selected from: -NH₂ and -NO₂;
R₃' is selected from: -H, halogen, -OC₀₋₁₀ alkyl and C₁₋₁₀ linear/branched alkyl;
R₄' is selected from: -H, -F, -Cl, -OCH₃, -CN, 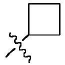
and -C≡C-R₁₀';
R₅' is selected from: -H, -F, -Cl, -CH₃, -CH₂NH₂, -CN and -OCH₃;
R₁₁' and R₁₂' are independently selected from: -H, -CF₃, -CHF₂, -CH₂F, -CH₃, -CH₂CH₃, -CH=CH₂, 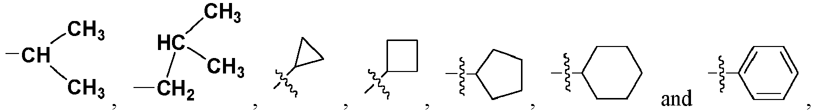 or R₁₁' and R₁₂', together with carbon atoms between R₁₁' and R₁₂', form 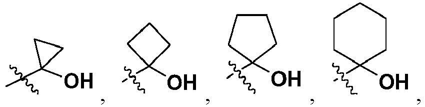 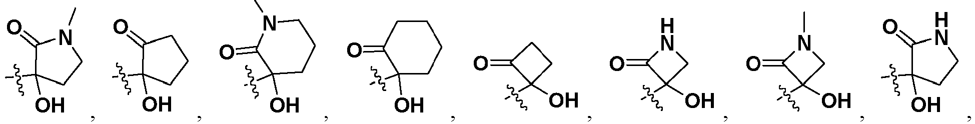 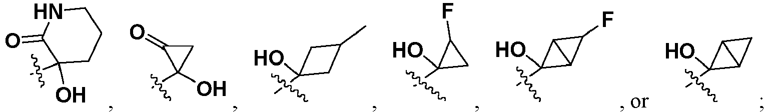 and
R₈' and R₉' are independently selected from: -H and -CH₃.

17. The compound, or the pharmaceutically acceptable salt, the stereoisomer, the ester, the prodrug, the solvate and the deuterated compound thereof according to claim 16, wherein R₁₃-R₃₄ are independently selected from: -H, halogen, -CN, C₁₋₄ linear/branched alkyl, -N(C₀₋₁₀ alkyl)(C₀₋₁₀ alkyl) and C₃₋₅ cycloalkyl, and preferably, R₁₃-R₃₄ are independently selected from: -H, -F, -Cl, -CN, -CH₃, -CH₂CH₃, -NH₂, 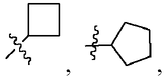 and

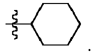

18. The compound, or the pharmaceutically acceptable salt, the stereoisomer, the ester, the prodrug, the solvate and the deuterated compound thereof according to claim 1, wherein the HPK1 inhibitor moiety has a structure selected from:

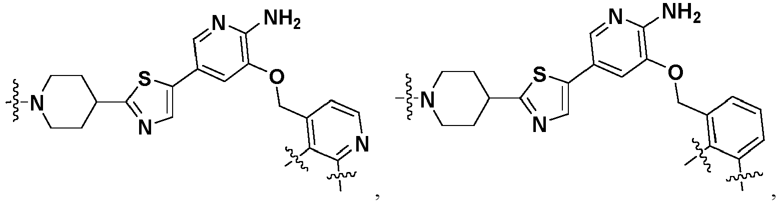

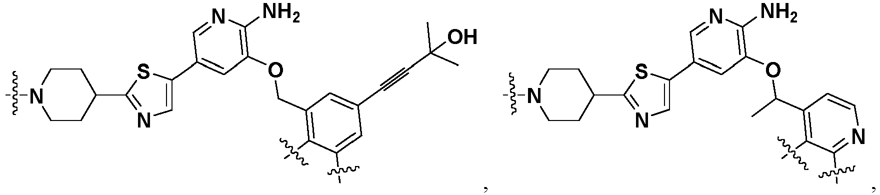

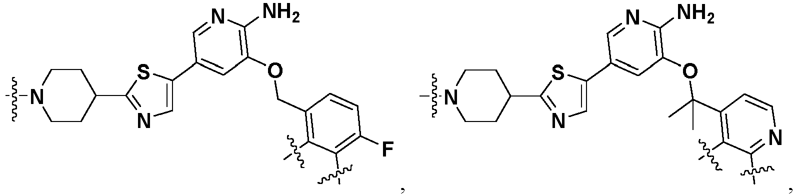

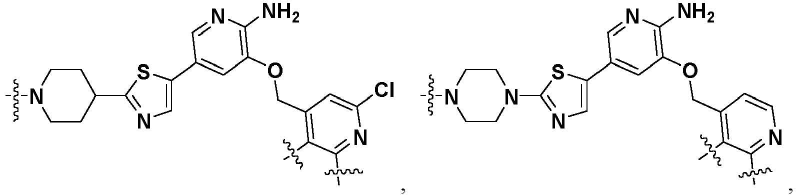

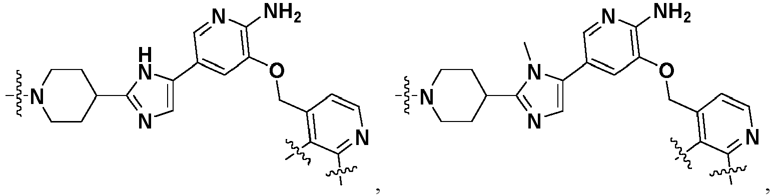

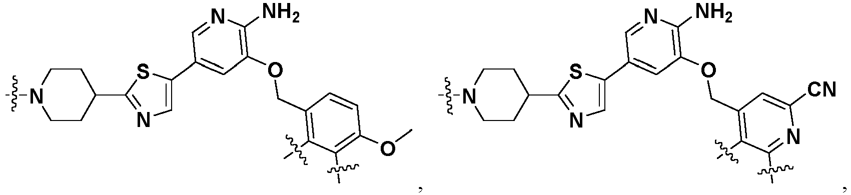

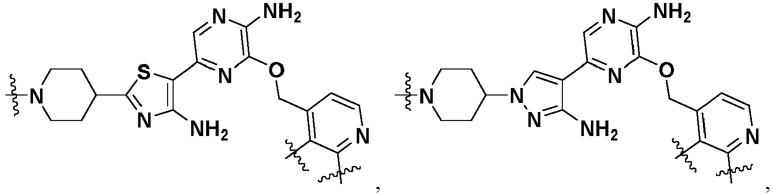

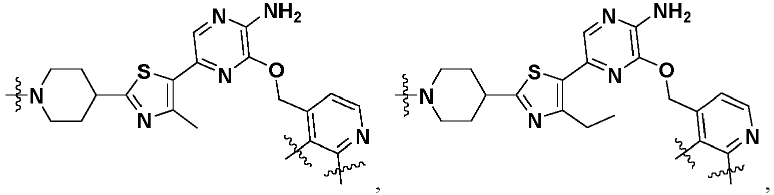

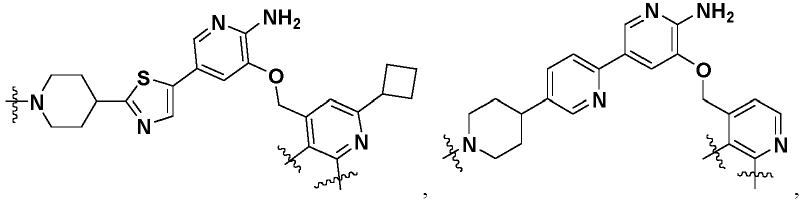

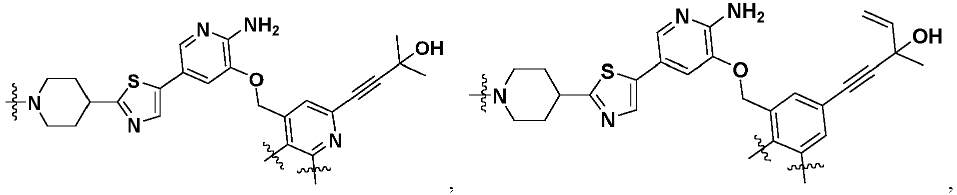

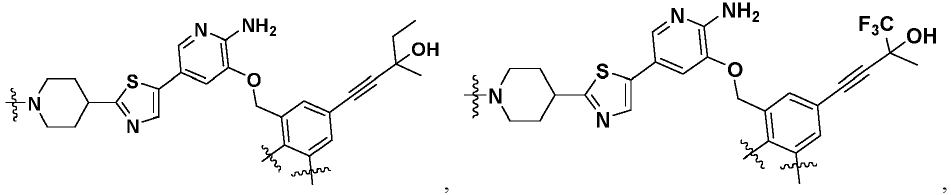

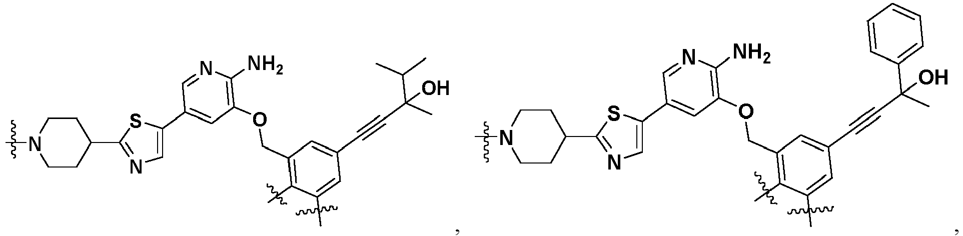

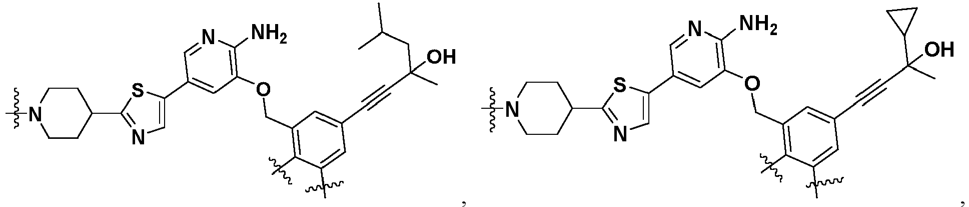

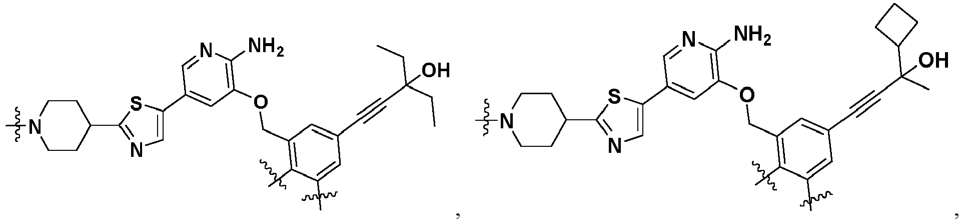

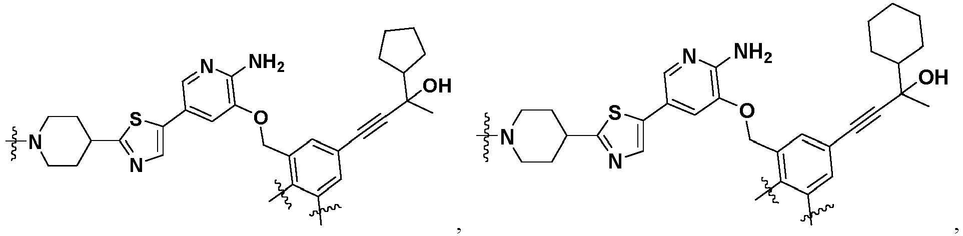

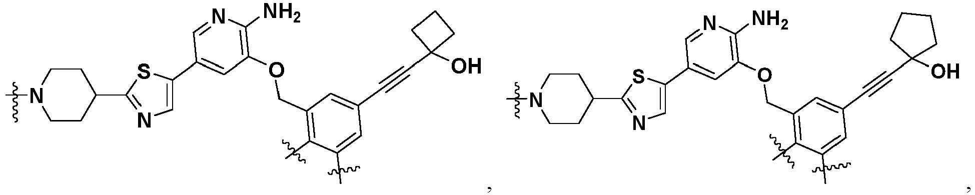

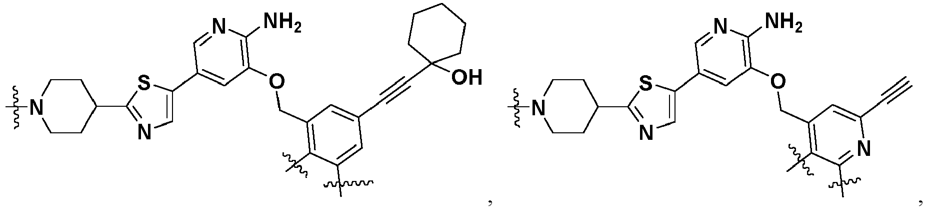

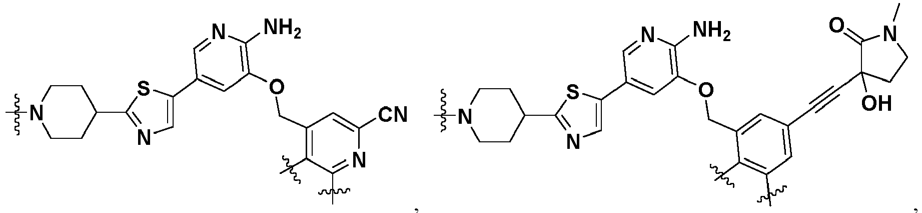

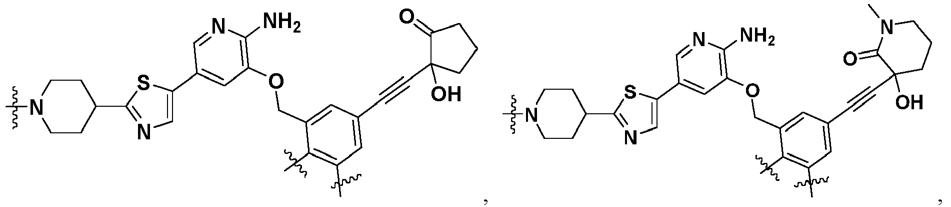

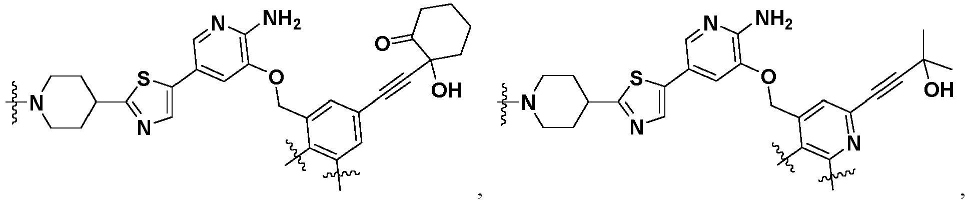

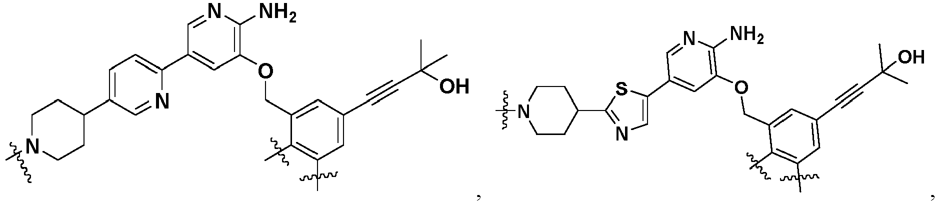

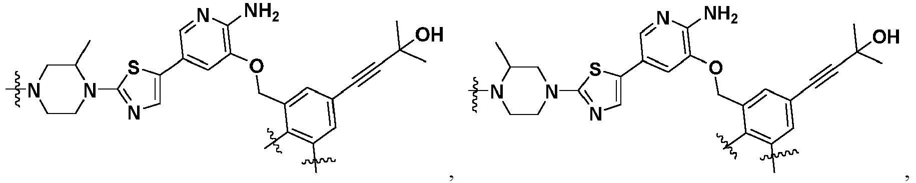

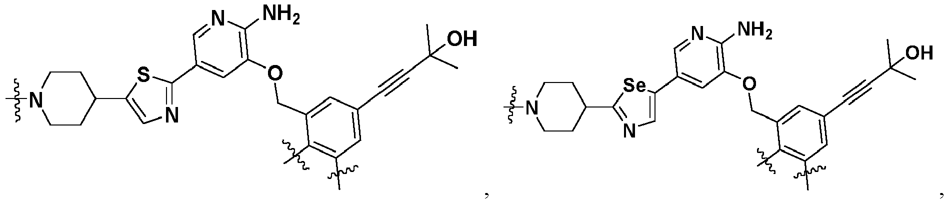

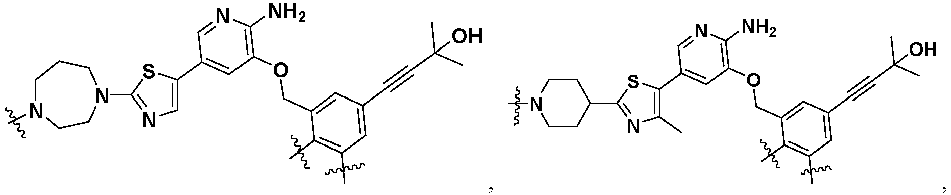

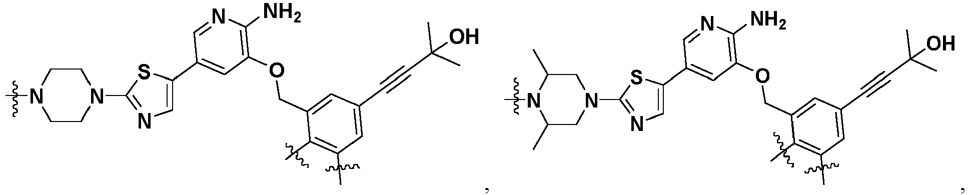

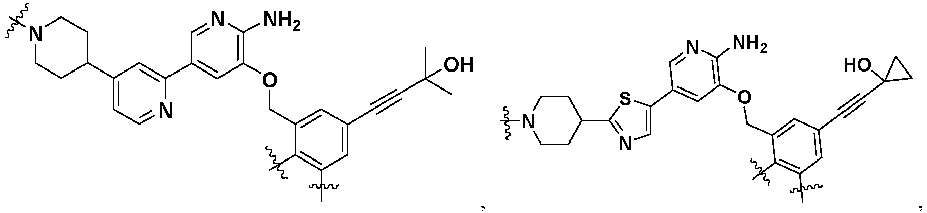

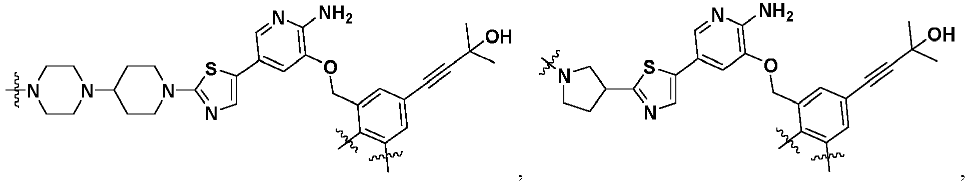

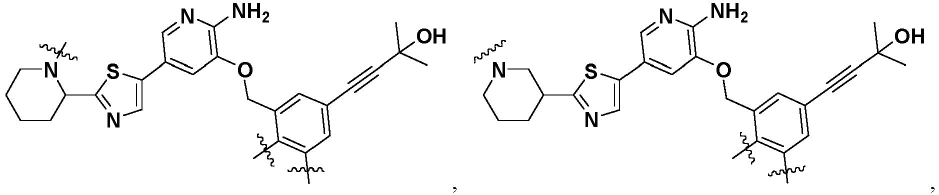

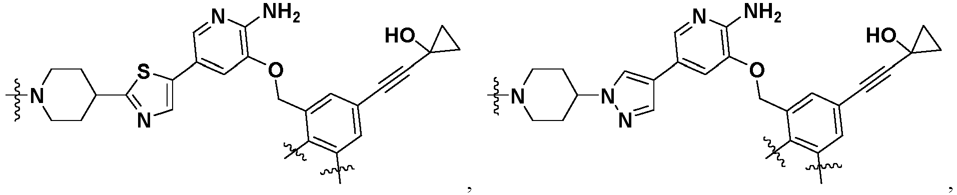

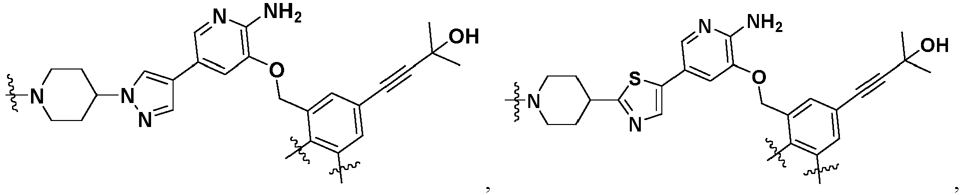

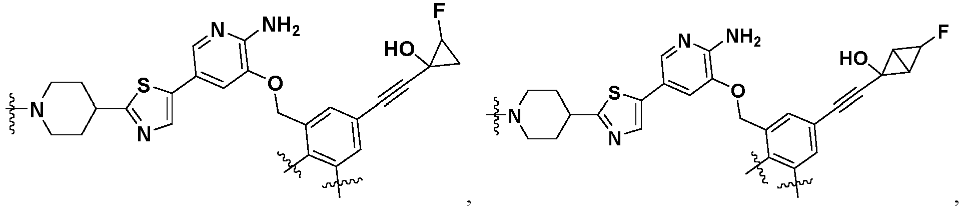

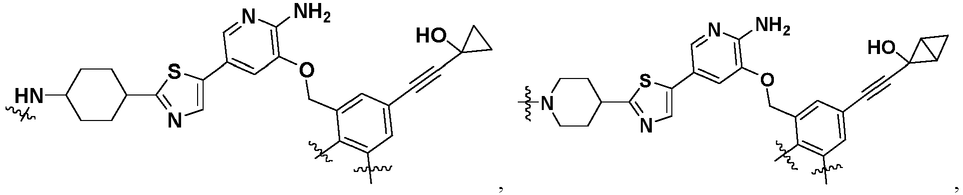

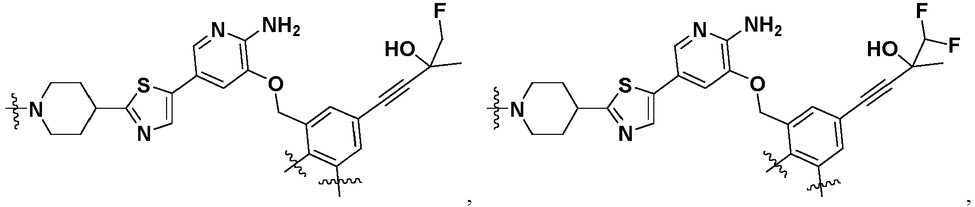

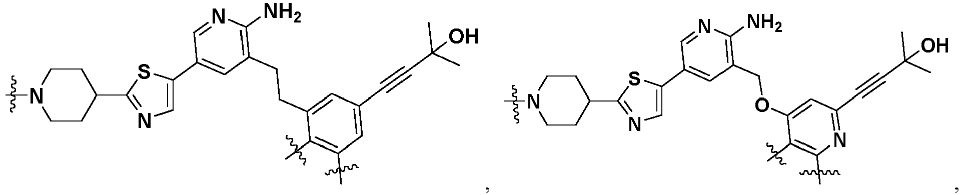

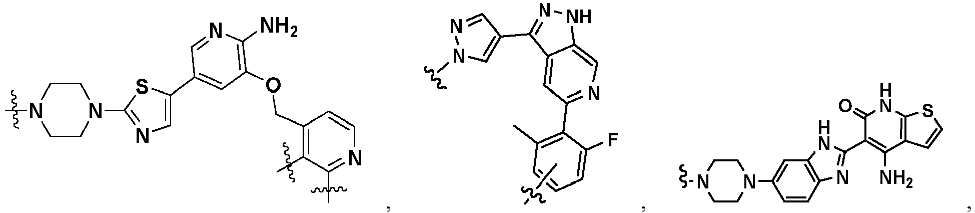

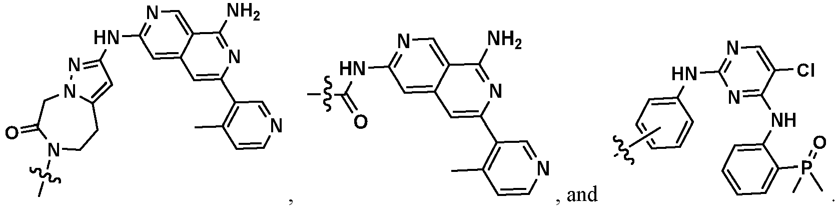

19. The compound, or the pharmaceutically acceptable salt, the stereoisomer, the ester, the prodrug, the solvate and the deuterated compound thereof according to claim 1, wherein the compound has a structural formula selected from: 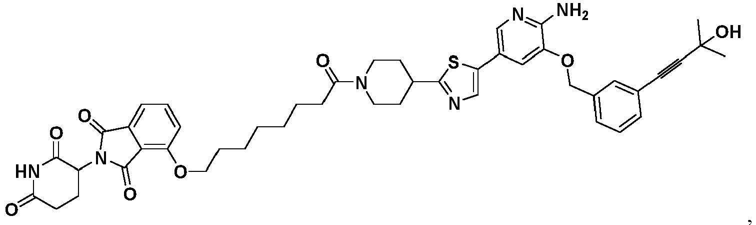 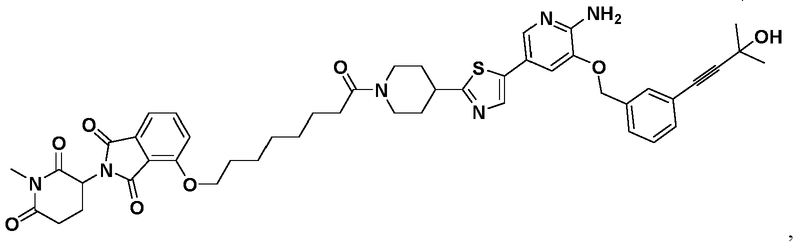 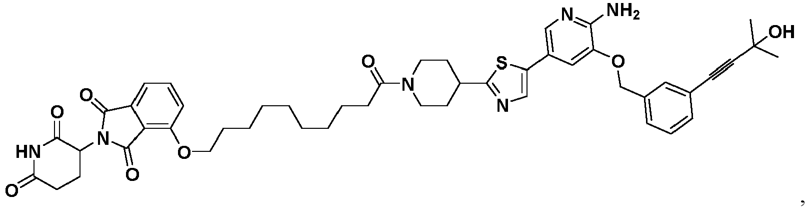 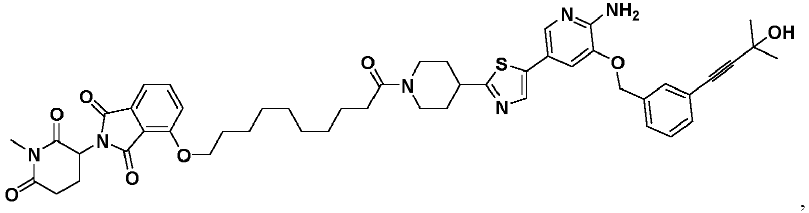 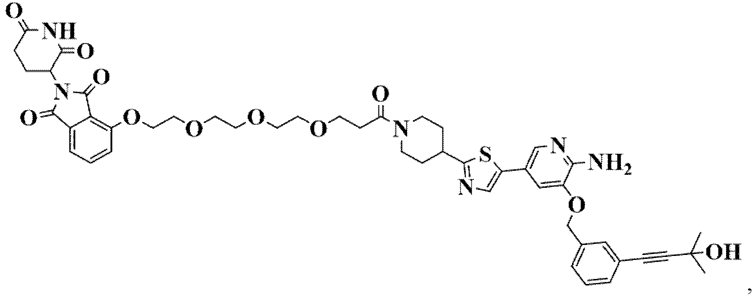 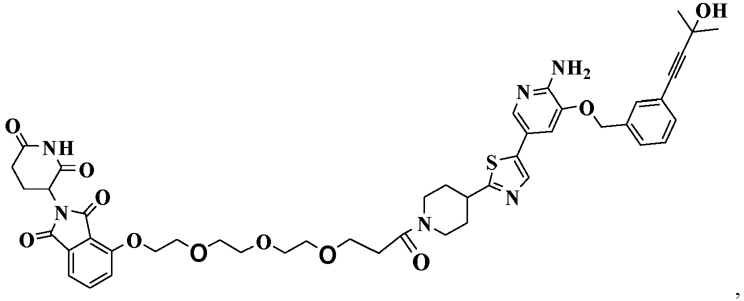 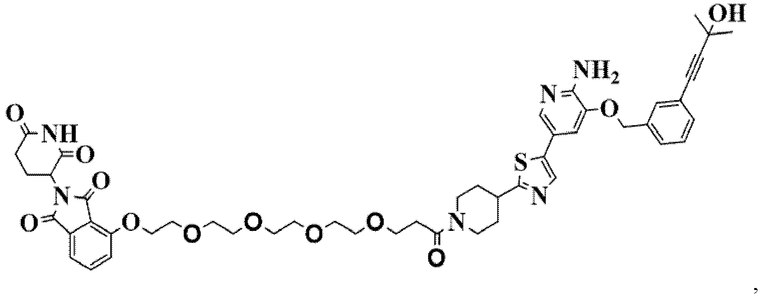 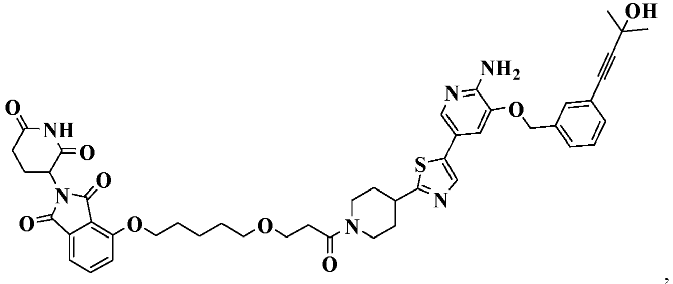 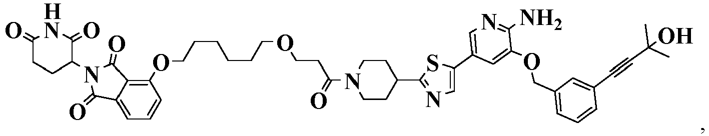 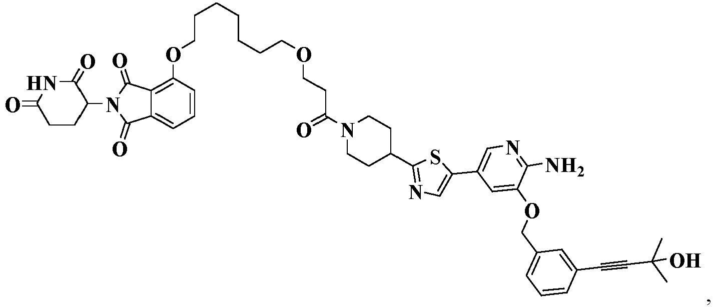 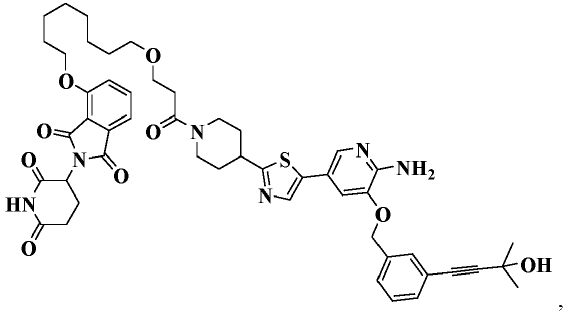 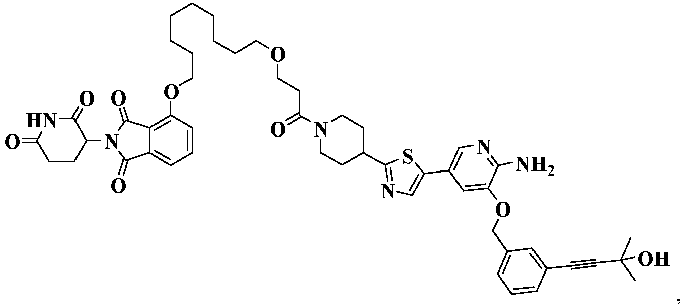 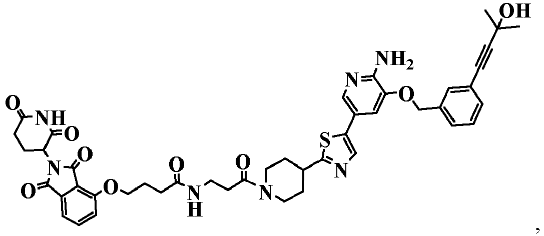 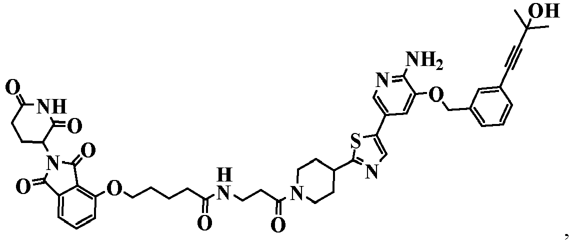 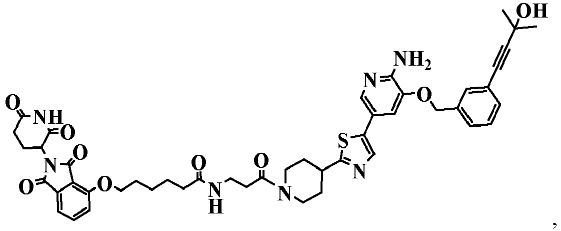 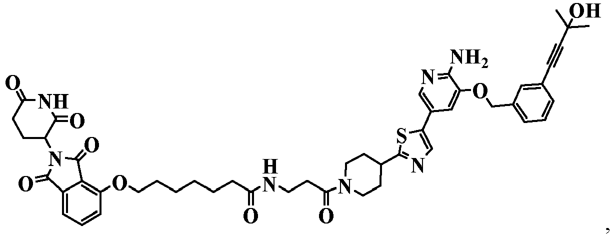 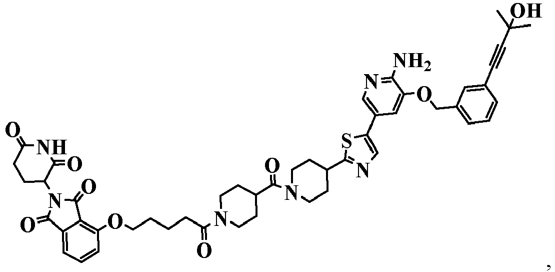 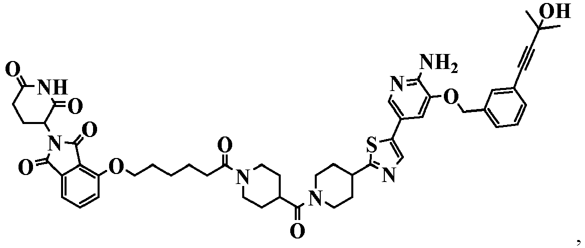 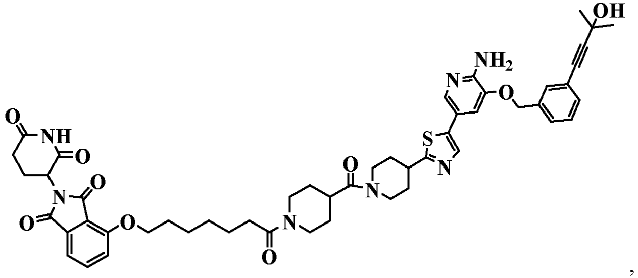 and

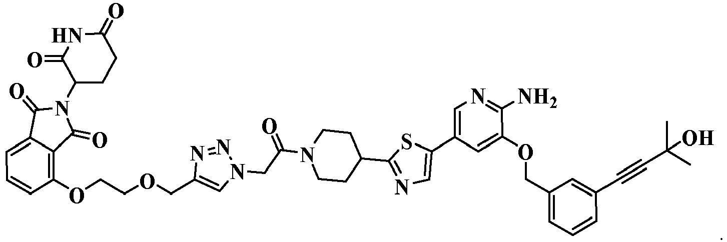

20. A pharmaceutical composition, comprising the compound of general formula I, or the pharmaceutically acceptable salt, the stereoisomer, the ester, the prodrug, the solvate and the deuterated compound thereof according to claim 1, and further comprising a pharmaceutically acceptable excipient.

21. Use of the compound of general formula I, and the pharmaceutically acceptable salt, the stereoisomer, the ester, the prodrug, the solvate and the deuterated compound thereof according to claim 1 for preparing a medicament for inhibiting the serine/threonine kinase family, and preferably for preparing a medicament for inhibiting hematopoietic progenitor kinase 1.

22. Use of the compound of general formula I, and the pharmaceutically acceptable salt, the stereoisomer, the ester, the prodrug, the solvate and the deuterated compound thereof according to claim 1 for preparing a medicament for treating a disease associated with hematopoietic progenitor kinase 1, wherein the disease associated with hematopoietic progenitor kinase 1 is selected from: inflammation, immune disease and cancer; the immune disease is selected from: lupus erythematosus, glomerulonephritis, rheumatoid arthritis, psoriasis, inflammatory bowel disease and autoimmune diabetes;
the cancer is selected from: lymphoma, blastoma, medulloblastoma, retinoblastoma, sarcoma, liposarcoma, synovial cell sarcoma, neuroendocrine tumor, carcinoid tumor, gastrinoma, islet cell carcinoma, mesothelioma, schwannoma, acoustic neuroma, meningioma, adenocarcinoma, melanoma, leukemia and lymphoid malignancy, squamous cell carcinoma, epithelial squamous cell carcinoma, lung carcinoma, small cell lung carcinoma, non-small cell lung carcinoma, adenocarcinoma lung carcinoma, squamous lung carcinoma, peritoneal carcinoma, hepatocellular carcinoma, gastric carcinoma, intestinal carcinoma, pancreatic carcinoma, glioblastoma, cervical carcinoma, ovarian carcinoma, liver carcinoma, bladder carcinoma, liver carcinoma, breast carcinoma, metastatic breast carcinoma, colon carcinoma, rectal carcinoma, colorectal carcinoma, uterine carcinoma, salivary gland carcinoma, kidney carcinoma, prostate carcinoma, vulval carcinoma, thyroid carcinoma, liver carcinoma, anal carcinoma, penile carcinoma, Merkel cell carcinoma, esophageal carcinoma, biliary tract carcinoma, head and neck carcinoma, and hematological malignancies.

23. The use according to claim 21, wherein the compound of general formula I, and the pharmaceutically acceptable salt, the stereoisomer, the ester, the prodrug, the solvate and the deuterated compound thereof are used alone or in combination with other pharmaceutical formulations and/or therapeutic methods;
the other pharmaceutical formulations are selected from: PD-1, PD-L1, CTLA-4, TIM-3, TGF-β and receptors thereof, LAG3 antagonists and TLR4, TLR7, TLR8, TLR9, and STING agonists;
the other therapeutic methods are selected from: radiotherapy and immunotherapy.

24. Use of the compound of general formula I, and the pharmaceutically acceptable salt, the stereoisomer, the ester, the prodrug, the solvate and the deuterated compound thereof according to claim 1 for preventing and/or treating cancer, immune disease and inflammation.

25. Use of the compound of general formula I, and the pharmaceutically acceptable salt, the stereoisomer, the ester, the prodrug, the solvate and the deuterated compound thereof according to claim 1 in cancer immunotherapy in combination with PD-1, PD-L1, CTLA-4, TIM-3, TGF-β and receptors thereof, LAG3 antagonists or TLR4, TLR7, TLR8, TLR9, and STING agonists.

26. Use of the compound of general formula I, and the pharmaceutically acceptable salt, the stereoisomer, the ester, the prodrug, the solvate and the deuterated compound thereof according to claim 1 in cancer immunotherapy in combination with CAR-T immunotherapy.
